(19) Europäisches Patentamt / European Patent Office / Office européen des brevets

(11) **EP 3 642 747 B1**

(12) **EUROPEAN PATENT SPECIFICATION**

(45) Date of publication and mention
of the grant of the patent:
**19.02.2025 Bulletin 2025/08**

(21) Application number: **18740026.2**

(22) Date of filing: **19.06.2018**

(51) International Patent Classification (IPC):
**G16B 20/00** *(2019.01)*   **G16B 20/10** *(2019.01)*
**G16B 20/20** *(2019.01)*   **G16B 30/00** *(2019.01)*
**G16B 30/10** *(2019.01)*   **G16B 40/00** *(2019.01)*

(52) Cooperative Patent Classification (CPC):
**G16B 20/00; G16B 20/10; G16B 20/20;**
**G16B 30/00; G16B 30/10; G16B 40/00**

(86) International application number:
**PCT/US2018/038342**

(87) International publication number:
**WO 2018/236911 (27.12.2018 Gazette 2018/52)**

(54) **METHODS AND SYSTEMS FOR DECOMPOSITION AND QUANTIFICATION OF DNA MIXTURES FROM MULTIPLE CONTRIBUTORS OF KNOWN OR UNKNOWN GENOTYPES**

VERFAHREN UND SYSTEME ZUR ZERSETZUNG UND QUANTIFIZIERUNG VON DNA-MISCHUNGEN VON MEHREREN SPENDERN MIT BEKANNTEN ODER UNBEKANNTEN GENOTYPEN

PROCÉDÉS ET SYSTÈMES DE DÉCOMPOSITION ET DE QUANTIFICATION DE MÉLANGES D'ADN PROVENANT DE MULTIPLES CONTRIBUTEURS AYANT DES GÉNOTYPES CONNUS OU INCONNUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **20.06.2017   US 201762522605 P**

(43) Date of publication of application:
**29.04.2020 Bulletin 2020/18**

(60) Divisional application:
**25151605.0**

(73) Proprietor: **Illumina, Inc.**
**San Diego, CA 92122 (US)**

(72) Inventors:
 • **LI, Yong**
 **San Diego**
 **California 92122 (US)**
 • **BRUAND, Jocelyne**
 **San Diego**
 **California 92122 (US)**
 • **KELLEY, Ryan**
 **San Diego**
 **California 92122 (US)**
 • **LEE, Chih**
 **San Diego**
 **California 92122 (US)**
 • **SCHEFFLER, Konrad**
 **San Diego**
 **California 92122 (US)**

(74) Representative: **Robinson, David Edward Ashdown**
**Marks & Clerk LLP**
**1 New York Street**
**Manchester M1 4HD (GB)**

(56) References cited:
**EP-A1- 3 285 193      WO-A1-2013/130848**
**WO-A1-2014/014498    WO-A1-2016/167408**

- "International Encyclopedia of the Social & Behavioral Sciences", vol. 15, 1 January 2001, ELSEVIER, NL, ISBN: 978-0-08-043076-8, article U.E. MAKOV: "Mixture Models in Statistics", pages: 9910 - 9915, XP055657054, DOI: 10.1016/B0-08-043076-7/00464-2
- SHALIZI C S: "Chapter 20: Mixture Models", 5 April 2011 (2011-04-05), XP055660261, Retrieved from the Internet <URL:https://www.stat.cmu.edu/~cshalizi/uADA/12/lectures/ch20.pdf> [retrieved on 20200121]
- JIANG P ET AL: "FetalQuant: deducing fractional fetal DNA concentration from massively parallel sequencing of DNA in maternal plasma", BIOINFORMATICS, vol. 28, no. 22, 8 September 2012 (2012-09-08), pages 2883 - 2890, XP055127069, ISSN: 1367-4803, DOI: 10.1093/bioinformatics/bts549
- NIELSEN R ET AL: "Genotype and SNP calling from next-generation sequencing data", NATURE REVIEWS GENETICS, vol. 12, no. 6, 1 June 2011 (2011-06-01), pages 443 - 451, XP055046801, ISSN: 1471-0056, DOI: 10.1038/nrg2986
- KIM S Y ET AL: "Estimation of allele frequency and association mapping using next-generation sequencing data", BMC BIOINFORMATICS, BIOMED CENTRAL, LONDON, GB, vol. 12, no. 1, 11 June 2011 (2011-06-11), pages 231, XP021102534, ISSN: 1471-2105, DOI: 10.1186/1471-2105-12-231

**Description**

## CROSS REFERENCE TO RELATED APPLICATIONS

This application claims benefits under 35 U.S.C. § 119(e) to U.S. Provisional Patent Application No. 62/522,605, entitled: METHODS FOR ACCURATE COMPUTATIONAL DECOMPOSITION OF DNA MIXTURES FROM CONTRIBUTORS OF UNKNOWN GENOTYPES, filed June 20, 2017.

## BACKGROUND

Sequencing data from a nucleic acid (e.g., DNA or RNA) mixture of closely related genomes is frequently found in research as well as clinical settings, and quantifying the mixing contributors has been a challenge when the original genomes are unknown. For example, in the context of microbiology and metagenomics, researchers and clinicians may need to quantify closely related bacterial strains of the same species in an environmental sample. In the setting of forensics, law enforcement personnel may need to quantify as well as identify human individuals from a blood sample containing DNA of multiple individuals. In the setting of biomedical research, scientists may need to determine the purity or extend of contamination in a cell or DNA sample.

Another application is Next Generation Sequencing (NGS) coupled liquid biopsy. NGS-coupled liquid biopsy is an emerging diagnosis strategy with potential applications in various clinical settings. In the context of organ or tissue transplant, NGS-coupled liquid biopsy provides a non-invasive approach for monitoring the health of allogeneic graft by quantifying the amount of allogeneic DNA in recipient blood. In some applications, the donor and recipient genomes are unknown or partially unknown.

The term chimera has been used in modern medicine to describe individuals containing cell populations originated from different individuals. The state of chimerism may occur spontaneously through inheritance, but is more frequently produced artificially via transplantation, transfusion, or sample contamination.

Chimerism leaves informative signals in different DNA types depending on the type of transplant. For bone marrow and hematopoietic stem cell transplants, blood genomic DNA (gDNA) collected post-transplant will have varying levels of chimerism depending on the engraftment state of the transplant. For solid organ transplants, chimerism signals can be seen in the blood cell-free DNA (cfDNA). Such signals can be extracted through non-invasive liquid biopsy, as contrast to the invasive tissue biopsy procedure that is the current standard of care for organ transplant monitoring.

Reproducible and accurate determinations of the relative contributions of donor genomes to a chimerism DNA sample would provide an informative tool for transplant monitoring, allowing researchers and clinicians to non-invasively and objectively measure the changes in dynamics among donor and recipient cells, which reflect the health status of the donor cells and organs. This disclosure introduces novel and improved methods for quantifying the relative contribution of each genome to a chimerism sample.

WO 2013/130848 A1 teaches informatics enhanced analysis of fetal samples subject to maternal contamination. Jiang P. et al. (2012), "FetalQuant: deducing fractional fetal DNA concentration from massively parallel sequencing of DNA in maternal plasma", Bioinformatics 2012;28(22):2883-2890, implements a statistical mixture model, which utilizes the maximum likelihood to estimate the fractional fetal DNA concentration directly from targeted MPS of DNA in maternal plasma.

## SUMMARY

Some implementations presented herein provide computer-implemented methods and systems for quantification and deconvolution of nucleic acid mixture samples including nucleic acid of two or more contributors of unknown genotypes. One aspect of the disclosure relates to methods for quantifying nucleic acid fractions in nucleic acid samples including nucleic acid (e.g., DNA or RNA) of two or more contributors having different genomes. In some implementations, the nucleic acid mixture samples include biological tissues, cells, peripheral blood, saliva, urine, and other biological fluid, as described below.

Because various methods and systems provided herein implement strategies and processes that use probabilistic mixture models and Bayesian inference techniques, the embodiments provide technological improvements over conventional methods in quantification and deconvolution of nucleic acid (e.g., DNA or RNA) mixture samples. Some implementations provide improved analytical sensitivity and specificity, providing more accurate deconvolution and quantification of nucleic acid mixture samples.

Some implementations allow accurate quantification of nucleic acid mixture samples with nucleic acid quantities that are too low for conventional methods to accurately quantify. Some implementations allow accurate quantification of 3-10ng of cell free DNA (cfDNA) mixture samples, which cannot be accurately quantified by conventional methods. Some implementations allow application to mixture samples with 3 or more contributors, which conventional methods cannot handle. Some implementations allow applications to mixtures with one or more unknown genomes, which conventional methods cannot handle. Some implementations described herein refer to a DNA sample, but it is understood that the implementations are also applicable to analyzing RNA samples.

Some embodiments provide a method of quantifying a nucleic acid sample comprising nucleic acid of two or more contributors, as defined by claim 1.

In some implementations, the mapping of (d) includes mapping using computer hashing or computer dynamic programming. In some implementations, the quantifying of (f) comprises quantifying using a novel optimization method combining a multi-iteration grid searching and a Broyden-Fletcher-Goldfarb-Shanno (BFGS) - quasi-Newton method. In some implementations, the quantifying of (f) comprises quantifying using an iterative weighted linear regression. These features require computers to perform and are rooted in computer technology.

In some implementations, the method further includes, determining, using the probabilistic mixture model and by the one or more processors, one or more genotypes of the one or more contributors at the one or more polymorphism loci.

In some implementations, the method further includes, determining, using the one or more fractions of nucleic acid of the one or more contributors, a risk of one contributor (a donee) rejecting a tissue or an organ transplanted from another contributor (a donor).

In some implementations, the nucleic acid molecules include DNA molecules or RNA molecules.

In some implementations, the transplant includes an allogeneic or xenogeneic transplant.

In some implementations, the extracted nucleic acid molecules include cell-free nucleic acid.

In some implementations, the extracted nucleic acid molecules include cellular DNA.

In some implementations, the one or more polymorphism loci include one or more biallelic polymorphism loci.

In some implementations, the one or more alleles at the one or more polymorphism loci include one or more single nucleotide polymorphism (SNP) alleles.

In some implementations, the probabilistic mixture model uses a single-locus likelihood function to model allele counts at a single polymorphism locus. The single-locus likelihood function includes:

$$M(n_{1i}, n_{2i} \mid p_{1i}, \theta)$$

$n_{1i}$ is the allele count of allele 1 at locus i, $n_{2i}$ is the allele count of allele 2 at locus i, $p_{1i}$ is an expected fraction of allele 1 at locus i, and $\theta$ includes one or more model parameters.

In some implementations, $p_{1i}$ is modeled as a function of: (i) genotypes of the contributors at locus $i$, or $g_i = (g_{11i}, ..., g_{D1i})$, which is a vector of copy number of allele 1 at locus $i$ in contributors 1... D; (ii) read count errors resulting from the sequencing operation in (c), or $\lambda$; and (iii) fractions of nucleic acid of contributors in the nucleic acid sample, or $\beta = (\beta_1, ..., \beta_D)$, wherein $D$ is the number of contributors. In some implementations, the contributors include two or more contributors, and $p_{1i} = p(g_i, \lambda, \beta) \leftarrow [(1- \lambda) g_i + \lambda (2- g_i)] / 2 \cdot \beta$, where • is vector dot product operator.

In some implementations, $p_{1i}$ is obtained using the $p_1'$ values in Table 3.

In some implementations, zero, one or more genotypes of the contributors are unknown. In some implementations, (f) includes marginalizing over a plurality of possible combinations of genotypes to enumerate the probability parameter $p_{1i}$. In some implementations, the method further includes determining a genotype configuration at each of the one or more polymorphism loci, the genotype configuration including two alleles for each of the one or more contributors. In some implementations, the single-locus likelihood function include a first binomial distribution. In some implementations, the first binomial distribution is expressed as follows:

$$n_{1i} \sim BN(n_i, p_{1i})$$

$n_{1i}$ is an allele count of nucleic acid sequence reads for allele 1 at locus $i$; and $n_i$ is a total read count at locus $i$, which equals to a total genome copy numbers $n''$. In some implementations, (f) includes maximizing a multiple-loci likelihood function calculated from a plurality of single-locus likelihood functions.

In some implementations, (f) includes: calculating a plurality of multiple-loci likelihood values using a plurality of potential fraction values and a multiple-loci likelihood function of the allele counts of nucleic acid sequence reads determined in (e); identifying one or more potential fraction values associated with a maximum multiple-loci likelihood value; and quantifying the one or more fractions of nucleic acid of the one or more contributors in the nucleic acid sample as the identified potential fraction value.

In some implementations, the multiple-loci likelihood function includes:

$$L(\boldsymbol{\beta}, \theta, \lambda, \pi ; n_1, n_2) = \Pi_i \left[ \Sigma g_i M(n_{1i}, n_{2i} \mid \boldsymbol{p}(g_i, \lambda, \boldsymbol{\beta}), \theta) \cdot \boldsymbol{P}(g_i \mid \pi) \right]$$

$L(\beta, \theta, \lambda, \pi ; n_1, n_2)$ is the likelihood of observing allele count vectors $n_1$ and $n_2$ for alleles 1 and 2; $p(g_i, \lambda, \beta)$ is the expected fraction or probability of observing allele 1 at locus $i$ based on the contributors' genotypes $g_i$ at locus i; $P(g_i|\pi)$ is the prior probability of observing the genotypes $g_i$ at locus i given a population allele frequency ($\pi$); and $\Sigma g_i$ denotes summing over a plurality of possible combinations of genotypes of the contributors.

In some implementations, the multiple-loci likelihood function includes:

$$L(\boldsymbol{\beta}, \lambda, \pi ; n_1, n_2) = \Pi_i \left[ \Sigma g_i BN(n_{1i} \mid n_i, \cdot \boldsymbol{p}(g_i, \lambda, \boldsymbol{\beta})) \cdot \boldsymbol{P}(g_i \mid \pi) \right]$$

In some implementations, the likelihood function includes:

$L(\beta, \lambda, \pi ; n_1, n_2) = \Pi_i \Sigma_{g1ig2i} BN(n_{1i} \mid n_i, p_{1i}(g_{1i}, g_{2i}, \lambda, \beta)) \cdot P(g_{1i}, g_{2i}|\pi)$
$L(\beta, \lambda, \pi ; n_1, n_2)$ is the likelihood of observing allele count vectors $n_1$ to $n_2$ for alleles 1 and 2 given parameters $\beta$ and $\pi$ ; $p_{1i}(g_{1i}, g_{2i}, \lambda, \beta)$ is a probability parameter, taken as $p_1'$ from Table 3, indicating a probability of allele 1 at locus $i$ based on the two contributors' genotypes $(g_{1i}, g_{2i})$; and $P(g_{1i}, g_{2i}|\pi)$ is a prior joint probability of observing the two contributors' genotypes given a population allele frequency ($\pi$).

In some implementations, the prior joint probability is calculated using marginal distributions $P(g_{1i}|\pi)$ and $P(g_{2i}|\pi)$ that satisfy the Hardy-Weinberg equilibrium.

In some implementations, the prior joint probability is calculated using genetic relationship between the two contributors.

In some implementations, the probabilistic mixture model accounts for nucleic acid molecule copy number errors resulting from extracting the nucleic acid molecules performed in (a), as well as the read count errors resulting from the sequencing operation in (c). In some implementations, the probabilistic mixture model uses a second binomial distribution to model allele counts of the extracted nucleic acid molecules for alleles at the one or more polymorphism loci. In some implementations, the second binomial distribution is expressed as follows:

$$n_{1i}'' \sim BN(n_i'', p_{1i})$$

$n_{1i}''$ is an allele count of extracted nucleic acid molecules for allele 1 at locus $i$; $n_i''$ is a total nucleic acid molecule count at locus $i$; and $p_{iu}$ is a probability parameter indicating the probability of allele 1 at locus $i$.

In some implementations, the first binomial distribution is conditioned on an allele fraction $n_{1i}''/n_i''$. In some implementations, the first binomial distribution is re-parameterized as follows:

$$n_{1i} \sim BN(n_i, n_{1i}''/n_i'')$$

$n_{1i}$ is an allele count of nucleic acid sequence reads for allele 1 at locus $i$; $n_i''$ is a total number of nucleic acid molecules at locus $i$, which equals to a total genome copy numbers $n''$; $n_i$ is a total read count at locus $i$; and $n_{1i}''$ is a number of extracted nucleic acid molecules for allele 1 at locus $i$.

In some implementations, the probabilistic mixture model uses a first beta distribution to approximate a distribution of $n_1/n''$. In some implementations, the first beta distribution has a mean and a variance that match a mean and a variance of the second binomial distribution. In some implementations, locus $i$ is modeled as biallelic and the first beta distribution is expressed as follows:

$$n_{i1}''/n'' \sim Beta((n''-1)p_{1i}, (n''-1)p_{2i})$$

$p_{1i}$ is a probability parameter indicating the probability of a first allele at locus $i$; and $p_{2i}$ is a probability parameter indicating the probability of a second allele at locus $i$.

In some implementations, (f) includes combining the first binomial distribution, modeling sequencing read counts, and the first beta distribution, modeling extracted nucleic acid molecule number, to obtain the single-locus likelihood function of $n_{1i}$ that follows a first beta-binomial distribution. In some implementations, the first beta-binomial distribution has the form: $n_{1i} \sim BB(n_i, (n''-1) \cdot p_{1i}, (n''-1) \cdot p_{2i})$, or an alternative approximation: $n_{1i} \sim BB(n_i, n'' \cdot p_{1i}, n'' \cdot p_{2i})$. In some implementations, the multiple-loci likelihood function includes:

$$L(\beta, n'', \lambda, \pi ; n_1, n_2) = \Pi i \left[ \Sigma g_i \, BB(n_{1i} \mid n_i, (n''-1) \cdot p_{1i}, (n''-1) \cdot p_{2i}) \cdot P(g_i \mid \pi) \right]$$

$L(\beta, n'', \lambda, \pi ; n_1, n_2)$ is the likelihood of observing allele count vectors $n_1$ and $n_2$ for alleles 1 and 2 at all loci, and $p_{1i} = p(g_i, \lambda, \beta)$, $p_{2i} = 1 - p_{1i}$.

In some implementations, the contributors include two contributors, and the multiple-loci likelihood function includes:

$$L(\beta, n'', \lambda, \pi ; n_1, n_2) = \Pi_i \Sigma_{g1ig2i} BB(n_{1i}, n_{2i} \mid n_i, (n''-1) \cdot p_{1i}(g_{1i}, g_{2i}, \lambda, \beta), (n''-1) \cdot p_{2i}(g_{1i}, g_{2i}, \lambda, \beta)) \cdot P(g_{1i}, g_{2i} \mid \pi).$$

$L(\beta, n'', \lambda, \pi ; n_1, n_2)$ is the likelihood of observing an allele count vector for the first allele of all loci ($n_1$) and an allele count vector for the second allele of all loci ($n_2$) given parameters $\beta$, $n''$, $\lambda$, and $\pi$; $p_{1i}(g_{1i}, g_{2i}, \lambda, \beta)$ is a probability parameter, taken as $p_1'$ from Table 3, indicating a probability of allele $1$ at locus $i$ based on the two contributors' genotypes $(g_{1i}, g_{2i})$; $p_{2i}(g_{1i}, g_{2i}, \lambda, \beta)$ is a probability parameter, taken as $p_2'$ from Table 3, indicating a probability of allele 2 at locus $i$ based on the two contributors' genotypes $(g_{1i}, g_{2i})$; and $P(g_{1i}, g_{2i} \mid \pi)$ is a prior joint probability of observing the first contributor's genotype for the first allele ($g_{1i}$) and the second contributor's genotype for the first allele ($g_{2i}$) at locus $i$ given a population allele frequency ($\pi$).

In some implementations, (f) includes estimating the total extracted genome copy number $n''$ from a mass of the extracted nucleic acid molecules. In some implementations, the estimated total extracted genome copy number $n''$ is adjusted according to fragment size of the extracted nucleic acid molecules.

In some implementations, the probabilistic mixture model accounts for nucleic acid molecule number errors resulting from amplifying the nucleic acid molecules performed in (b), as well as the read count errors resulting from the sequencing operation in (c). In some implementations, the amplification process of (b) is modeled as follows:

$$x_{t+1} = x_t + y_{t+1}$$

$x_{t+1}$ is the nucleic acid copies of a given allele after cycle $t+1$ of amplification; $x_t$ is the nucleic acid copies of a given allele after cycle $t$ of amplification; $y_{t+1}$ is the new copies generated at cycle $t+1$, and it follows a binomial distribution $y_{t+1} \sim BN(x_t, r_{t+1})$; and $r_{t+1}$ is the amplification rate for cycle $t+1$.

In some implementations, the probabilistic mixture model uses a second beta distribution to model allele fractions of the amplified nucleic acid molecules for alleles at the one or more polymorphism loci.

In some implementations, locus $i$ is biallelic and the second beta distribution is expressed as follows:

$$n_{1i}' / (n_{1i}' + n_{2i}') \sim Beta(n'' \cdot \rho_i \cdot p_{1i}, n'' \cdot \rho_i \cdot p_{2i})$$

$n_{1i}'$ is an allele count of amplified nucleic acid molecules for a first allele at locus $i$; $n_{2i}'$ is an allele count of amplified nucleic acid molecules for a second allele at locus $i$; $n''$ is a total nucleic acid molecule count at any locus; $\rho_i$ is a constant related to an average amplification rate r; $p_{1i}$ is the probability of the first allele at locus $i$; and $p_{2i}$ is the probability of the second allele at locus $i$. In some implementations, $\rho_i$ is $(1+r)/(1-r) / [1-(1+r)^{-t}]$, and r is the average amplification rate per cycle. In some implementations, $\rho_i$ is approximated as $(1+r)/(1-r)$.

In some implementations, (f) includes combining the first binomial distribution and the second beta distribution to obtain the single-locus likelihood function for $n_{1i}$ that follows a second beta-binomial distribution. In some implementations, the second beta-binomial distribution has the form:

$$n_{1i} \sim BB(n_i, n'' \cdot \rho_i \cdot p_{1i}, n'' \cdot \rho_i \cdot p_{2i})$$

$n_{1i}$ is an allele count of nucleic acid sequence reads for the first allele at locus $i$; $p_{1i}$ is a probability parameter indicating the probability of a first allele at locus $i$; and $p_{2i}$ is a probability parameter indicating the probability of a second allele at locus $i$.

In some implementations, (f) includes, by assuming the one or more polymorphism loci have a same amplification rate, re-parameterizing the second beta-binomial distribution as: $n_{1i} \sim BB(n_i, n'' \cdot (1+r)/(1-r)p_{1i}, n'' \cdot (1+r)/(1-r) \cdot p_{2i})$,

where *r* is an amplification rate. In some implementations, the multiple-loci likelihood function includes:

$$L(\beta, n'', r, \lambda, \pi ; n_1, n_2) = \Pi i [\Sigma g_i BB(n_{1i} \mid n_i, n'' \cdot (1+r)/(1-r) \cdot p_{1i}, n'' \cdot (1+r)/(1-r) \cdot p_{2i}) \cdot P(g_i \mid \pi)]$$

In some implementations, the contributors include two contributors and the multiple-loci likelihood function includes:

$$L(\beta, n'', r, \lambda, \pi ; n_1, n_2) = \Pi_i \Sigma_{g1ig2i} [BB(n_{1i} \mid n_i, n'' \cdot (1+r)/(1-r) \cdot p_{1i}(g_{1i}, g_{2i}, \lambda, \beta), n'' \cdot (1+r)/(1-r) \cdot p_{2i}(g_{1i}, g_{2i}, \lambda, \beta)) \cdot P(g_{1i}, g_{2i} \mid \pi)]$$

$L(\beta, n'', r, \lambda, \pi ; n_1, n_2)$ is the likelihood of observing an allele count vector for the first allele of all loci ($n_1$) and an allele count vector for the second allele of all loci ($n_2$) given parameters $\beta$, $n''$, $r$, $\lambda$, and $\pi$.

In some implementations, (f) includes, by defining a relative amplification rate of each polymorphism locus to be proportional to a total reads of the locus, re-parameterizing the second beta-binomial distribution as:

$$n_{1i} \sim BB(n_i, c' \cdot n_i \cdot p_{1i}, c' \cdot n_i \cdot p_{2i})$$

$c'$ is a parameter to be optimized; and $n_i$ is the total reads at locus *i*.

In some implementations, the multiple-loci likelihood function includes:

$$L(\beta, n'', c', \lambda, \pi ; n_1, n_2) = \Pi i [\Sigma g_i BB(n_{1i} \mid n_i, c' \cdot n_i \cdot p_{1i}, c' \cdot n_i \cdot p_{2i}) \cdot P(g_i \mid \pi)]$$

In some implementations, the probabilistic mixture model accounts for nucleic acid molecule number errors resulting from extracting the nucleic acid molecules performed in (a) and amplifying the nucleic acid molecules performed in (b), as well as the read count errors resulting from the sequencing operation in (c). In some implementations, the probabilistic mixture model uses a third beta distribution to model allele fractions of the amplified nucleic acid molecules for alleles at the one or more polymorphism loci, accounting for the sampling errors resulting from extracting the nucleic acid molecules performed in (a) and amplifying the nucleic acid molecules performed in (b). In some implementations, locus *i* is biallelic and the third beta distribution has the form of:

$$n_{1i}' / (n_{1i}' + n_{2i}') \sim Beta(n'' \cdot (1+ r_i)/2 \cdot p_{1i}, n'' \cdot (1+ r_i)/2 \cdot p_{2i})$$

$n_{1i}'$ is an allele count of amplified nucleic acid molecules for a first allele at locus *i*; $n_{2i}'$ is an allele count of amplified nucleic acid molecules for a second allele at locus *i*; $n''$ is a total nucleic acid molecule count; $r_i$ is the average amplification rate for locus i; $p_{1i}$ is the probability of the first allele at locus *i*; and $p_{2i}$ is a probability of the second allele at locus *i*. In some implementations, (f) includes combining the first binomial distribution and the third beta distribution to obtain the single-locus likelihood function of $n_{1i}$ that follows a third beta-binomial distribution.

In some implementations, the third beta-binomial distribution has the form:

$$n_{1i} \sim BB(n_i, n'' \cdot (1+ r_i)/2 \cdot p_{1i}, n'' \cdot (1+ r_i)/2 \cdot p_{2i})$$

$r_i$ is an amplification rate.

In some implementations, the multiple-loci likelihood function includes:

$$L(\beta, n'', r, \lambda, \pi ; n_1, n_2) = \Pi i [\Sigma g_i BB(n_{1i} \mid n_i, n'' \cdot (1+r)/2 \cdot p_{1i}, n'' \cdot (1+r)/2 \cdot p_{2i}) \cdot P(g_i \mid \pi)]$$

In some implementations, the multiple-loci likelihood function includes:

$$L(\beta, n'', r, \lambda, \pi ; n_1, n_2) = \Pi_i \Sigma_{g1ig2i} BB(n_{1i} \mid n_i, n'' \cdot (1+r)/2 \cdot p_{1i}(g_{1i}, g_{2i}, \lambda, \beta), n'' \cdot (1+r)/2 \cdot p_{2i}(g_{1i}, g_{2i}, \lambda, \beta)) \cdot P(g_{1i}, g_{2i} \mid \pi)$$

$L(n_1, n_2| \beta, n'', r, \lambda, \pi)$ is the likelihood of observing allele counts for the first allele vector $n_1$ and an allele count for the second allele vector $n_2$ given parameters $\beta$, $n''$, $r$, $\lambda$, and $\pi$.

In some implementations, the method further includes: (g) estimating one or more confidence intervals of the one or more fractions of nucleic acid of the one or more contributors using the hessian matrix of the log-likelihood using numerical differentiation.

In some implementations, the mapping of (d) comprises using computer hashing and computer dynamic programming. In some implementations, the mapping of (d) includes identifying, by the one or more processors using computer hashing and computer dynamic programing, reads among the nucleic acid sequence reads matching any sequence of a plurality of unbiased target sequences, wherein the plurality of unbiased target sequences includes sub-sequences of the reference sequence and sequences that differ from the subsequences by a single nucleotide. In some implementations, the plurality of unbiased target sequences includes five categories of sequences encompassing each polymorphic site of a plurality of polymorphic sites: (i) a reference target sequence that is a sub-sequence of the reference sequence, the reference target sequence having a reference allele with a reference nucleotide at the polymorphic site; (ii) alternative target sequences each having an alternative allele with an alternative nucleotide at the polymorphic site, the alternative nucleotide being different from the reference nucleotide; (iii) mutated reference target sequences including all possible sequences that each differ from the reference target sequence by only one nucleotide at a site that is not the polymorphic site; (iv) mutated alternative target sequences including all possible sequences that each differ from an alternative target sequence by only one nucleotide at a site that is not the polymorphic site; and (v) unexpected allele target sequences each having an unexpected allele different from the reference allele and the alternative allele, and each having a sequence different from the previous four categories of sequences.

In some implementations, the method further includes estimating a sequencing error rate $\lambda$ at the variant site base on a frequency of observing the unexpected allele target sequences of (v). In some implementations, (e) includes using the identified reads and their matching unbiased target sequences to determine allele counts of the nucleic acid sequence reads for the alleles at the one or more polymorphism loci. In some implementations, the plurality of unbiased target sequences includes sequences that are truncated to have the same length as the nucleic acid sequence reads. In some implementations, the plurality of unbiased target sequences includes sequences stored in one or more hash tables, and the reads are identified using the hash tables.

Another aspect of the disclosure provides a system for quantifying a nucleic acid sample including nucleic acid of two or more contributors, as defined by claim 26.

In some implementations, the system includes a tool for extracting nucleic acid molecules from the nucleic acid sample. In some implementations, the probability distributions include a first binomial distribution as follows:

$$n_{1i} \sim BN(n_i, p_{1i}).$$

$n_{1i}$ is an allele count of nucleic acid sequence reads for allele 1 at locus $i$; $n_i$ is a total read count at locus $i$, which equals to a total genome copy numbers $n''$; and $p_{1i}$ is a probability parameter indicating the probability of allele 1 at locus $i$.

An additional aspect of the disclosure provides a computer program product including a non-transitory machine readable medium storing program code that, when executed by one or more processors of a computer system, causes the computer system to implement a method of quantifying a nucleic acid sample including nucleic acid of two or more contributors, as defined by claim 28.

Although the examples herein concern humans and the language is primarily directed to human concerns, the concepts described herein are applicable to genomes from any plant or animal. These and other objects and features of the present disclosure will become more fully apparent from the following description and appended claims, or may be learned by the practice of the disclosure as set forth hereinafter.

## BRIEF DESCRIPTION OF THE DRAWINGS

Figures 1A-1C show an overview of a method and statistical model designed for contributor DNA quantification.

Figure 2A shows a block diagram illustrating a process for quantifying one or more fractions of nucleic acid (e.g., DNA or RNA) of one or more contributors in the nucleic acid sample.

Figure 2B shows a block diagram illustrating various components of a probabilistic mixture model.

Figure 2C schematically illustrates sequencing errors that convert one allele to another allele and true alleles to unexpected alleles.

Figure 3 shows a block diagram illustrating a process for evaluating a nucleic acid sample including nucleic acid of one or more contributors.

Figure 4 shows block diagram of a typical computer system that can serve as a computational apparatus according to certain embodiments.

Figure 5 shows one implementation of a dispersed system for producing a call or diagnosis from a test sample.

Figure 6 shows options for performing various operations of some implementations at distinct locations.

Figure 7 shows the performance of disclosed and baseline methods each under different choices of cfDNA length parameter.

Figure 8 shows analytical accuracy of some implementations in another format.

Figure 9 shows the coefficient of variance (CV) of 16 conditions for determining .limit of quantification (LOQ) for some implementations.

## DETAILED DESCRIPTION

### Definitions

Unless otherwise indicated, the practice of the method and system disclosed herein involves conventional techniques and apparatus commonly used in molecular biology, microbiology, protein purification, protein engineering, protein and DNA sequencing, and recombinant DNA fields, which are within the skill of the art. Such techniques and apparatus are known to those of skill in the art and are described in numerous texts and reference works (See e.g., Sambrook et al., "Molecular Cloning: A Laboratory Manual," Third Edition (Cold Spring Harbor), [2001]); and Ausubel et al., "Current Protocols in Molecular Biology" [1987]).

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The headings provided herein are not intended to limit the disclosure.

Unless defined otherwise herein, all technical and scientific terms used herein have the same meaning as commonly understood by one of ordinary skill in the art. Various scientific dictionaries that include the terms included herein are well known and available to those in the art. Although any methods and materials similar or equivalent to those described herein find use in the practice or testing of the embodiments disclosed herein, some methods and materials are described.

The terms defined immediately below are more fully described by reference to the Specification as a whole. It is to be understood that this disclosure is not limited to the particular methodology, protocols, and reagents described, as these may vary, depending upon the context they are used by those of skill in the art. As used herein, the singular terms "a," "an," and "the" include the plural reference unless the context clearly indicates otherwise.

Unless otherwise indicated, nucleic acids are written left to right in 5' to 3' orientation and amino acid sequences are written left to right in amino to carboxy orientation, respectively.

The term "chimerism sample" is used herein to refer to a sample believed to contain DNA of two or more genomes. Chimerism analysis is used herein to refer to the biological and chemical processing of a chimerism sample and/or the quantification of the nucleic acid of two or more organisms in the chimera sample. In some implementations, a chimerism analysis also determines some or all of the sequence information of the genomes of the two or more organisms.

The term donor DNA (dDNA) refers to DNA molecules originating from cells of a donor of a transplant. In various implementations, the dDNA is found in a sample obtained from a donee who received a transplanted tissue/organ from the donor.

Circulating cell-free DNA or simply cell-free DNA (cfDNA) are DNA fragments that are not confined within cells and are freely circulating in the bloodstream or other bodily fluids. It is known that cfDNA have different origins, in some cases from donor tissue DNA circulating in a donee's blood, in some cases from tumor cells or tumor affected cells, in other cases from fetal DNA circulating in maternal blood. In general, cfDNA are fragmented and include only a small portion of a genome, which may be different from the genome of the individual from which the cfDNA is obtained.

The term non-circulating genomic DNA (gDNA) or cellular DNA are used to refer to DNA molecules that are confined in cells and often include a complete genome.

The term "allele count" refers to the count or number of sequence reads of a particular allele. In some implementations, it can be determined by mapping reads to a location in a reference genome, and counting the reads that include an allele sequence and are mapped to the reference genome.

A beta distribution is a family of continuous probability distributions defined on the interval [0, 1] parameterized by two positive shape parameters, denoted by, e.g., $\alpha$ and $\beta$, that appear as exponents of the random variable and control the shape of the distribution. The beta distribution has been applied to model the behavior of random variables limited to intervals of finite length in a wide variety of disciplines. In Bayesian inference, the beta distribution is the conjugate prior probability distribution for the Bernoulli, binomial, negative binomial and geometric distributions. For example, the beta distribution can be used in Bayesian analysis to describe initial knowledge concerning probability of success. If the random variable $X$ follows the beta distribution, the random variable $X$ is written as $X \sim \text{Beta}(\alpha, \beta)$.

A binomial distribution is a discrete probability distribution of the number of successes in a sequence of n independent experiments, each asking a yes-no question, and each with its own Boolean-valued outcome: a random variable containing single bit of information: positive (with probability p) or negative (with probability q = 1 - p). For a single trial, i.e., n = 1, the binomial distribution is a Bernoulli distribution. The binomial distribution is frequently used to model the number of successes in a sample of size n drawn with replacement from a population of size N. If the random variable $X$ follows the binomial distribution with parameters $n \in \mathbb{N}$ and $p \in [0,1]$, the random variable $X$ is written as $X \sim B(n, p)$.

Poisson distribution, denoted as Pois() herein, is a discrete probability distribution that expresses the probability of a given number of events occurring in a fixed interval of time and/or space if these events occur with a known average rate and independently of the time since the last event. The Poisson distribution can also be used for the number of events in other specified intervals such as distance, area or volume. The probability of observing k events in an interval according to a Poisson distribution is given by the equation:

$$P(k \text{ events in interval}) = \frac{\lambda^k e^{-\lambda}}{k!}$$

where $\lambda$ is the average number of events in an interval or an event rate, also called the rate parameter e is 2.71828, Euler's number, or the base of the natural logarithms, k takes values 0, 1, 2, ..., and $k!$ is the factorial of $k$.

Gamma distribution is a two-parameter family of continuous probability distributions. There are three different parametrizations in common use: with a shape parameter k and a scale parameter $\theta$; with a shape parameter $\alpha$ = k and an inverse scale parameter $\beta$ = 1/$\theta$, called a rate parameter; or with a shape parameter k and a mean parameter $\mu$ = k/$\beta$. In each of these three forms, both parameters are positive real numbers. The gamma distribution is the maximum entropy probability distribution for a random variable X for which E[X] = k$\theta$ = $\alpha$/$\beta$ is fixed and greater than zero, and E[ln(X)] = $\psi$(k) + ln($\theta$) = $\psi$($\alpha$) - ln($\beta$) is fixed ($\psi$ is the digamma function).

Polymorphism and genetic polymorphism are used interchangeably herein to refer to the occurrence in the same population of two or more alleles at one genomic locus, each with appreciable frequency.

Polymorphism site and polymorphic site are used interchangeably herein to refer to a locus on a genome at which two or more alleles reside. In some implementations, it is used to refer to a single nucleotide variation with two alleles of different bases.

Allele frequency or gene frequency is the frequency of an allele of a gene (or a variant of the gene) relative to other alleles of the gene, which can be expressed as a fraction or percentage. An allele frequency is often associated with a particular genomic locus, because a gene is often located at with one or more locus. However, an allele frequency as used herein can also be associated with a size-based bin of DNA fragments. In this sense, DNA fragments such as cfDNA containing an allele are assigned to different size-based bins. The frequency of the allele in a size-based bin relative to the frequency of other alleles is an allele frequency.

The term "parameter" herein refers to a numerical value that characterizes a property of a system such as a physical feature whose value or other characteristic has an impact on a relevant condition such as a sample or DNA fragments. In some cases, the term parameter is used with reference to a variable that affects the output of a mathematical relation or model, which variable may be an independent variable (i.e., an input to the model) or an intermediate variable based on one or more independent variables. Depending on the scope of a model, an output of one model may become an input of another model, thereby becoming a parameter to the other model.

The term "plurality" refers to more than one element.

The term "paired end reads" refers to reads from paired end sequencing that obtains one read from each end of a nucleic acid fragment. Paired end sequencing may involve fragmenting strands of polynucleotides into short sequences called inserts. Fragmentation is optional or unnecessary for relatively short polynucleotides such as cell free DNA molecules.

The terms "polynucleotide," "nucleic acid" and "nucleic acid molecules" are used interchangeably and refer to a covalently linked sequence of nucleotides (i.e., ribonucleotides for RNA and deoxyribonucleotides for DNA) in which the 3' position of the pentose of one nucleotide is joined by a phosphodiester group to the 5' position of the pentose of the next. The nucleotides include sequences of any form of nucleic acid, including, but not limited to RNA and DNA molecules such as cfDNA or cellular DNA molecules. The term "polynucleotide" includes, without limitation, single- and double-stranded polynucleotide.

The term "test sample" herein refers to a sample typically derived from a biological fluid, cell, tissue, organ, or organism, comprising a nucleic acid or a mixture of nucleic acids. Such samples include, but are not limited to sputum/oral fluid, amniotic fluid, blood, a blood fraction, or fine needle biopsy samples (e.g., surgical biopsy, fine needle biopsy, etc.), urine, peritoneal fluid, pleural fluid, and the like. Although the sample is often taken from a human subject (e.g., patient), the assays can be used in samples from any mammal, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc. If such methods of pretreatment are employed with respect to the sample, such pretreatment methods are typically such that the nucleic acid(s) of interest remain in the test sample, sometimes at a concentration proportional to that in an untreated test sample (e.g., namely, a sample that is not subjected to any such pretreatment method(s)). Such "treated" or "processed" samples are still considered to be biological "test" samples with respect to the methods described herein.

The term "Next Generation Sequencing (NGS)" herein refers to sequencing methods that allow for massively parallel sequencing of clonally amplified molecules and of single nucleic acid molecules. Non-limiting examples of NGS include sequencing-by-synthesis using reversible dye terminators, and sequencing-by-ligation.

The term "read" refers to a sequence obtained from a portion of a nucleic acid sample. Typically, though not necessarily, a read represents a short sequence of contiguous base pairs in the sample. The read may be represented symbolically by the base pair sequence (in A, T, C, or G) of the sample portion. It may be stored in a memory device and processed as appropriate to determine whether it matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

The term "genomic read" is used in reference to a read of any segments in the entire genome of an individual.

As used herein, the terms "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain embodiments, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

Aligned reads or tags are one or more sequences that are identified as a match in terms of the order of their nucleic acid molecules to a known sequence from a reference genome. Alignment can be done manually, although it is typically implemented by a computer program, as it would be impossible to align reads in a reasonable time period for implementing the methods disclosed herein. One example of an program from aligning sequences is the Efficient Local Alignment of Nucleotide Data (ELAND) computer program distributed as part of the Illumina Genomics Analysis pipeline. Alternatively, a Bloom filter or similar set membership tester may be employed to align reads to reference genomes. See US Patent Application No. 61/552,374 filed October 27, 2011. The matching of a sequence read in aligning can be a 100% sequence match or less than 100% (non-perfect match).

The term "mapping" used herein refers to specifically assigning a sequence read to a larger sequence, e.g., a reference genome, a subsequence of the larger sequence using alignment or membership assignment.

As used herein, the term "reference genome" or "reference sequence" refers to any particular known genome sequence, whether partial or complete, of any organism or virus which may be used to reference identified sequences from a subject. For example, a reference genome used for human subjects as well as many other organisms is found at the National Center for Biotechnology Information at ncbi.nlm.nih.gov. A "genome" refers to the complete genetic information of an organism or virus, expressed in nucleic acid sequences.

In various embodiments, the reference sequence is significantly larger than the reads that are aligned to it. For example, it may be at least about 100 times larger, or at least about 1000 times larger, or at least about 10,000 times larger, or at least about $10^5$ times larger, or at least about $10^6$ times larger, or at least about $10^7$ times larger.

In one example, the reference sequence is that of a full length human genome. Such sequences may be referred to as genomic reference sequences. In another example, the reference sequence is limited to a specific human chromosome such as chromosome 13. In some embodiments, a reference Y chromosome is the Y chromosome sequence from human genome version hg19. Such sequences may be referred to as chromosome reference sequences. Other examples of reference sequences include genomes of other species, as well as chromosomes, sub-chromosomal regions (such as strands), etc., of any species.

In various embodiments, the reference sequence is a consensus sequence or other combination derived from multiple individuals. However, in certain applications, the reference sequence may be taken from a particular individual.

The term "derived" when used in the context of a nucleic acid or a mixture of nucleic acids, herein refers to the means whereby the nucleic acid(s) are obtained from the source from which they originate. For example, in one embodiment, a mixture of nucleic acids that is derived from two different genomes means that the nucleic acids, e.g., cfDNA, were naturally released by cells through naturally occurring processes such as necrosis or apoptosis. In another embodiment, a mixture of nucleic acids that is derived from two different genomes means that the nucleic acids were extracted from two different types of cells from a subject. For instance, a mixture of nucleic acids includes nucleic acids originating from donor cells and donee cells obtained from an organ transplant subject. In some implementations, a mixture of nucleic acids comprise biological materials of two or more contributor individuals. For example, a forensic sample including biological materials of two or more individuals includes DNA of the two or more individuals.

The term "based on" when used in the context of obtaining a specific quantitative value, herein refers to using another quantity as input to calculate the specific quantitative value as an output.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

As used herein, the term "corresponding to" sometimes refers to a nucleic acid sequence, e.g., a gene or a chromosome, that is present in the genome of different subjects, and which does not necessarily have the same sequence in all genomes, but serves to provide the identity rather than the genetic information of a sequence of interest, e.g., a gene or chromosome.

The term "contributor" herein refers to a human contributor as well as a non-human contributor such as a mammal, an invertebrate, a vertebrate, a fungus, a yeast, a bacterium, and a virus. Although the examples herein concern humans and the language is primarily directed to human concerns, the concepts disclosed herein are applicable to genomes from any plant or animal, and are useful in the fields of veterinary medicine, animal sciences, research laboratories and such.

The term "sensitivity" as used herein refers to the probability that a test result will be positive when the condition of interest is present. It may be calculated as the number of true positives divided by the sum of true positives and false negatives.

The term "specificity" as used herein refers to the probability that a test result will be negative when the condition of interest is absent. It may be calculated as the number of true negatives divided by the sum of true negatives and false positives.

The term "primer," as used herein refers to an isolated oligonucleotide that is capable of acting as a point of initiation of synthesis when placed under conditions inductive to synthesis of an extension product (e.g., the conditions include nucleotides, an inducing agent such as DNA polymerase, and a suitable temperature and pH). The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer, use of the method, and the parameters used for primer design.

## Introduction

This disclosure provides methods and systems for quantification and deconvolution of nucleic acid mixture samples including nucleic acid of two or more contributors of unknown genotypes, providing various advantages and technological improvements. For instance, some implementations apply probabilistic mixture modeling, Bayesian inference techniques, and numerical optimization methods to quantify contributor DNA in a mixture without knowing contributor's genotypes.

Sequencing data from a nucleic acid (e.g., DNA or RNA) mixture of closely related genomes is frequently found in research as well as clinical settings, and quantifying the mixing contributors has been a challenge when the original genomes are unknown.

Conventional methods of chimerism analysis (for bone marrow and blood stem cell transplants only) utilize capillary electrophoresis (CE) fragment analysis or quantitative polymerase chain reaction (qPCR) analysis of short tandem repeats (STRs) or small insertions and deletions (Indels). These methods tend to have poor limit of quantification, dynamic range, or reproducibility. They have limited number of targets, complicated workflow, and time-consuming and inaccurate manual input for analysis. The conventional methods tend to comprise among these different metrics. CE approach has a LOQ ranging from 1%-5%, and suffers from low reproducibility. These limitations can be significant in clinical use. For example, an actual chimerism result of 99% will be reported as 100%. The qPCR approach can achieve low LOQ of 0.1% but that requires 66ng or more chimerism DNA not considering the DNA required for pure baseline samples. Neither 66ng nor 10ng is possible for routine cfDNA analysis for solid organ transplant. In addition, the dynamic range of qPCR-based chimerism suffers, and chimerism predictions when the minor contributor is greater than 30% are not reliable.

Given the high input DNA requirement, CE and qPCR approaches are only applicable to bone marrow or blood stem cell transplant. Neither approach works for solid organ transplant monitoring, for which the cfDNA amount from a typical blood draw is much less than 10ng. In addition, even at the same amount, cfDNA is not as effective as gDNA as PCR template.

Besides high DNA input requirement, both CE and qPCR approaches require pure pre-transplant baseline samples to be available. They are also associated with complex assay and require manual intervention in selecting the appropriate markers before quantification.

In addition to these, there are two fundamental challenges in chimerism quantification that our methods systematically addressed, while existing methods do not work.

The first challenge is to quantify chimerism sample with more than two contributors, corresponding to transplant with more than one donor. Multi-donor transplants are common for bone marrow and blood stem cell transplants. It also occurs in solid organ transplant, for example for 2nd kidney transplant following the failure of previous kidney, or when solid organ transplant coincide with blood transfusion from another donor.

The second challenge is to quantify chimerism samples when one of the contributors is unknown. This occurs frequently in clinical setting, for example 1) when the donor genome is not available, 2) when in the multi-donor cases, when an old organ's donor genome is not available, or 3) when solid organ transplant recipient also received blood transfusion from unknown donors.

While conventional methods do not address these challenges, the methods disclose herein can accurately quantify chimerism samples when there is an unknown donor. When there is only one donor neither the donor or recipient genome are required using the disclosed methods. Further, the disclosed methods can work with arbitrary number of donors. Some empirical studies have validated the performance of the disclosed method for 4 donors and achieved an LOQ of less than 0.35% at10ng total gDNA input.

In some implementations, the disclosed methods achieve 0.1% to 0.2% LOQ with as low as 3ng cfDNA input, and achieve a broad dynamic range from 0.1% - 99.9%. Some implementations do not require baseline genomes to be known, although knowing the baseline can improve performance. The disclosed methods can work with chimerism samples of arbitrary number of donors, and have been experimentally validated for samples with 0-4 donors, which covers nearly all clinically relevant cases for solid organ transplant, bone marrow transplant, and hematopoietic stem cell transplants. In addition, the disclosed methods do not require any manual intervention in selecting genetic markers, allowing digitization and automation of quantification of nucleic acids.

Some implementations provide methods and systems for quantifying contributor DNA from multi-marker targeted-resequencing data of blood cfDNA or gDNA samples. Some implementations provide methods and systems for quantifying contributor DNA from multi-marker targeted-resequencing data of blood cfDNA or gDNA samples using novel probabilistic models and numerical optimization methods. Some implementations provide methods and systems for quantifying contributor DNA for genetically related donor and recipient of unknown genotypes using Bayesian modeling with prior distributions that encode genetic-relationship. By using genetic-relationship information to provide prior information in a Bayesian framework, quantification of DNA mixture can be improved compared to methods that do not use the genetic-relationship information.

Some implementations provide methods and systems for estimating the confidence interval of DNA quantification by numerically computing the Cramer-Rao bound from the estimated Hessian matrices of log-likelihood functions.

Allelic bias in short sequencing read mapping confounds DNA quantification. In some implementations, we reduce the confounding effect through an unbiased mapping strategy of reads spanning variant sites.

Implementations described herein can accurately estimate the contributor DNA fraction even though the genotypes for the contributing genomes are totally unknown. The allele fraction of a marker site after PCR amplification can be reliably modeled with a beta-distribution.

Using the unbiased reference DNA sequence database containing both reference and alternate allele, one can remove read mapping biases towards the reference alleles, and reliably estimate the allele counts and sequencing error at the variant sites.

Implementations described herein can estimate the confidence interval of the predicted contributor DNA fractions with a single sequencing run of a mixture DNA sample.

Formally, the problem of contributor DNA quantification (CDQ) is stated as following: Given the sequencing data of a DNA sample comprised of one or more contributors, determine the fraction of each contributor in the sample. When the genotypes of the contributor genomes are unknown, the CDQ problem is referred to as blind contributor DNA quantification (blind-CDQ); the opposite is referred to as non-blind-CDQ. Some descriptions regarding some implementations refer to the two contributors as the donor and the recipient, but they do not limit the applications of the methods to the organ donation setting. In some description hereinafter regarding some implementations, a contributor is equivalent to a donor, and the other contributor is equivalent to a donee.

Blind-CDQ is a harder problem compared to non-blind CDQ, but it is of wider application to all scenarios where only a single sequencing experiment of the mixture sample is achievable, while the non-blind-CDQ requires prior sequencing experiments to determine genotypes of the contributors (e.g. organ donors and recipients).

The computational methods described in this document address both the blind-CDQ and the non-blind-CDQ problems with single, two, or multiple contributors.

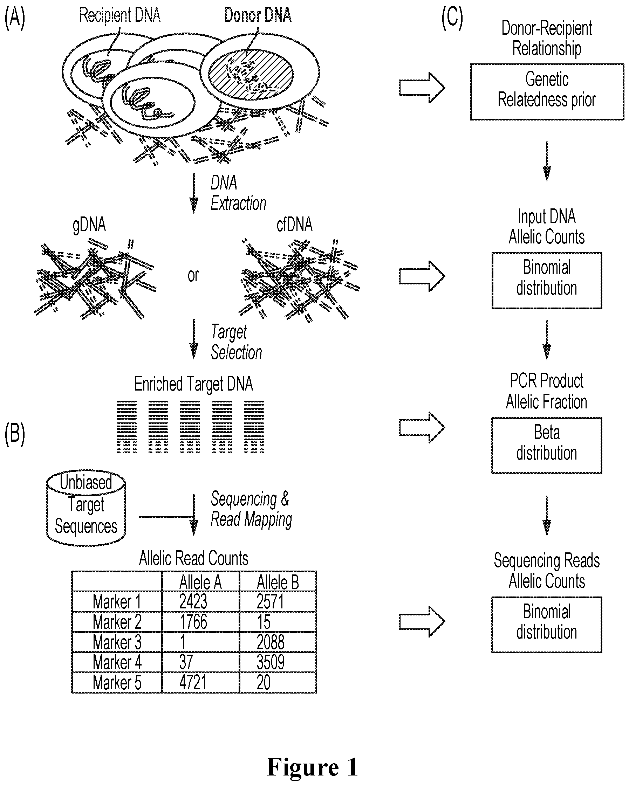

Figures 1A-1C show an overview of methods and statistical model designed for contributor DNA quantification. Figure 1A shows an experimental pipeline for sequencing based allogeneic DNA detection. Figure 1B shows an unbiased read mapping workflow for allele counting. Figure 1C shows a hierarchical, probabilistic mixture model for allelic counts per marker locus.

Some implementations apply experimental pipeline as depicted in Figure 1A. This generic experimental pipeline has the following steps.

1) A blood or other type of sample is obtained containing DNA from multiple genetic origins.

2) The appropriate type of DNA is extracted, e.g. cellular DNA (also referred to as gDNA) or cell free DNA (cfDNA), depending on the application.

3) Specific variant sites or polymorphism sites of the genome is targeted and enriched by approaches such as PCR amplification or hybridization. The variant sites are prior-selected to be variable among diverse populations of human (or another organism of interest). Alternatively, untargeted (whole genome) sequencing can be done, and all variant sites will be covered.

4) The DNA sample is sequenced by NGS or other DNA sequencing techniques such as some of the ones described hereinafter to obtain sequencing reads that cover variant sites of interest.

The computational method for CDQ has three main components:

1) Allele Counting: an computer program based on hashing and dynamic programming for unbiased counting of sequencing reads from each allele for each target marker site (Figure 1B), and
2) Contributor DNA Quantification: a hierarchical probabilistic model and novel combination of multi-iteration grid search strategy with BFGS - quasi-Newton method, or in some implementations an iterative weighted linear regression, for quantifying the contributor DNA fraction (Figure 1C).
3) Confidence interval (uncertainly) determination: around the quantified mixture fractions, variances are determined based on the hessian matrix of the log likelihood function base on information inequality.

The totality of these components for chimerism quantification is impossible to execute manually by human experts or be carried out in their heads. They require computers and are computer-implemented technology. These computational components allow the disclosed methods to achieve unparalleled quantification sensitivity, dynamic range, and reproducibility. They also enable the disclosed methods to reliably quantify diverse set of chimerism samples, including cfDNA or gDNA, 3-10ng or more input DNA, 0 to 4 or more donors, and genetically related or unrelated donor with known or unknown genomes.

Although some implementation only address "relative quantification" here, meaning that the implementations estimate the percentage or fraction of the DNA sample that is originated from the contributor sources, rather than the absolute amount (in terms of mass or copy numbers). Additional steps can be taken to convert the relative abundance to absolute abundance if the total amount of input DNA is measured or known.

**Overview** of Processes **for Quantifying Contributor Fractions in a Nucleic Acid Sample**

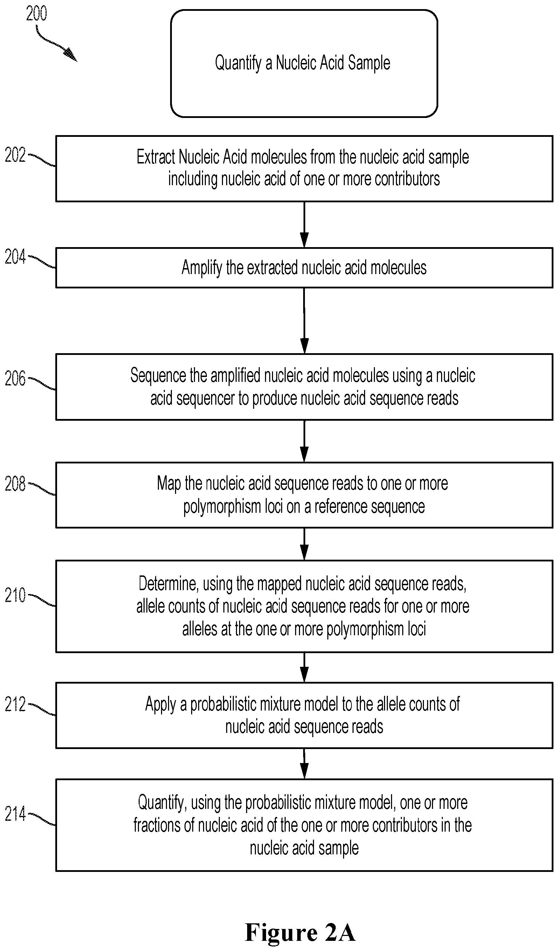

Figure 2A shows a block diagram illustrating a process 200 for quantifying one or more fractions of nucleic acid (e.g., DNA or RNA) of one or more contributors in the nucleic acid sample. The method is implemented on a computer system that includes one or more processors and system memory such as the systems described hereinafter. Descriptions herein refer to DNA in some implementations and applications, but one skilled in the art appreciates that other forms of nucleic acids can also be analyzed using the implementations described herein. The various implementations described herein can be used to analyze a nucleic acid sample containing nucleic acid from one or more contributors. In some implementations, methods and systems are provided to quantify one or more fractions of nucleic acid of the one or more contributors. In some descriptions herein, the nucleic acid sample is referred to as a mixture sample because the sample can include nucleic acid from two more contributors. However, it is understood that the use of the term "mixture" indicates the possibility that the sample includes two or more contributors' nucleic acid, and it does not exclude the possibility that the sample includes nucleic acid from only a single contributor. In the latter case, a fraction of 1 or a percentage of 100% (or values within a margin of error) may be determined for the one contributor.

In some implementations, the one or more contributors of the nucleic acid sample include a donor of a transplant and a donee of the transplant. In some implementations, the transplant includes an allogeneic or a xenogeneic transplant. In some implementations, the nucleic acid sample is a biological sample obtained from the donee. In some implementations, the nucleic acid sample includes cell-free nucleic acid. In some implementations, the sample includes cellular DNA. In some implementations, the nucleic acid sample includes nucleic acid from zero, one, or more contaminant genomes and one genome of interest. In some implementations, the nucleic acid sample includes a biological sample obtained from a cell culture, which can be a mixture of multiple cell lines of different genetic origins in some implementations.

Process 200 involves extracting nucleic acid molecules from the nucleic acid sample using techniques such as those described herein. See block 202.

Process 200 further involves amplifying or enriching the extracted nucleic acid molecules. See block 204. Various amplification or enrichment techniques such as those described herein may be used. In some implementations, PCR are used to amplify the extracted nucleic acid molecules. In some implementations, the amplification targets specific polymorphisms, which amplification is also referred to as targeted enrichment. In other implementations, whole genome amplification may be performed, and allele data for specific polymorphism sites may be obtained by sequencing.

Process 200 also involves sequencing the amplified or enriched nucleic acid molecules using a nucleic acid sequencer to produce nucleic acid sequence reads. See block 206. Various sequencing techniques and devices are further described hereinafter, which may be applied in operation 206.

Process 200 further involves mapping the nucleic acid sequence reads to one or more polymorphism loci on a reference sequence. In some implementations, alignment techniques may be used to map the nucleic acid sequence reads to one or more polymorphism loci. In other implementations, an unbiased mapping techniques may be used to match the nucleic acid sequence reads to the polymorphism loci. See block 208. In some implementations, the nucleic acid sequence reads are mapped to specific alleles at the polymorphism loci. The unbiased mapping technique is further described hereinafter. In some implementations, the one or more polymorphism loci (or polymorphic loci) include biallelic loci. In some implementations, the alleles at the one or more polymorphism loci include single nucleotide polymorphism (SNP) alleles.

In some implementations, unique molecular indexes (UMIs) are attached to the extracted nucleic acid molecules, which are then amplified, sequenced, and mapped to the polymorphism loci or alleles. The unique molecular indices provide mechanisms to reduce the errors that can occur in the sample processing and analysis steps. For instance, different reads sharing a same unique molecular index (UMI) can be combined or collapsed to determine a sequence from which the reads are derived, effectively removing errors that have occurred during amplification and sequencing.

Process 200 further involves determining, using the method nucleic acid sequence reads, allele counts of nucleic acid sequence reads for alleles at the one or more polymorphism loci. See block 210.

Process 200 also involves applying the probabilistic mixture model to the allele counts of nucleic acid sequence reads. The probabilistic mixture model uses probability distributions to model allele count of nucleic acid sequence reads at the one or more polymorphism loci. The probability distributions account for errors and noises in the nucleic acid sequence reads. The probabilistic mixture model treats each allele count of nucleic acid sequence reads as a random sample from a probability distribution.

In the equations hereinafter, the notations below are used.

*d:* indicator for donors, *d = 1, 2..., D, where D* is the total number of contributors. *D* can be any natural number. In some implementations, *D* is 5 or smaller. In some implementations, D is 9 or smaller.

*a*: indicator for alleles. In some implementations, the alleles include biallelic SNPs, *and a = 1* or 2.

i: indicator for marker loci, $i = 1 ... I$, where $I$ is the total number of markers, e.g. 300.

$g_{dai}$: genotypes of contributor $d$ allele type $a$ for marker $i$. It takes value 0, 1, or 2, representing the number of copies of allele $a$ for this locus in this contributor.

$n_{ai}$, $n_{ai}'$, $n_{ai}''$: copies of reads, DNA molecules after amplification, and DNA molecules before amplification, of allele type $a$ and marker locus $i$ .

$n_i$, $n_i'$, $n_i''$: total copies of reads, nucleic acid molecules after amplification, and DNA molecules before amplification, for marker locus $i$.

$r_{ai}$: fractions of read counts for allele type $\alpha$ and marker locus $i$.

$p_{ai}$: probability of seeing a read of allele type $a$ at a given marker locus $i$.

Note that for $g_{dai}$, $n_{ai}$, $n_{ai}'$, $n_{ai}''$, $n_i$, $n_i'$, $n_i''$, $r_{ai}$, and $p_{ai}$, the subscript $i$ is sometimes omitted when the implementations are focused on a single locus.

$\beta_d$: fraction of nucleic acids from contributor $d$ that contribute to a mixture sample.

$\lambda$: Sequencing error rate.

Bold letters represent vectors or matrices:

$g = [g_{d1i}]_{i = 1...I,\ d=1...D}$: genotype matrix with reference allele counts in all contributors and all loci.

$g_i = [_{gd1i}]_{d=1...D}$: genotype vector with reference allele counts for all contributors and given locus $i$.

$r = [r_{1i}]_{i = 1...i}$: allele fraction vector with fractions of allele 1 reads for every loci.

$n = [n_i]_{i = 1...i}$: read count vector with read count for every loci.

$p = [p_{1i}]_{i = 1...i}$: vector with expected allele 1 fraction for every loci.

$\beta = [\beta_d]_{d = 1...D}$: contributor fraction vector with relative fraction of each contributor contributing to the nucleic acid sample.

In some implementations, the probabilistic mixture model uses a single-locus likelihood function to model allele counts at a single polymorphism locus, the single-locus likelihood function can be expressed as:
$M(n_{1i}, n_{2i} | p_{1i}, \theta)$, where $n_{1i}$ is the allele count of allele 1 at locus i, $n_{2i}$ is the allele count of allele 2 at locus i, $p_{1i}$ is an expected fraction of allele 1 at locus i, and $\theta$ includes one or more model parameters.

In some implementations, $p_{1i}$ is modeled as a function $p(g_i, \lambda, \beta)$ of: (i) genotypes of the contributors at locus $i$, or $g_i = (g_{11i}, ..., g_{D1i})$, which is a vector of copy number of allele 1 at locus $i$ in contributors 1... D; (ii) read count errors, or $\lambda$, resulting from the sequencing; and (iii) fractions of nucleic acid of contributors in the nucleic acid sample, or $\beta = (\beta_1, ..., \beta_D)$, where $D$ is the number of contributors.

In some implementations, $p_{1i}$ is calculated as $p_{1i} = p(g_i, \lambda, \beta) \leftarrow [(1- \lambda)\ g_i + \lambda\ (2-g_i)] / 2 \bullet \beta$, where • is vector dot product operator.

In some implementations, the contributors include two contributors, and $p_{1i}$ is obtained using the $p_1'$ values in Table 3 described hereinafter.

In some implementations (method S), the single-locus likelihood function is a probability distribution that includes a first binomial distribution. In some implementations, the first binomial distribution includes a quantity parameter indicating the total allele count at a locus and a probably parameter indicating a probability of the first allele at the locus. In some implementations, the first binomial distribution is expressed as follows:

$$n_{1i} \sim BN(n_i, p_{1i})$$

where $n_{1i}$ is an allele count of nucleic acid sequence reads for allele 1 at locus $i$; $\boldsymbol{n_i}$ is a total read count at locus $i$; and $p_{1i}$ is a probability parameter indicating the probability of allele 1 at locus $i$.

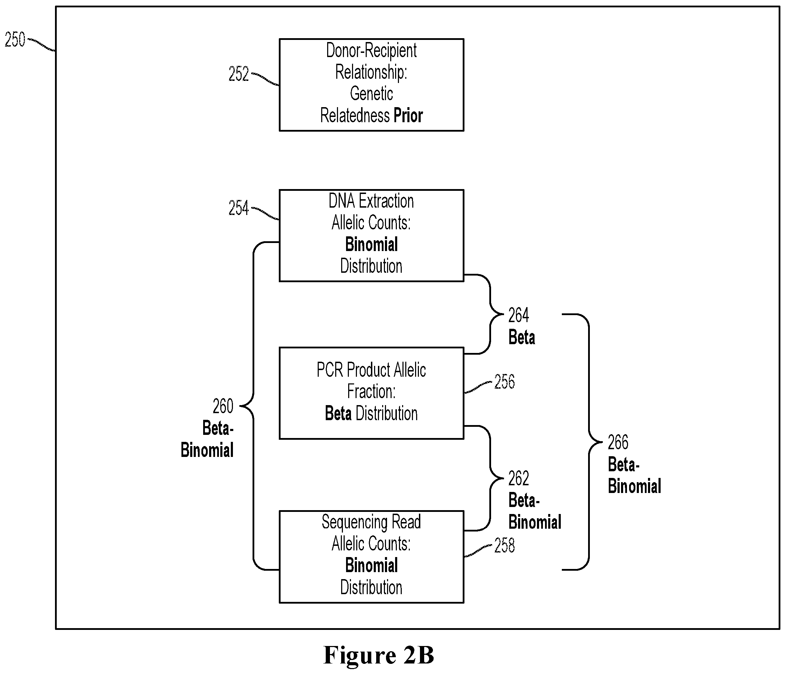

In some implementations, the probability parameter $p$ is a function of a fraction of nucleic acid of a contributor, or $\beta$. The probably parameter is also a function of genotypes of the one or more contributors g. The probability parameter is also a function of errors resulting from the sequencing operation of 206, or $\lambda$. In some implementations, zero, one or more genotypes of the contributors were unknown. In some implementations the probabilistic mixture model includes various probability distributions as shown in Figure 2B.

Returning to Figure 2A, process 200 involves quantifying, using the probabilistic mixture model, one or more fractions of nucleic acid of the one or more contributors in the nucleic acid sample. See block 214. In some implementations, the quantifying includes marginalizing over a plurality of possible combinations of genotypes to enumerate the probability parameter $p$. In some implementations, the quantifying includes determining $\beta$, the fractions of nucleic acid of the contributors, using a multiple-loci likelihood function of the allele counts of nucleic acid sequence reads determined in operation 210 conditioned on parameters of the probabilistic mixture model.

In some implementations, the quantification includes calculating a plurality of likelihood values using a plurality of potential fraction values and a multiple-loci likelihood function of the allele counts of nucleic acid sequence reads. The quantification also involves identifying a potential fraction value that is associated with a likelihood value that is the maximum value among the plurality of likelihood values. In some implementations, the plurality of likelihood values is obtained for a plurality of parameters and the values thereof in a multidimensional grid. The quantification also involves quantifying the fraction of nucleic acid of the contributor(s) in the nucleic acid sample at the identified potential fraction value having the maximum likelihood. In some implementations, the multiple-loci likelihood function includes a plurality of marginal distributions for the one or more polymorphism loci.

In some implementations, the multiple-loci likelihood function of the one or more contributors, with known, unknown, or partially known genotypes, is computed as following:

$$L(\boldsymbol{\beta}, \theta, \lambda, \pi ; n_1, n_2) = \Pi_i \left[ \Sigma_{gi} M(n_{1i}, n_{2i} \mid p(g_i, \lambda, \boldsymbol{\beta}), \theta) \cdot P(g_i \mid \pi) \right]$$

where $L(\beta, \theta, \lambda, \pi ; n_1, n_2)$ is the likelihood of observing allele count vectors $n_1$ and $n_2$ for alleles 1 and 2; $p(g_i, \lambda, \beta)$ is the expected fraction or probability of observing allele 1 at locus $i$ based on the contributors' genotypes $g_i$ at locus i; $P(gi|\pi)$ is the prior probability of observing the genotypes $g_i$ at locus i given a population allele frequency ($\pi$); and, $\Sigma g_i$ denotes summing over a plurality of possible combinations of genotypes of the contributors, subjecting to constraints of the known genotypes for some or all the contributors.

In some implementations, the prior joint probability is calculated using marginal distributions $P(g_{1i}|\pi)$ and $P(g_{2i}|\pi)$ that satisfy the Hardy-Weinberg equilibrium.

In some implementations, all genotypes are known, and the multi-loci likelihood function is computed using the genotype vector $g_i$ representing the known genotype combination for the contributors: $L(\beta, \theta, \lambda, \pi ; n_1, n_2) = \Pi_i [M(n_{1i}, n_{2i} \mid p(g_i, \lambda, \beta), \theta) \cdot P(g_i \mid \pi)]$.

In some implementations, the probabilistic mixture model accounts for nucleic acid molecule number errors resulting from extracting the nucleic acid molecules performed in 202, as well as the read count errors resulting from the sequencing operation in 206.

In some implementations, the probabilistic mixture model uses a second binomial distribution to model allele counts of the extracted nucleic acid molecules for alleles at the one or more polymorphism loci. In some implementations, the second binomial distribution is expressed as follows:

$$n_{1i}'' \sim BN(n_i'', p_{1i})$$

where $n_{1i}''$ is an allele count of extracted nucleic acid molecules for allele $1$ at locus $i$; $n_i''$ is a total nucleic acid molecule count at locus $i$, which equals to a total genome copy numbers $n''$; and $p_{1i}$ is a probability parameter indicating the probability of allele $1$ at locus $i$.

In some implementations, the first binomial distribution is conditioned on an allele fraction $n_{1i}''/n_i''$. In some implementations, the first binomial distribution is re-parameterized as follows:

$$n_{1i} \sim BN(n_i, n_{1i}''/n_i'')$$

where $n_{1i}$ is an allele count of nucleic acid sequence reads for allele $1$ at locus $i$.

In some implementations, the probabilistic mixture model uses a first beta distribution to approximate a distribution of $n_{1i}''/n''$. In some implementations, the first beta distribution has a mean and a variance that match a mean and a variance of the second binomial distribution.

In some implementations, locus $i$ is modeled as biallelic and the first beta distribution is expressed as follows:

$$n_{1i}''/n'' \sim Beta((n''-1)p_{1i}, (n''-1)p_{2i})$$

where $p_{1i}$ is a probability parameter indicating the probability of a first allele at locus $i$; and $p_{2i}$ is a probability parameter indicating the probability of a second allele at locus $i$.

In some implementations, the process includes combining the first binomial distribution, modeling sequencing read counts, and the first beta distribution, modeling extracted nucleic acid molecule number, to obtain the single-locus likelihood function of $n_{1i}$ that follows a first beta-binomial distribution.

In some implementations, the first beta-binomial distribution has the form:

$$n_{1i} \sim BB(n_i, (n''-1) \cdot p_{1i}, (n''-1) \cdot p_{2i}),$$

or an alternative approximation:

$$n_{1i} \sim BB(n_i, n'' \cdot p_{1i}, n'' \cdot p_{2i}).$$

In some implementations, the multiple-loci likelihood function can be expressed as:

$$L(\beta, n'', \lambda, \pi ; n_1, n_2) = \Pi_i \left[ \Sigma_{gi} BB(n_{1i} \mid n_i, (n''-1) \cdot p_{1i}, (n''-1) \cdot p_{2i}) \cdot P(g_i \mid \pi) \right]$$

where $L(\beta, n'', \lambda, \pi ; n_1, n_2)$ is the likelihood of observing allele count vectors $n_1$ and $n_2$ for alleles 1 and 2 at all loci, and $p_{1i} = p(g_i, \lambda, \beta)$, $p_{2i} = 1 - p_{1i}$.

In some implementations, the contributors include two contributors, and the multiple-loci likelihood function is expressed as:

$$L(\beta, n'', \lambda, \pi ; n_1, n_2) = \Pi_i \Sigma_{g1ig2i} BB(n_{1i}, n_{2i} \mid n_i, (n''-1) \cdot p_{1i}(g_{1i}, g_{2i}, \lambda, \beta), (n''-1) \cdot p_{2i}(g_{1i}, g_{2i}, \lambda, \beta)) \cdot P(g_{1i}, g_{2i} \mid \pi)$$

where $L(\beta, n'', \lambda, \pi ; n_1, n_2)$ is the likelihood of observing an allele count vector for the first allele of all loci ($n_1$) and an allele count vector for the second allele of all loci ($n_2$) given parameters $\beta$, $n''$, $\lambda$, and $\pi$; $p_{1i}(g_{1i}, g_{2i}, \lambda, \beta)$ is a probability parameter, taken as $p_1'$ from Table 3, indicating a probability of allele $1$ at locus $i$ based on the two contributors' genotypes ($g_{1i}, g_{2i}$); $p_{2i}(g_{1i}, g_{2i}, \lambda, \beta)$ is a probability parameter, taken as $p_2'$ from Table 3, indicating a probability of allele 2 at locus $i$ based on the two contributors' genotypes ($g_{1i}, g_{2i}$); and $P(g_{1i}, g_{2i} \mid \pi)$ is a prior joint probability of observing the first contributor's genotype for the first allele ($g_{1i}$) and the second contributor's genotype for the first allele ($g_{2i}$) at locus $i$ given a population allele frequency ($\pi$).

In some implementations, operation 214 includes estimating the total extracted genome copy number $n''$ from a mass of the extracted nucleic acid molecules. In some implementations, the estimated total extracted genome copy number $n''$ is adjusted according to fragment size of the extracted nucleic acid molecules as further described hereinafter.

In some implementations, the probabilistic mixture model accounts for nucleic acid molecule number errors resulting from amplifying the nucleic acid molecules performed in 204, as well as the read count errors resulting from the sequencing operation in 206. In some implementations, the nucleic acid amplification process is modeled as follows:

$$x_{t+1} = x_t + y_{t+1}$$

wherein $x_{t+1}$ is the nucleic acid copies of a given allele after cycle $t+1$ of amplification; $x_t$ is the nucleic acid copies of a given allele after cycle $t$ of amplification; $y_{t+1}$ is the new copies generated at cycle $t+1$, and it follows a binomial distribution $y_{t+1} \sim BN(x_t, r_{t+1})$; and $r_{t+1}$ is the amplification rate for cycle $t+1$.

In some implementations, the probabilistic mixture model uses a second beta distribution to model allele fractions of the amplified nucleic acid molecules for alleles at the one or more polymorphism loci. In some implementations, locus $i$ is modeled as biallelic and the second beta distribution is expressed as follows:

$$n_{1i}' / (n_{1i}' + n_{2i}') \sim Beta(n'' \cdot \rho_i \cdot p_{1i}, \ n'' \cdot \rho_i \cdot p_{2i})$$

where $n_{1i}'$ is an allele count of amplified nucleic acid molecules for a first allele at locus $i$; $n_{2i}'$ is an allele count of amplified nucleic acid molecules for a second allele at locus $i$; $n''$ is a total nucleic acid molecule count at any locus; $\rho_i$ is a constant related to an average amplification rate $r_i$ over all amplification cycles; $p_{1i}$ is the probability of the first allele at locus $i$; and $p_{2i}$ is the probability of the second allele at locus $i$. In some implementations, $\rho_i$ is $(1+r_i)/(1-r_i)/[1-(1+r_i)^{-t}]$. In some implementations, $\rho_i$ is approximated as $(1 +r_i)/(1-r_i)$.

In some implementations, operation 214 includes combining the first binomial distribution and the second beta distribution to obtain the single-locus likelihood function for $n_{1i}$ that that follows a second beta-binomial distribution. In some implementations, the second beta-binomial distribution has the form:

$n_{1i} \sim BB(n_i, n'' \cdot \rho_i \cdot p_{1i}, n'' \cdot \rho_i \cdot p_{2i})$, wherein $n_{1i}$ is an allele count of nucleic acid sequence reads for the first allele at locus $i$; $p_{1i}$ is a probability parameter indicating the probability of a first allele at locus $i$; and $p_{2i}$ is a probability parameter indicating the probability of a second allele at locus $i$.

In some implementations, operations 214 includes, by assuming the one or more polymorphism loci have a same amplification rate, re-parameterizing the second beta-binomial distribution as:

$n_{1i} \sim BB(n_i, n'' \cdot (1+r)/(1-r) \cdot p_{1i}, n'' \cdot (1+r)/(1-r) \cdot p_{2i})$, wherein $r$ is an amplification rate.

In some implementations, operation 214 includes quantifying the one or more fractions of nucleic acid of the one or more contributors in the nucleic acid sample using a multiple-loci likelihood function obtained using the second beta-binomial distribution, the multiple-loci likelihood function is as follows:

$$L(\beta, n'', r, \lambda, \pi \; ; n_1, n_2) = \Pi i \left[ \Sigma g_i \ BB(n_{1i} \mid n_i, n'' \cdot (1+r)/(1-r) \cdot p_{1i}, n'' \cdot (1+r)/(1-r) \cdot p_{2i}) \bullet P(g_i \mid \pi) \right]$$

In some implementations, the contributors include two contributors and the multiple-loci likelihood function comprises:

[00253] $$L(\beta, n'', r, \lambda, \pi \; ; n_1, n_2) = \Pi_i \Sigma_{g1ig2i} \ [BB(n_{1i} \mid n_i, n'' \cdot (1+r)/(1-r) \cdot p_{1i}(g_{1i}, g_{2i}, \lambda, \beta), n'' \cdot (1+r)/(1-r) \cdot p_{2i}(g_{1i}, g_{2i}, \lambda, \beta)) \cdot P(g_{1i}, g_{2i} \mid \pi)]$$

wherein $L(\beta, n'', r, \lambda, \pi \; ; n_1, n_2)$ is the likelihood of observing an allele count vector for the first allele of all loci ($n_1$) and an allele count vector for the second allele of all loci ($n_2$) given parameters $\beta$, $n''$, $r$, $\lambda$, and $\pi$.

In some implementations, operation 214 includes, by defining a relative amplification rate of each polymorphism locus to be proportional to a total reads per locus, re-parameterizing the second beta-binomial distribution as:

$n_{1i} \sim BB(n_i, c' \cdot n_i \cdot p_{1i}, c' \cdot n_i \cdot p_{2i})$, wherein $c'$ is a parameter to be optimized.

In some implementations, operation 214 includes quantifying the one or more fractions of nucleic acid of the one or more contributors in the nucleic acid sample using a multiple-loci likelihood function obtained using the second beta-binomial distribution, the multiple-loci likelihood function follows:

$$L(\beta, n'', c', \lambda, \pi \; ; n_1, n_2) = \Pi i \left[ \Sigma g_i \ BB(n_{1i} \mid n_i, c' \cdot n_i \cdot p_{1i}, c' \cdot n_i \cdot p_{2i}) \bullet P(g_i \mid \pi) \right]$$

In some implementations, the probabilistic mixture model accounts for nucleic acid molecule number errors resulting from extracting the nucleic acid molecules performed in 202 and amplifying the nucleic acid molecules performed in 204, as well as the read count errors resulting from the sequencing operation in 206.

In some implementations, the probabilistic mixture model uses a third beta distribution to model allele fractions of the amplified nucleic acid molecules for alleles at the one or more polymorphism loci, accounting for the sampling errors resulting from extracting the nucleic acid molecules performed in 202 and amplifying the nucleic acid molecules performed in 204. In some implementations, locus $i$ is modeled as biallelic and the third beta distribution has the form of:

$$n_{1i}' / (n_{1i}' + n_{2i}') \sim Beta(n'' \cdot (1+ r_i)/2 \cdot p_{1i}, \ n'' \cdot (1+ r_i)/2 \cdot p_{2i})$$

where $n_{1i}'$ is an allele count of amplified nucleic acid molecules for a first allele at locus $i$; $n_{2i}'$ is an allele count of amplified nucleic acid molecules for a second allele at locus $i$; $n''$ is a total nucleic acid molecule count; $r_i$ is the average amplification rate for locus $i$; $p_{i1}$ is the probability of the first allele at locus $i$; and $p_{2i}$ is a probability of the second allele at locus $i$.

In some implementations, operation 214 includes combining the first binomial distribution and the third beta distribution to obtain the single-locus likelihood function of $n_{1i}$ that follows a third beta-binomial distribution. In some implementations, the third beta-binomial distribution has the form:

$$n_{1i} \sim BB(n_i,\ n'' \cdot (1+ r_i )/2 \cdot p_{1i},\ n'' \cdot (1+ r_i )/2 \cdot p_{2i})$$

where $r_i$ is an amplification rate.

In some implementations, the multiple-loci likelihood function is:

$$L(\beta,\ n'',\ r,\ \lambda,\ \pi\ ;\ n_1,\ n_2) = \Pi_i\ [\Sigma g_i\ BB(n_{1i}\ |\ n_i,\ n'' \cdot (1+ r )/2 \cdot p_{1i},\ n'' \cdot (1+ r )/2 \cdot p_{2i})$$
$$\cdot\ P(g_i\ |\ \pi)]$$

where r is an amplification rate assumed to be equal for all loci.

In some implementations, the contributors include two contributors, and the multiple-loci likelihood function is:

$$L(\beta,\ n'',\ r,\ \lambda,\ \pi\ ;\ n_1,\ n_2) = \Pi_i\Sigma_{g1ig2i}\ BB(n_{1i}\ |\ n_i,\ n'' \cdot (1+ r )/2 \cdot p_{1i}(g_{1i},\ g_{2i},\ \lambda,\ \beta),\ n'' \cdot$$
$$(1+ r )/2 \cdot p_{2i}(g_{1i},\ g_{2i},\ \lambda,\ \beta))\ \cdot P(g_{1i},\ g_{2i}|\pi)$$

where $L(n_1, n_2|\ \beta, n'', r, \lambda, \pi)$ is the likelihood of observing allele counts for the first allele vector $n_1$ and an allele count for the second allele vector $n_2$ given parameters $\beta, n'', r, \lambda$, and $\pi$.

In some implementations, process 200 further includes estimating, using the Cramer-Rao inequality, one or more confidence intervals of the one or more fractions of nucleic acid of the one or more contributors.

In some implementations, the mapping operation of 208 includes identifying reads among the nucleic acid sequence reads matching any sequence of a plurality of unbiased target sequences, wherein the plurality of unbiased target sequences includes sub-sequences of the reference sequence and sequences that differ from the subsequences by a single nucleotide.

In some implementations, the plurality of unbiased target sequences comprises five categories of sequences encompassing each polymorphic site of a plurality of polymorphic sites: (i) a reference target sequence that is a sub-sequence of the reference sequence, the reference target sequence having a reference allele with a reference nucleotide at the polymorphic site; (ii) alternative target sequences each having an alternative allele with an alternative nucleotide at the polymorphic site, the alternative nucleotide being different from the reference nucleotide; (iii) mutated reference target sequences comprising all possible sequences that each differ from the reference target sequence by only one nucleotide at a site that is not the polymorphic site; (iv) mutated alternative target sequences comprising all possible sequences that each differ from an alternative target sequence by only one nucleotide at a site that is not the polymorphic site; and (v) one or more unexpected allele target sequences each having an unexpected allele different from the reference allele and the alternative allele, and each having a sequence different from the previous four categories of sequences. In some implementations, the five categories of sequences have the same length and are located at the same region of a genome.

In some implementations, operation 208 includes using the identified reads and their matching unbiased target sequences to determine allele counts of the nucleic acid sequence reads for the alleles at the one or more polymorphism loci. In some implementations, the plurality of unbiased target sequences includes sequences that are truncated to have the same length as the nucleic acid sequence reads. In some implementations, the plurality of unbiased target sequences includes sequences stored in one or more hash tables, and the reads are subsequently identified using the hash tables.

In some implementations, the process 200 further includes a procedure to determine if a contributor of known genotype is a true contributor to a mixture sample by comparing two versions of maximized multi-loci likelihood values, one version using a genotype matrix containing the known genotype for the contributor, another version using a genotyping matrix with unknown genotype for the contributor.

In some implementations, the process further includes determining one or more genotypes of the one or more contributors at the one or more polymorphism loci. In some implementations, the process includes determining, using the one or more fractions of nucleic acid of the one or more contributors, a risk of one contributor (a donee) rejecting a tissue or an organ transplanted from another contributor (a donor). In many applications, the risk is not or cannot be based solely on the estimated contributor fractions. Instead, the contributor fractions are used as an intermediate parameter or intermediate result for determining the risk. In various implementations, other parameters obtained from other methods are combined with the contributor fractions to determine the risk. Such other methods include, and are not limited to, tissue biopsy, serum creatinine measurement, HLA-DSA (donor specific antibody) analysis.

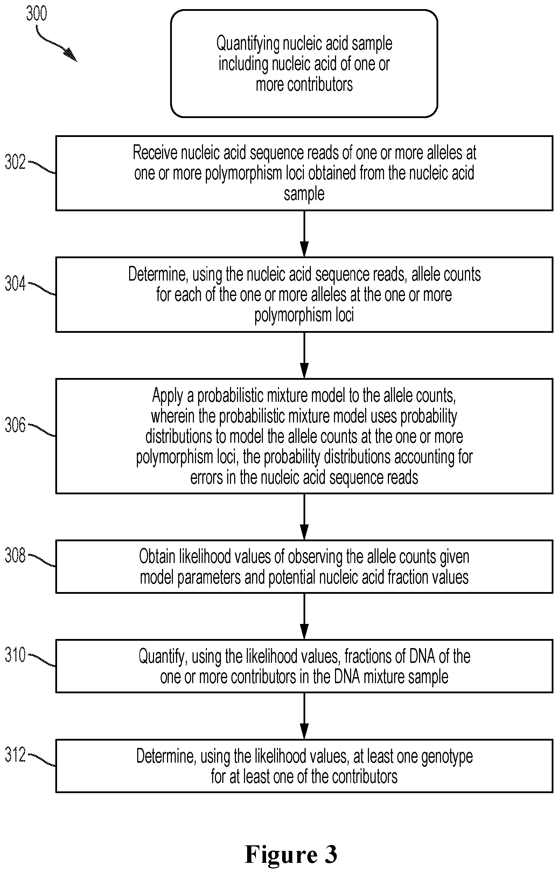

Figure 3 shows a block diagram illustrating process 300 for evaluating a nucleic acid sample including nucleic acid of one or more contributors. Process 300 starts by receiving nucleic acid sequence reads of one or more alleles at one or more polymorphism loci obtained from the nucleic acid sample. See block 302. In some implementations, the nucleic acid sequence reads were obtained by sequencing the nucleic acid in the nucleic acid sample using various techniques described herein.

In some implementations, unique molecular indexes (UMIs) are attached to the extracted nucleic acid molecules, which are then amplified, sequenced, and mapped to the polymorphism loci or alleles. The unique molecular indices provide mechanisms to reduce the errors that can occur in the sample processing and analysis steps. For instance, different reads sharing a same unique molecular index (UMI) can be combined or collapsed to determine a sequence from which the reads are derived, effectively removing errors that have occurred during sample processing and sequencing. US Patent Application No. 15/130,668, filed April 16, 2016, and US Patent Application No. 15/863,737, filed January 5, 2018 describe various methods and systems for sequencing nucleic acids using unique molecular indexes.

When UMI is used in an assay, the redundant DNA molecules resulting from PCR amplification of a template nucleotide acid are collapsed into a single read. For such experimental procedure, a preferred model for single locus read counts is the first beta-binomial distribution, which combined the first binomial distribution, modeling sequencing read counts, and the first beta distribution, modeling extracted nucleic acid molecule number.

When UMI is not used in an assay, nucleic acid extraction, amplification, and sequencing all contribute to the statistical variability in read counts. For such experimental procedure, a preferred model for single locus read counts is the third beta-binomial distribution, which combined the first binomial distribution, modeling sequencing read counts, the third beta distribution, modeling allele fractions of the amplified nucleic acid molecules, and the first beta distribution, modeling allele fractions in extracted nucleic acid molecule.

Process 300 further involves determining, using the nucleic acid sequence reads, allele counts for the one or more alleles at the one or more polymorphism loci.

Process 300 also involves applying the probabilistic mixture model to the allele counts. The probabilistic model uses probabilistic distributions to model allele counts of alleles at the one or more polymorphism loci. The probabilistic distributions count for errors in the allele data. The errors include errors originating from nucleic acid extraction, sample processing, and sequencing operations.

In some implementations, the probabilistic distributions include a first binomial distribution. In some implementations, the first binomial distribution includes a parameter indicating the total allele count at a locus and a probability parameter indicating the probability of the first allele at the locus. In some implementations, the probability parameter is a function of the fractions of nucleic acid of the one or more contributors in the nucleic acid sample. The probability parameter is also a function of genotypes of the one or more contributors, or G, and a function of errors in the nucleic acid sequence read data, or $\theta$. In some implementations, the errors in the read data include errors originating from nucleic acid extraction, sample processing, and sequencing operations.

Process 300 also involves obtaining likelihood values of observing the allele data given model parameters and potential nucleic acid fraction values. See block 308.

In some implementations, process 300 involves quantifying, using the likelihood values, fractions of nucleic acid of the one or more contributors in the nucleic acid sample. See block 310.

In some implementations, process 300 further involves determining, using the likelihood values, at least one genotype for at least one of the contributors. See block 312.

In some implementations, genotypes of the contributors were unknown prior to process 300.

In some implementations, the probabilistic mixture model uses a beta distribution to model the errors in the allele data. In some implementations, the beta distribution is defined by a mean parameter and a concentration parameter. In some implementations, the concentration parameter has discrete prior representing different noise conditions. The concentration parameter varies across loci.

In some implementations, the quantification of operation 310 includes combining the first binomial distribution and the beta distribution to obtain a marginal distribution that follows a beta-binomial distribution.

In some implementations, the quantification of 310 includes quantifying the fractions of nucleic acid of the one or more contributors in the nucleic acid sample using a multiple-loci likelihood function of the allele data. In some implementations, the quantification involves calculating a plurality of likelihood values using a plurality of potential fraction values and a multiple-loci likelihood function of the allele counts. The quantification also involves identifying a potential fraction vector associated with the maximum likelihood value, and quantifying the fractions of nucleic acid of the one or more contributors in the nucleic acid sample using the identified potential fraction vector.

In some implementations, the multiple-loci likelihood function depends on P(G|$\pi$), which is a prior probability of the genotype of the one or more contributors given a population allele frequency ($\pi$). In some implementations, the prior probability is calculated considering a dummy allele with a fixed prior probability representing mechanistic drop-out.

In some implementations, the one or more contributors include two or more contributors. In some implementations, process 300 includes an operation of determining a total number of contributors in the one or more contributors. In some implementations, one or more genotypes of the one or more contributors were unknown, and process 300 includes an operation of determining an allele configuration at each of the one or more polymorphism loci, the allele configuration comprising an allele for each of the one or more contributors. In some implementations, process 300 includes an operation of determining an estimated probability for the allele configuration.

In some implementations, process 300 further includes obtaining a posterior probability that a specific contributor among the one or more contributors has a specific genotype. In some implementations, process 300 further includes calling, based on the posterior probability, that the nucleic acid sample includes nucleic acid from the specific contributor. In some implementations, obtaining the posterior probability that a specific contributor among the one or more contributors has a specific genotype includes: (i) multiplying prior probabilities of genotype configurations by likelihoods of the genotype configurations; (ii) normalizing a product of (i) by a sum over genotype space; and (iii) summing over genotype configurations containing the specific genotype to obtain the posterior probability.

In some implementations, the specific genotype includes a multiple-locus genotype, and the method further includes: summing, over all contributors, a posterior probability that a contributor has the specific genotype at all loci; and determining, based on the summed probability, the specified multiple-locus genotype appears in any contributor.

In some implementations, the nucleic acid sample is a forensic sample and the data of the multiple-locus genotype is obtained from a person of interest. The process further includes determining that the person of interest is a contributor of the nucleic acid sample.

In some implementations, the probabilistic mixture model uses a second binomial distribution to model stutter errors in the allele data. In some implementations, the second binomial distribution is expressed as follows:

$$s_{ik} \sim BN(n_{i(k+1)},\ r_i)$$

where $s_{ik}$ is a stutter allele count at locus $i$ of a stutter allele that appears to be allele $k$ but actually results from a stutter error of allele $k+1$; $n_{i(k+1)}$ is an original allele count of allele $k+1$ at locus $i$; and $r_i$ is a stutter rate for locus $i$.

In some implementations, the stutter rate r varies across loci and has a prior representing different noise conditions, the prior being shared across loci.

In some implementations, operation 310 includes quantifying fractions of nucleic acid of the one or more contributors in the nucleic acid sample using a multiple-loci likelihood function including a product of likelihoods of non-stutter allele counts and likelihoods of stutter allele counts.

In some implementations, applying the probabilistic mixture model includes adding a fixed number of molecules to an allele count assigned to allele $k+1$ when determining a number of molecules from which stutter can potentially originate.

In some implementations, the probabilistic mixture model uses a dummy out-of-sample allele to model natural drop-out. In some implementations, the prior of the dummy out-of-sample allele is proportional to a number of unobserved alleles. In some implementations, the number of unobserved alleles is estimated by: interpolating all integers between the shortest and longest observed integer-valued alleles, adding any observed non-integer-valued alleles, and returning the maximum of the resulting value and a criterion value.

In some implementations, applying the probabilistic mixture model involves pruning genotype configurations from data used to quantify the fractions of nucleic acid of the one or more contributors in the nucleic acid sample. In some implementations, pruning genotype configurations involves: limiting genotype configurations that are plausible by constructing a list of required alleles and excluding loci with not enough contributors to explain all required alleles. In some implementations, the list of required alleles consists essentially of alleles having allele counts above a threshold and too high to be plausible due to stutter drop-in. In some implementations, the threshold is a sum of (i) a maximum non-stutter allele count, and (ii) a value multiplied by a count of potential stutter donor alleles. In some implementations, pruning genotype configurations involves removing genotype configurations that have poor matches between the allele data and expected allele counts. In some implementations, the genotype configurations that have poor matches have root mean squared error (RMSE) values larger than one or more thresholds.

In some implementations, the alleles at the one or more polymorphism loci include single nucleotide polymorphism (SNP) alleles and/or short tandem repeat (STR) alleles.

## Method for Unbiased Mapping of Reads to Marker Sites

Conventional computational methods for mapping nucleic acid (e.g., DNA or RNA) sequencing reads to the genome can be biased by the reference genome used. Since only one allele (the reference allele) for each variant site is present in the reference genome, mismatches between the reads and references are treated as sequencing errors in existing read mapping strategies. The problem is that when reads containing the non-reference alleles are treated as containing sequencing errors, the alignment confidence (score) is decreased, and hence they are less likely to be retained as confidently mapped reads in subsequent filtering steps. This mapping bias will skew the allele counts (Figure 1B), and subsequently compromise the estimation of contributor DNA fractions.

To address the mapping bias issue and enable optimal CDQ, some implementations provide a novel workflow for mapping reads to variant sites. The new read mapping approach enables unbiased counting of alleles and estimation of sequencing error on variant sites and non-variant sites.

The read mapping workflow is as follows. The workflow first generates five types of sequences (see Table 1) based on 1) the reference sequences and 2) the known alleles of the variant sites. If more than one single mutation is allowed per sequence, more types of sequences will be generated. The five types of sequences are referred to as ref, alt, ref.mut, alt.mut, and snp.mut respectively. For example, for each biallelic SNP marker site covered by a target sequence of length L, there are one ref, one alt, [L - 1] x 3 ref.mut, [L - 1] x 3 alt.mut, and 2 snp.mut sequences. All five types of sequences are then included in the database of "unbiased target sequences" (Figure 1B). Depending on the length of the reads from the sequencer, the unbiased target sequences are then truncated into two versions. Let r be the read length. Version 1 of the truncated target sequences comprises the r 5' bases of all unbiased target sequences, while version 2 of the truncated target sequences comprises the reverse complement of the r 3' bases of all unbiased target sequences. Redundant sequences in truncated target sequences are then removed. The unique sequences in the two truncated sequence databases are then recorded into two hash tables. Next, sequencing reads are counted using the hash tables. For pair end sequencing strategies, R1 reads and R2 reads are counted using the first and second hash tables respectively. For non-pair end sequencing, all reads are counted using the first hash table. Finally, for each marker site, the counts are aggregated into the five types defined above depending on which type the truncated unbiased target sequences corresponds to in Table 1.

A similar strategy can be implemented when sequence alignment tools are used instead of using hash table for the mapping. For each marker site, the ref and alt types of sequences are generated to form the unbiased sequence database. Each sequencing read is then aligned to this database with up to a predefined number of sequencing errors. The mapped reads are then categorized based on Table 1. For SNP markers only the bi-allelic scenario is presented here, but the method extends to multi-allelic loci.

**Table 1. Definition of five types of target sequences to be generated from the reference sequence around a variant site.**

| Type | Definition |
|---|---|
| ref | SNP site taking reference allele |
| alt | SNP site taking alternative allele |
| ref.mut | Single mutation on non SNP site when the SNP site is ref |
| alt.mut | Single mutation on non SNP site when the SNP site is alt |
| snp.mut | SNP site taking neither reference nor alternative alleles |

The proposed read mapping workflow addresses the read mapping bias issue when tested using real data. With the workflow, the observed error rates of the reference to alternative errors and the alternative to reference errors are identical. The sequencing error rate on the non-variant sites on the reference DNA copy and that on the alternative DNA copy are also identical.

**Linking Contributor DNA Fraction with Allele Fractions**

*Sequencing Error-Free Scenario*

We denote $n_1$ as the number of contributor 1 (e.g. organ recipient) cells and $n_2$ as the number of contributor 2 (e.g. organ donor) cells that supplied DNA to the sample. Based on these cells, the implementations define the contributor 2 fraction as $\beta_2 = n_2/(n_1+ n_2)$. For two-contributor scenario, we denote $\beta_2$ as $\beta$ for short. Depending on the genotypes of the two contributors at each specific locus, the two alleles have different fractions (see Table 2 for details), and the generic formula for calculating them is $p_1 = [g_{11}(1-\beta) + g_{21}\cdot\beta] /2$ and $p_2 = [g_{12} (1-\beta) + g_{22}\cdot\beta] /2$. Note that $g_{11}$ and $g_{12}$ are the contributor 1 (recipient) genotype, i.e. copies of allele 1 and 2 in the recipient genome; $g_{21}$ and $g_{22}$ are contributor 2 (donor) genotype, i.e. copies of allele 1 and 2 in the donor genome.

In matrix notation, the relationship for multiple contributor cases is generally implemented as $\boldsymbol{p} \leftarrow \boldsymbol{g}/2\bullet \beta$, where $\boldsymbol{p}$

is a vector of expected allele 1 fraction for all loci, $g$ is a matrix of genotype of all loci in all contributor, and $\beta = [\beta_1, \beta_2, ..., \beta_D]$ is the vector of nucleic acid fractions for all constructors. The implementation is generally applied to single-, two-, and multi-contributor scenarios.

**Table 2: The binomial model parameters expected allele 1 and allele 2 fractions $p_1$ and $p_2$ for the 9 possible genotype combinations between a contributor 1 and contributor 2 pair for a given variant site**

| $g_{11}$ | $g_{21}$ | $p_1$ | $p_2$ |
|---|---|---|---|
| 0 | 0 | 0 | 1 |
| 0 | 1 | $\beta/2$ | $1-\beta/2$ |
| 0 | 2 | $\beta$ | $1-\beta$ |
| 1 | 0 | $(1-\beta)/2$ | $(1+\beta)/2$ |
| 1 | 1 | $1/2$ | $1/2$ |
| 1 | 2 | $(1+\beta)/2$ | $(1-\beta)/2$ |
| 2 | 0 | $1-\beta$ | $\beta$ |
| 2 | 1 | $1-\beta/2$ | $\beta/2$ |
| 2 | 2 | 1 | 0 |

### *General Scenario with Sequencing Error*

When there are two known alleles at a variant site, sequencing errors will convert one allele to another in addition to converting the two known alleles to the two remaining nucleotides at this locus. The consequence is that the allele fractions in the sequenced reads will deviate from the allele fractions in the NGS input sample.

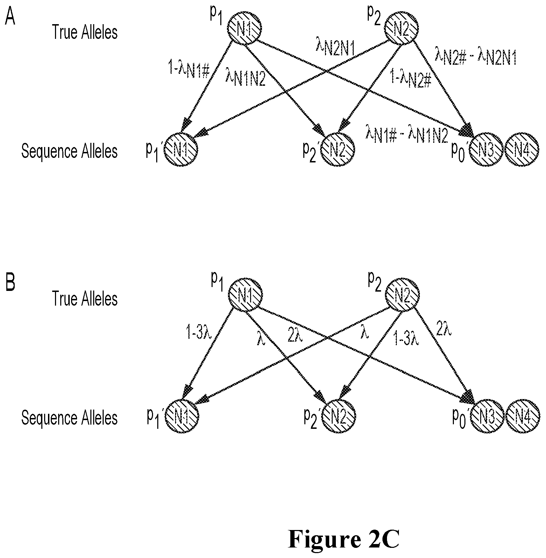

Figure 2C schematically illustrates sequencing errors that convert one allele to another allele and true alleles to unexpected alleles. Panel (A) shows nucleotide-dependent sequencing error, and panel (B) shows uniform sequencing error.

Let $N_1$, $N_2$ be the allele 1 and allele 2 nucleotides. Let $p_1'$, $p_2'$ be the probability of observing allele 1 and allele 2 reads respectively, whether it is real or due to sequencing error; and $p_0' = 1 - p_1' - p_2'$ be the probability of observing the two unexpected alleles due to sequencing error. Let $\lambda_{N1N2}$ be the mutation rate (probability) from $N_1$ to $N_2$, where $N_1$ and $N_2$ are unique to each SNP site, and

$\lambda_{N1\#}$: mutation probability from $N_1$ to any of the 3 nucleotide non-$N_1$ nucleotides.

The transition diagram among the 4 nucleotide of a SNP site is shown in Figure 2C. Based on this, the implementations obtain the following equations for converting from true allele fractions $p_1$, $p_2$ to observed allele fractions $p_1'$, $p_2'$, and $p_0'$:

$$p_1' = p_1 - p_1 \cdot \lambda_{N1\#} + p_2 \cdot \lambda_{N2N1}$$

$$p_2' = p_2 - p_2 \lambda_{N2\#} + p_1 \cdot \lambda_{N1N2}$$

$$p_0' = p_1 \cdot (\lambda_{N1\#} - \lambda_{N1N2}) + p_2 \cdot (\lambda_{N2\#} - \lambda_{N2N1}).$$

When the implementations assume uniform sequencing error rate that is independent to the nucleotide identity, the implementations have,

$$p_1' = p_1 \cdot (1 - 3 \cdot \lambda) + p_2 \cdot \lambda$$

$$p_2' = p_2 \cdot (1 - 3 \cdot \lambda) + p_1 \cdot \lambda$$

$$p_0' = 2\lambda.$$

When the implementations ignore the unexpected alleles

$$p_1' = (p_1 \cdot (1 - 3 \cdot \lambda) + p_2 \cdot \lambda)/(1-2\lambda)$$

$$p_2' = (p_2 \cdot (1 - 3 \cdot \lambda) + p_1 \cdot \lambda)/(1-2\lambda),$$

with $o(\lambda^2)$ approximation error, these are rewritten as

$$p_1' = p_1 \cdot (1 - \lambda) + p_2 \cdot \lambda$$

$$p_2' = p_2 \cdot (1 - \lambda) + p_1 \cdot \lambda$$

Or for locus $i$ and substituting g and $\beta$ for $p$:

$$p_{1i}' \leftarrow \Sigma_d [(g_{d1i} \cdot (1-\lambda) + g_{d2i} \cdot \lambda] \cdot \beta_d)/2$$

$$p_{2i}' \leftarrow \Sigma_d [(g_{d2i} \cdot (1-\lambda) + g_{d1i} \cdot \lambda] \cdot \beta_d)/2$$

which is referred to as an error-adjusted-genotype weighted mixing coefficients.

The formula linking contributor 2 fraction $\beta$ with the observed allele fraction $p_1'$ in two contributor scenario is listed in Table 3.

**Table 3: Expected probabilities of observing alleles 1 and 2 allowing for sequencing errors, conditioned on each donor/recipient genotype combination in a two-contributor setting. Here a uniform sequencing error rate $\lambda_{N1N2} = \lambda$ is used for all nucleotide pairs $N_1$ and $N_2$. Since mutation rate $\lambda$ is small, a first order approximation is used.**

| $g_{11}$ | $g_{21}$ | $p_1'$ | $p_2'$ |
|---|---|---|---|
| 0 | 0 | $\lambda$ | $1-\lambda$ |
| 0 | 1 | $\beta/2 + \lambda - \beta\lambda$ | $1 - \beta/2 - \lambda + \beta\lambda$ |
| 0 | 2 | $\beta+\lambda-2\beta\lambda$ | $1 - \beta - \lambda + 2\beta\lambda$ |
| 1 | 0 | $(1-\beta)/2 + \beta\lambda$ | $(1+\beta)/2 - \beta\lambda$ |
| 1 | 1 | $1/2$ | $1/2$ |
| 1 | 2 | $(1+\beta)/2 - \beta\lambda$ | $(1-\beta)/2 + \beta\lambda$ |
| 2 | 0 | $1 - \beta - \lambda + 2\beta\lambda$ | $\beta + \lambda - 2\beta\lambda$ |
| 2 | 1 | $1 - \beta/2 - \lambda + \beta\lambda$ | $\beta/2 + \lambda - \beta\lambda$ |
| 2 | 2 | $1 - \lambda$ | $\lambda$ |

In matrix format, error-adjusted-genotype for allele 1 accounting for sequencing error $\lambda$ is implemented as: G $\leftarrow$ [(1- $\lambda$) $g$ + $\lambda$ (2- $g$)] / 2

For general cases with more than two contributors, the expected mixing fraction vector for allele 1 is computed as: $p \leftarrow G \cdot \beta$, which is implemented for nucleic acid mixtures with single, two, or multiple contributors.

When $\lambda$ = 0, the implementation has the special case: $p \leftarrow g/2 \cdot \beta$

### Overview of the DNA Extraction, PCR (Amplification), and *Sequencing* Models

Three probabilistic models (Figure 1C) are provided to model the three major components in the generic experimental pipeline (Figure 1A): 1) DNA/RNA extraction; 2) DNA/RNA amplification (e.g., PCR) as an approach for enriching target DNA/RNA; 3) sequencing (e.g., NGS sequencing). These and other modeling components are then integrated to implement the single-locus model and compute the single-locus likelihood function $M(n_{1i}, n_{2i} \mid p_{1i}, \theta)$.

The following notations are used in the mathematical models detailed in Table 4 and the remaining of this section.

B(): beta function

Beta(), BN(), Pois(), Gamma(): beta distribution, binomial distribution, Poisson distribution, and Gamma distribution

NB() denotes a negative binomial distribution, which is a discrete probability distribution of the number of successes in a sequence of independent and identically distributed Bernoulli trials before a specified (non-random) number of failures (denoted r) occurs.

**Table 4: Statistical models for the three major components in the generic experiment pipeline (Figure 1). The model for each component is conditioned on the previous component. The models are per each locus and locus index i is omitted.**

| gDNA or cfDNA extracted from a blood sample (Model E) | Copies of allele 1: $n_1'' \sim Pois(c \cdot p_1)$ | Copies of allele 2: $n_2'' \sim Pois(c \cdot p_2)$ |
|---|---|---|
| | Copies of allele 1 given the total copies of the locus: $n_1'' \mid n'' \sim BN(n'', p_1)$, where $n'' = n_1'' + n_2''$. | |
| PCR amplified DNA (Model P) | Copies of allele 1: $n_1' \sim Gamma(n_1'' \cdot \rho, \theta)$ | Copies of allele 2: $n_2' \sim Gamma(n_2'' \cdot \rho, \theta)$ |
| | Fraction of allele 1 of the locus in the PCR product, conditioning on allele 1 and allele 2 copies in the extracted DNA: $n_1'/n' \mid n_1'', n_2'' \sim Beta(n_1'' \cdot \rho, n_2'' \cdot \rho)$, where $n' = n_1' + n_2'$. Ignoring DNA sampling variation (hence $n_1'' = n'' \cdot p_1$, $n_2'' = n'' \cdot p_2$): $n_1'/n' \sim Beta(n'' \cdot \rho \cdot p_1, n'' \cdot \rho \cdot p_2)$ | |
| Copies of sequenced reads mapped to the loci (without sequencing error) (Model S) | Copies of allele 1 given the total copies of the locus, conditioning on fraction of allele 1 of a locus in the PCR product: $n_1 \mid n, n_1'/n' \sim BN(n, p = n_1'/n')$, where $n = n_1 + n_2$. | |

### *DNA Extraction Model: Model E*

When cfDNA or cellular DNA is extracted from a blood sample, the obtained DNA is a small sample from the large pool of DNA, and hence the implementations model the counts of two alleles at each locus as two Poisson distributions. Hence the DNA copy ($n_1''$) for allele 1 at a locus conditioned on the total counts $n''$ follows the binomial distribution: $n_1'' \sim BN(n'', p_1)$, with mean $\mu_0 = n'' \cdot p_1$ and variance $\delta_0^2 = n'' \cdot p_1 \cdot p_2$.

When gDNA is extracted from a sample, the resulting gDNA amount for each locus can again be variable due to extraction losses. Viewing $p_1$ as the fraction of allele 1 in the input sample, the amount of allele 1 in the extracted DNA can again be modeled by a binomial distribution: $n_1'' \sim BN(n'', p_1)$.

### *PCR Amplification Model: Model P*

We model the PCR amplification process as a stochastic process in order to obtain a probabilistic distribution of allele 1 counts in the PCR product. Let $x_t$ be the DNA copies of a given allele after cycle t of PCR amplification, let $r_t$ be the amplification rate for cycle t, and let $y_t$ be the new copies generated at cycle t. By assuming each piece of DNA has a probability $r_t$ of getting amplified and added to the DNA pool, the implementations have the following model for amplification:

$x_{t+1} = x_t + y_{t+1}$, where $y_{t+1} \sim BN(x_t, r_{t+1})$ follows a binomial distribution with $x_t$ and $r_{t+1}$ as parameters.

Based on this model, the implementations assume that the DNA copy number for a locus in the PCR product follows the Gamma distribution approximately. Below is the justification.

Step 1: Using Yule process (a continuous time stochastic process) to approximate PCR (a discrete time stochastic process).

The PCR process $x_{t+1} = x_t + y_{t+1}$, where $y_{t+1} \sim BN(x_t, r_{t+1})$ is a discrete time pure-birth process: in a given cycle of time t, each copy of DNA "gives birth" independently at some rate $r_t$. The continuous time version of the pure-birth process is well-known as the Yule-Furry Process. For the continuous time birth process, the final copy number for a locus at a given time t is known to follow a negative binomial distribution. The implementations can use the same distribution to approximate the discrete time birth process, when the total number of PCR cycles is not close to 1.

Step 2: Using Gamma distribution (a continuous distribution) to approximate negative binomial distribution (a discrete distribution).

A negative binomial random variable can be written as a sum of independent and identically distributed (i.i.d.) geometric random variables. The exponential distribution is known to be the continuous version of the geometric distribution. Hence, the sum of i.i.d. exponential random variables, which follows the Gamma distribution, is the continuous version of the sum of binomial random variable, which is negative binomial.

Below the implementations that estimate the parameters of the Gamma distributions of the allele counts in the PCR products.

Based on the law of total variance $var(x_{t+1}) = var(E(x_{t+1}|x_t) + E(var(x_{t+1}|x_t)))$, the implementations can derive the mean and variance of $x_t$ recursively as follows:

$$\mu_{t+1} = \mu_t \cdot (1+r_{t+1})$$

$$\delta_{t+1}^2 = \mu_t \cdot r_{t+1} \cdot (1-r_{t+1}) + \delta_t^2 \cdot (1+r_{t+1})^2,$$

where $\mu_t = E(x_t)$, $\delta_t^2 = var(x_t)$.

Assuming an average amplification rate per PCR cycle $r_{t+1} = r$, the implementations have

$$\mu_t = \mu_0 \cdot (1+r)^t$$

$$\delta_t^2 = \mu_0 \cdot (1+r)^t \cdot [(1+r)^t - 1] \cdot (1-r)/(1+r) + \delta_0^2 \cdot (1+r)^{2t}$$

Notice that $\mu_0$ and $\delta_0^2$ are the mean and variance of DNA allele counts in the PCR amplification input, and they can be computed based on the DNA extraction model (model E) described above. Alternatively, if the implementations do not treat cfDNA/cellular DNA allele counts as random variables, the implementations have $\mu_0 = n_1''$ or $n_2''$, and $\delta_0^2 = 0$.

The corresponding gamma distribution $G(x_t | k, \theta) = x^{k-1}e^{-x/\theta}[\theta^k \cdot \Gamma(k)]$ that matches this mean and variance has parameters:

$$\theta = [(1+r)^t - 1] \cdot (1-r)/(1+r) + \delta_0^2/\mu_0 \cdot (1+r)^t$$

$$k = \mu_0 \cdot (1+r)^t / [[(1+r)^t - 1] \cdot (1-r)/(1+r) + \delta_0^2/\mu_0 \cdot (1+r)^t].$$

For a given locus with two alleles and two initial copies $(n_1'', n_2'')$, assuming identical amplification rate $r_1 = r_2 = r$ for two alleles for each locus, the two corresponding gamma distributions $G(n_1' | k_1, \theta_1)$ and $G(n_2' | k_2, \theta_2)$ have the following parameters:

$$\theta_1 = [(1+r)^t - 1] \cdot (1-r)/(1+r) + p_2 \cdot (1+r)^t$$

$$\theta_2 = [(1+r)^t - 1] \cdot (1-r)/(1+r) + p_1 \cdot (1+r)^t$$

$$k_1 = n''p_1 / [[1 - (1+r)^{-t}] \cdot (1-r)/(1+r) + p_2]$$

$$k_2 = n''p_2 / [[1 - (1+r)^{-t}] \cdot (1-r)/(1+r) + p_1].$$

When the implementations condition the PCR model on the DNA extraction model, s.t. $\mu_0 = n_1''$ or $n_2''$ and $\delta_0^2 = 0$, the implementations then have

$$\theta_1 = [(1+r)^t - 1] \cdot (1-r)/(1+r)$$

$$\theta_2 = [(1+r)^t - 1] \cdot (1-r)/(1+r)$$

$$k_1 = n_1'' \cdot (1+r)/(1-r) / [1-(1+r)^{-t}]$$

$$k_2 = n_2'' \cdot (1+r)/(1-r) / [1-(1+r)^{-t}].$$

Hence the allele copies $n_1'$ and $n_2'$ in the PCR product follow two Gamma distributions with identical scale parameters $\theta_1$ and $\theta_2$, which are only dependent on the PCR process (the number of cycles and amplification rate). Therefore,

$$n_1'/ (n_1' + n_2') \sim \text{Beta}(n_1'' \cdot \rho, n_2'' \cdot \rho),$$

where $\rho$ is a constant related to the amplification rate r, which is only dependent on the PCR process: $\rho = (1+r)/(1-r) / [1-(1+r)^{-t}]$, or approximately $\rho = (1+r)/(1-r)$ when the number of cycles t is large. For a specific locus, this is written as $n_{1i}'/ (n_{1i}' + n_{2i}') \sim \text{Beta}(n_{1i}'' \cdot \rho_i, n_{2i}'' \cdot \rho_i)$, to capture the locus specific PCR amplification rate.

If the implementations ignore DNA sampling and assume all loci have the same total DNA copy number $n_i'' = n''$, then $n_{1i}'' = n'' \cdot p_{1i}$ and $n_{2i}'' = n'' \cdot p_{2i}$. The allele fraction for a locus in the PCR product follows:

$$n_{1i}'/ (n_{1i}' + n_{2i}') \sim \text{Beta}(n'' \cdot \rho_i \cdot p_{1i}, n'' \cdot \rho_i \cdot p_{2i}).$$

Note that without the Gamma distribution approximation, the allele counts of PCR products have $n_1' \sim \text{NB}(r_1, p)$ and $n_2' \sim \text{NB}(r_2, p)$, and the ratio $n_1'/(n_1' + n_2')$ has no closed form distribution. With the Gamma distribution approximation, $n_1' \sim \text{Gamma}(n_1'' \cdot \rho, \theta)$ and $n_2' \sim \text{Gamma}(n_2'' \cdot \rho, \theta)$, and $n_1'/(n_1' + n_2')$ follows the beta distribution.

### Sequencing Read Count Model: Model S

NGS sequencing is a process that samples from the pool of DNA molecules supplied to the sequencer and reads out the sequences of these molecules. The fraction of allele 1 for a locus i in the PCR product is $n_{1i}'/ (n_{1i}' + n_{2i}')$. This fraction determines the probability that allele 1 reads occur in the sequencing results. Conditioning on $n_i$, the total number of reads per locus, the distribution of $n_{1i}$, the allele 1 read count of a locus, is then modeled as a binomial distribution $n_{1i} \sim \text{BN}(n_i, n_1'/ (n_1' + n_2'))$.

### Modeling the Genetic Relationship between the Contributors as a Prior Distribution

If the contributor genotypes are completely known, they can be directly incorporated (using Table 2 or Table 3) as parameters of the component models described above. However, when the genotypes are unknown, the implementations make use of the genetic-relationship information between the donor and recipient in a two-contributor setting to achieve accurate mixture quantification. Genetic relationship is commonly available in clinical applications such as organ transplant. Here we present the implementation for two-contributor scenario, but this "genetic prior" approach can be generalized to any number of contributors.

We formulate different types of donor-recipient relationships as distinct prior distributions on the space of possible genotype combinations of the donor (contributor 2) and recipient (contributor 1). Assuming Hardy-Weinberg equilibrium, the genotype distribution for a given loci for a single individual is $P(g = [0,1,2]) = [(1-\pi)^2, 2\pi(1-\pi), \pi^2]$, where $\pi$ is the population frequency of allele 1, g is the allele 1 copy number. Notice that all genetic relationships are the results of parent-child relationships. Based on the genetic-relationships between parent and child for a give biallelic marker site (Table 5), the implementations can compute the joint distribution for any genetic relationship among two or multiple contributors.

**Table 5: Probability distribution of child genotype given parents' genotypes (father genotype $g_{Father}$ and mother genotype $g_{Mother}$) for a given locus, as well as the joint distribution between father and mother assuming they are not relatives.**

| $g_{Father}$ | $g_{Mother}$ | Child probability for genotype [0, 1, 2] respectively conditioned on parent genotypes | $P(g_{Father}, g_{Mother})$ |
|---|---|---|---|
| 0 | 0 | [1, 0, 0] | $(1-\pi)^4$ |
| 0 | 1 | [1/2, 1/2, 0] | $2\pi(1-\pi)^3$ |
| 0 | 2 | [0, 1, 0] | $\pi^2(1-\pi)^2$ |
| 1 | 0 | [1/2, 1/2, 0] | $2\pi(1-\pi)^3$ |
| 1 | 1 | [1/4, 1/2, 1/4] | $4\pi^2(1-\pi)^2$ |
| 1 | 2 | [0, 1/2, 1/2] | $2\pi^3(1-\pi)$ |
| 2 | 0 | [0, 1, 0] | $\pi^2(1-\pi)^2$ |
| 2 | 1 | [0, 1/2, 1/2] | $2\pi^3(1-\pi)$ |
| 2 | 2 | [0, 0, 1] | $\pi^4$ |

The prior distributions for various types of genetic-relationship between two contributors are further provided below.

### *Joint Distribution between Father and Child Genotypes*

As an example, the Father-Child donor-recipient genotype (GT) joint distribution is computed using the following formula:

P(Recipient = Me GT, Donor = Father GT) = $\Sigma_{mother\ GT}$ [P(Me GT|Father GT, Mother GT) · P(Father GT, Mother GT)],

where values of P(Me GT|Father GT, Mother GT) and P(Father GT, Mother GT) are taken from the Table 5 columns 3 and 4 respectively.

### *Joint Distribution between Sibling Genotypes*

As an example, the Me-Sibling donor-recipient genotype joint distribution is computed using the following formula, based on the conditional independence of two sibling genotypes given parents genomes:

P(Recipient = Me GT, Donor = Sibling GT) = $\Sigma_{mother\ GT}$ $\Sigma_{father\ GT}$ [P(Me GT |Father GT, Mother GT) · P(Sibling GT| Father GT, Mother GT) · P(Father GT, Mother GT)],

Where values of P(Me GT|Father GT, Mother GT), P(Sibling GT|Father GT, Mother GT) , and P(Father GT, Mother GT) are taken from the Table 5 columns 3, column 3, and column 4 respectively.

### *Joint Distribution between Uncle-Nephew Genotypes*

As a example, the Uncle/Aunt-Nephew/Niece donor-recipient genotype joint distribution is computed using the following formula:

P(Recipient = Me GT, Donor = Uncle GT) = $\Sigma_{\text{grandmother GT}}$ $\Sigma_{\text{grandfather GT}}$ $\Sigma_{\text{mother GT}}$ $\Sigma_{\text{father GT}}$ [P(Me GT|Father GT, Mother GT) · P(Mother GT) · P(Father GT|GrandFather GT, GrandMother GT) · P(Uncle GT|GrandFather, GrandMother GT) · P(GrandFather GT, GrandMother GT)] = $\Sigma_{\text{mother GT}}$ $\Sigma_{\text{father GT}}$ P(Me GT|Father GT, Mother GT) · P(Mother GT) · P(Father GT, Uncle GT),

where values of P(Me GT|Father GT, Mother GT) is taken from column 3 of table 5, and P(Father GT, Uncle GT) is the same as P(Recipient = Me GT, Donor = Sibling GT).

In matrix notation, this can be computed using the parent/child prior matrix, the sibling prior matrix, and the single genome prior vector

= [P(Me GT, Father GT)]$_{\text{Me, Father}}$ • diag( 1 / [P(Father GT)]$_{\text{Father}}$ ) • [P(Father GT, Uncle GT)]$_{\text{Father, Uncle}}$

### *Joint Distribution between Cousin Genotypes*

Assuming cousin is genetically linked by their fathers, who are brother, and the mothers are genetically unrelated, then, P(Recipient = Me GT, Donor = Cousin GT) = $\Sigma_{\text{aunt GT}}$ $\Sigma_{\text{uncle GT}}$ $\Sigma_{\text{mother GT}}$ $\Sigma_{\text{father GT}}$ P(Me GT|Father GT, Mother GT) · P(Mother GT) · P(Father GT, Uncle GT) · P(Aunt GT) · P(Cousin GT|Uncle GT, Aunt GT) = $\Sigma_{\text{aunt GT}}$ $\Sigma_{\text{uncle GT}}$ P(Me GT, Uncle GT) · P(Aunt GT) · P(Cousin GTIUncle GT, Aunt GT) = $\Sigma_{\text{uncle GT}}$ P(Me GT, Uncle GT) · P(Cousin GT, Uncle GT) / P(Uncle GT)

In matrix notation, this can be computed using the uncle/niece prior matrix, the parent/child prior matrix, and the single genome prior vector = [P(Me GT, Uncle GT)]$_{\text{Me, Uncle}}$ • diag( 1 / [P(Uncle GT)]$_{\text{Uncle}}$ ) • [P(Cousin GT, Uncle GT)]$_{\text{Uncle, Cousin}}$

Notice that P(Cousin GT, Uncle GT) is the same as parent-child relationship.

### *Joint Distribution between Half Sibling Genotypes*

Assuming half sibling is linked by a single mother, and the two fathers are unrelated:

P(Recipient = Me GT, Donor = HafSib GT) = $\Sigma_{\text{Father GT}}$ $\Sigma_{\text{Mother GT}}$ $\Sigma_{\text{StepFather GT}}$ P(Me GT|Father GT, Mother GT) · P(HalfSib GT|StepFather GT, Mother GT) ·P(Mother GT) · P(Father GT) · P(StepFather GT) = $\Sigma_{\text{Mother GT}}$ P(Me GT, Mother GT) · P(HalfSib GT, Mother GT) / P(Mother GT)

In matrix notation, this can be computed using the two parent child prior matrix, and the single genome prior vector = [P(Me GT, Mother GT)]$_{\text{Me, Mother}}$ • diag( 1 / [P(Mother GT)]$_{\text{Mother}}$) • [P(HalfSib GT, Mother GT)]$_{\text{HalfSib, Mother}}$

Note that under HardyWeinburg equilibrium, half sibling relationship follows the same distribution as the uncle/aunt/nephew/niece relationship. This may not be true without HardyWeinburg equilibrium.

### *Summary*

The results from the above derivations is summarized in Table 6, and the specific instances given population SNP allele frequency $\pi$ = 0.5 is provided in Table 7. Additional relationships, such as grandparent-grandchild relationship or multi-contributor relationship, can be derived based on the same underlying principle.

**Table 6: Prior distributions P(g$_{11}$, g$_{21}$) of related or unrelated genomes. Assuming all SNPs are from autosomes, all married couples are genetically-unrelated, and in Hardy Weinberg equilibrium. g$_{11}$ is the recipient genome, g$_{21}$ is the donor genome.**

| $g_{11}$ | $g_{21}$ | Donor's relationship to Recipient | | | | | |
|---|---|---|---|---|---|---|---|
| | | Parent or Child | Sibling | Half Siblings | Uncle/Aunt or Nephew/Niece | Cousin | Unrelated |
| 0 | 0 | $(1-\pi)^3$ | $(1-\pi)^2(1-\pi/2)^2$ | $(1-\pi)^3(1-\pi/2)$ | $(1-\pi)^3(1-\pi/2)$ | $(1-\pi)^3(1-3\pi/4)$ | $(1-\pi)^4$ |
| 0 | 1 | $\pi(1-\pi)^2$ | $\pi(1-\pi)^2(1-\pi/2)$ | $\pi(1-\pi)^2(3/2-\pi)$ | $\pi(1-\pi)^2(3/2-\pi)$ | $\pi(1-\pi)^2(7/4-3\pi/2)$ | $2\pi(1-\pi)^3$ |
| 0 | 2 | 0 | $\pi^2(1-\pi)^2/4$ | $\pi^2(1-\pi)^2/2$ | $\pi^2(1-\pi)^2/2$ | $3/4\pi^2(1-\pi)^2$ | $\pi^2(1-\pi)^2$ |
| 1 | 0 | $\pi(1-\pi)^2$ | $\pi(1-\pi)^2(1-\pi/2)$ | $\pi(1-\pi)^2(3/2-\pi)$ | $\pi(1-\pi)^2(3/2-\pi)$ | $\pi(1-\pi)^2(7/4-3\pi/2)$ | $2\pi(1-\pi)^3$ |
| 1 | 1 | $\pi(1-\pi)$ | $\pi(1-\pi)[1+\pi-\pi^2)]$ | $\pi(1-\pi)[1/2+2\pi-2\pi^2)]$ | $\pi(1-\pi)[1/2+2\pi-2\pi^2]$ | $\pi(1-\pi)[1/4+3\pi-3\pi^2]$ | $4\pi^2(1-\pi)^2$ |
| 1 | 2 | $\pi^2(1-\pi)$ | $\pi^2(1-\pi)(1/2+\pi/2)$ | $\pi^2(1-\pi)(1/2+\pi)$ | $\pi^2(1-\pi)(1/2+\pi)$ | $\pi^2(1-\pi)(1/4+3\pi/2)$ | $2\pi^3(1-\pi)$ |
| 2 | 0 | 0 | $\pi^2(1-\pi)^2/4$ | $\pi^2(1-\pi)^2/2$ | $\pi^2(1-\pi)^2/2$ | $3/4\pi^2(1-\pi)^2$ | $\pi^2(1-\pi)^2$ |
| 2 | 1 | $\pi^2(1-\pi)$ | $\pi^2(1-\pi)(1/2+\pi/2)$ | $\pi^2(1-\pi)(1/2+\pi)$ | $\pi^2(1-\pi)(1/2+\pi)$ | $\pi^2(1-\pi)(1/4+3\pi/2)$ | $2\pi^3(1-\pi)$ |
| 2 | 2 | $\pi^3$ | $\pi^2(1/2+\pi/2)^2$ | $\pi^3(1/2+\pi/2)$ | $\pi^3(1/2+\pi/2)$ | $\pi^3(1/4+3\pi/4)$ | $\pi^4$ |

**Table 7: Prior distributions P(g$_{11}$, g$_{21}$) of related or unrelated genomes given SNP population allele frequency $\pi$ = 0.5.**

| $g_{11}$ | $g_{21}$ | Donor's relationship to Recipient | | | | | |
|---|---|---|---|---|---|---|---|
| | | Parent or Child | Sibling | Half Sibling | Uncle/Aunt or Nephew/Niece | Cousin | Unrelated |
| 0 | 0 | 8/64 | 9/64 | 6/64 | 6/64 | 5/64 | 4/64 |
| 0 | 1 | 8/64 | 6/64 | 8/64 | 8/64 | 8/64 | 8/64 |
| 0 | 2 | 0 | 1/64 | 2/64 | 2/64 | 3/64 | 4/64 |
| 1 | 0 | 8/64 | 6/64 | 8/64 | 8/64 | 8/64 | 8/64 |
| 1 | 1 | 16/64 | 20/64 | 16/64 | 16/64 | 16/64 | 16/64 |
| 1 | 2 | 8/64 | 6/64 | 8/64 | 8/64 | 8/64 | 8/64 |
| 2 | 0 | 0 | 1/64 | 2/64 | 2/64 | 3/64 | 4/64 |
| 2 | 1 | 8/64 | 6/64 | 8/64 | 8/64 | 8/64 | 8/64 |
| 2 | 2 | 8/64 | 9/64 | 6/64 | 6/64 | 5/64 | 4/64 |

The distributions for parent-child and sibling relationship are quite different from unrelated, while uncle/aunt-nephew/niece are close to unrelated. In the case when the donor genotype is unknown, the implementations can infer the genetic relationship by evaluating the likelihood function of fitted models of each of the above genetic relationships. Alternatively, the implementations can allow multiple free parameters in the genetic priors distribution (with additional constraints that the marginal distributions should follow Hardy-Weinberg equilibrium), and estimate these parameters together with the estimation of donor fraction.

**Adjustment of DNA Copy Numbers based on DNA Length**

For an amplicon-based assay that involves PCR DNA amplification, the DNA length impacts the effectiveness of the DNA as PCR template. In the extreme, when DNA fragments are shorter than the intended amplicon length, they are 0% effective as PCR template. To correct for this effect, we used the following procedure to adjust the DNA copy numbers using the average DNA length, which varies depending on the type of the input DNA. Some implementations adjust the effective input DNA molecule number based on the average length of input DNA template. In some implementation, the effective input DNA molecule number is adjusted according to the equation below:

$$n'' = w/w_0 \cdot (L - L_a + 1)/L,$$

where n'' is the effective input DNA molecule number (haploid), w is the input DNA amount, $w_0$ ($3.59 \times 103$ ng/copy) is the weight of haploid human genome, L is the average length of input DNA template, and $L_a$ is the average amplicon length (110bp for our amplicon design).

DNA template efficiency is defined as e = $(L - L_a + 1)/L$, which is defined for L >= $L_a$. Table 8 shows example DNA types and their efficiency as PCR templates.

**Table 8. DNA type and their efficiency as PCR template**

| DNA type | DNA length parameter (L) | Template Efficiency (e) |
|---|---|---|
| genomic DNA (gDNA) | 100,000 | 0.9989 |
| cell free DNA (cfDNA) | 165 | 0.3394 |
| mock cfDNA (mcfDNA) | 160 | 0.3188 |

### Integration of the Modeling Components

The components of the probabilistic mixture model are integrated to provide a solution to the contributor DNA quantification (CDQ) problem. The population allele frequency $\pi$ for each SNP site can be obtained from public databases such as dbSNP. If one selects the most informative SNP markers, i.e. SNPs with $\pi = 0.5$, in an experiment design, one can set $\pi = 0.5$ for all loci and let $P(g_{11}, g_{21})$ be the genetic-relationship prior distribution as described in the previous section.

On a schematic level, Figure 2B shows a block diagram illustrating various components of the probabilistic mixture model 250. Some components are optional in some implementations. The probabilistic mixture model 250 includes a binomial distribution 258 for modeling allelic counts of sequencing reads. In some implementations, the probabilistic mixture model also includes a component for modeling donor-donee (or recipient) relationship using a genetic relationship prior distribution 252. In some implementations, the probabilistic mixture model also includes a binomial distribution 254 for modeling DNA extraction allelic counts. In some implementations, the probabilistic mixture model 250 also includes a beta distribution 256 for modeling PCR product or amplification product allelic fraction. See block 256.

In some implementations, the mixture model combines the binomial distribution 208 with binomial distribution 254 to model both the DNA extraction errors and sequencing errors. In such implementations, the mixture model uses a beta-binomial distribution 260 to model the allelic counts of sequencing reads while capturing variability in the allelic counts due to DNA extraction.

In some implementations, the probabilistic mixture model 250 combines beta distribution 256 and binomial distribution 258, and uses a beta-binomial distribution 262 to model both errors in the PCR or amplification process and errors of sequencing process.

In some implementations, the probabilistic mixture model 250 combines binomial distribution 254, beta distribution 256, and binomial distribution 258 to account for variance resulting from DNA extraction, amplification process, and sequencing process, respectively. In such implementations, probabilistic mixture model 200 first uses a beta distribution 264 to approximate the effects of binomial distribution 254 and beta distribution 256. The probabilistic mixture model 250 then combines beta distribution 264 and binomial distribution 258 using beta-binomial distribution 256.

### *The Sequencing Model: Model S*

A basic version of the full model ignores the DNA extraction model and the PCR model, and only considers the sequencing model. For each locus, the sequencing read count for the reference allele is modeled by a binomial distribution (Figure 1C), $n_{1i} \sim BN(n_i, p_{1i})$, where the value of parameter $p_{1i}(g_{1i}, g_{2i}, \lambda, \beta)$ is a function on the donor-recipient genotype combination for the loci (Table 2 and Table 3). Given that the genotypes are unknown, the implementations marginalize over the 9 possible genotype combinations for each locus with $P(g_{1i}, g_{2i}|\pi)$ as prior distribution (Table 6 and Table 7). The complete likelihood function across all loci is the product of the marginal distributions for all loci:

$L(\beta, \lambda, \pi ; n_1, n_2) = \Pi_i \Sigma_{g1ig2i} BN(n_{1i}|n_i, p_{1i}(g_{1i}, g_{2i}, \lambda, \beta)) \cdot P(g_{1i}, g_{2i}|\pi)$, where $L(\beta, \lambda, \pi ; n_1, n_2)$ is the likelihood of observing

allele count vectors $n_1$ to $n_2$ for alleles 1 and 2 given parameters $\beta$ and $\pi$; $p_{1i}(g_{1i}, g_{2i}, \lambda, \beta)$ is a probability parameter, taken as $p_1'$ from Table 3, indicating a probability of allele *1* at locus *i* based on the two contributors' genotypes $(g_{1i}, g_{2i})$; and $P(g_{1i}, g_{2i}|\pi)$ is a prior joint probability of observing the two contributors' genotypes given a population allele frequency $(\pi)$.

Expanding it to multiple contributors, the likelihood function can be expressed as:

$$L(\boldsymbol{\beta}, \lambda, \pi; n_1, n_2) = \Pi_i [\Sigma g_i BN(n_{1i} \mid n_i, \cdot p(g_i, \lambda, \boldsymbol{\beta})) \cdot P(g_i \mid \pi)]$$

### The Extraction-Seq Compound Model: Model ES

A more advanced model combines the DNA extraction model as well as the Sequencing model. The implementations ignore the PCR step (i.e. assume that, for each locus, the allele fraction in the PCR product is the same as the allele fraction in the DNA sample), and only model DNA sampling and sequencing steps For each locus, there is a binomial distribution for the allele counts in the input DNA sample. This captures the locus-to-locus variability of the allele fractions in the input DNA provided to the NGS sequencing.

For the DNA extraction model, the implementations have $n_{1i}" \sim BN(n", p_{1i})$, while conditioning on the DNA extraction model, the sequencing model is $n_{1i}|n_{1i}", n" \sim BN(n_i, n_{1i}"/n")$, where $n_i" = n"$ is the copies of haploid genomes the input DNA correspond to. Unfortunately, the marginal distribution of $n_{1i}$ has no closed form formula. The implementations approximate the distribution of $n_{1i}"/n"$ with a beta distribution Beta(a, b), and the best Beta distribution is selected by matching the mean and variance of $n_{1i}"/n"$ with those derived from the binomial model $n_{1i}" \sim BN(n", p_{1i})$:

$$p_{1i} = a/(a+b)$$

$$p_{1i} \cdot (1-p_{1i})/n" = ab/(a+b)^2/(a+b+1).$$

Solving the equations gives the beta distribution Beta$((n"-1)p_{1i}, (n"-1)p_{2i})$ as the best approximation. With this approximation to the DNA extraction model, the marginal distribution of $n_{1i}$ then follows a beta-binomial distribution of the form:

$$n_{1i} \sim BB(n_i, (n"-1) \cdot p_{1i}, (n"-1) \cdot p_{2i}).$$

Or in an alternative approximation:

$$n_{1i} \sim BB(n_i, n" \cdot p_{1i}, n" \cdot p_{2i}).$$

The corresponding full likelihood function considering the genetic-relationship prior is then:

$$L(\beta, n", \lambda, \pi; n_1, n_2) = \Pi_i [\Sigma g_i BB(n_{1i} \mid n_i, (n"-1) \cdot p_{1i}, (n"-1) \cdot p_{2i}) \cdot P(g_i \mid \pi)]$$

wherein $L(\beta, n", \lambda, \pi; n_1, n_2)$ is the likelihood of observing allele count vectors $n_1$ and $n_2$ for alleles *1* and *2* at all loci, and $p_{1i} = p(g_i, \lambda, \beta)$, $p_{2i} = 1 - p_{1i}$.

Notice that both n" and $\pi = 0.5$ are known parameters, and the final full likelihood function has only a single unknown parameter $\beta$, the donor DNA fraction.

The input DNA (haploid) copy numbers n" can be derived from the input DNA mass. When input DNA amount is 8ng, $n" = 8$ ng / [$3.59 \times 10^{-3}$ ng/copy] = 2228.412.

### PCR-Seq Compound Model: Model PS

Ignoring the DNA extraction model, and assuming a known genotype combination for a given locus, then the PCR model: $n_{1i}'/(n_{1i}' + n_{2i}') \sim Beta(n" \cdot \rho_i \cdot p_{1i}, n" \cdot \rho_i \cdot p_{2i})$ and Sequencing model $n_{1i} \sim BN(n_i, n_1'/(n_1' + n_2'))$ can be combined into the beta-binomial distribution: $BB(n_i, n" \cdot \rho i \cdot p_{1i}, n" \cdot \rho_i \cdot p_{2i})$. Notice that both the underlying loci specific PCR amplification

rates $\rho_i$ are unknown. If the implementations assume all loci have the same inherent amplification rate, then the implementations have, $BB(n_i, c \cdot p_{1i}(g_{11}, g_{21}, \beta), c \cdot p_{2i}(g_{11}, g_{21}, \beta))$.

The complete likelihood model across all loci is then: $L(\beta, n'', c, \lambda, \pi; n_1, n_2) = \Pi i [\Sigma g_i BB(n_{1i}| n_i, c \cdot p_{1i}, c \cdot p_{2i}) \cdot P(g_i | \pi)]$, where c and $\beta$ are two parameters to be estimated.

Alternatively, the implementations can define the relative amplification rate of each locus to be proportional to the total reads per locus, and re-parameterize the beta-binomial as $n_{1i} \sim BB(n_i, c' \cdot n_i \cdot p_{1i}, c' \cdot n_i \cdot p_{2i})$, where c' is a parameter to be optimized; and $n_i$ is the total reads at locus $i$.

The complete likelihood model across all loci is then: $L(\beta, n'', c', \lambda, \pi; n_1, n_2) = \Pi i [\Sigma g_i BB(n_{1i}| n_i, c' \cdot n_i \cdot p_{1i}, c' \cdot n_i \cdot p_{2i}) \cdot P(g_i | \pi)]$, where c' and $\beta$ are two parameters to be estimated

### *Extraction-PCR-Seq Compound Model: Model EPS*

All three components in the Extraction-PCR-sequencing generic experimental pipeline can be modeled together by a beta-binomial if the implementations combine DNA extraction and PCR models into one model and approximate it by a single beta distribution. Intuitively, although the expected value of allele 1 fraction in the PCR product ($n_1'/n'$, see Table 4) remains $p_1$, the uncertainty (variance) of $n_1'/n'$ originates from both the DNA extraction and the PCR steps. To obtain a beta distribution beta(a,b) to model DNA extraction and PCR together, the implementations compute the unconditional mean and variance of $n_{1i}'/n'$ based on the following laws: $E(n_{1i}'/n') = E(E(n_{1i}'/n_i' | n_{1i}''/n''))$, and $var(n_{i1}'/n') = var(E(n_{1i}'/n_i' | n_{1i}''/n'')) + E(var(n_{1i}'/n_i' | n_{1i}''/n''))$. This gives: $E(n_{1i}'/n') = p_{1i}$, and $var(n_{1i}'/n') = p_{1i}p_{2i} / n'' + p_{1i}p_{2i} / (n'' \cdot \rho_i + 1) - p_1p_2 / [n'' \cdot (n'' \cdot \rho_i + 1)]$, where $\rho_i = (1+r_i)/(1-r_i) > 1$ is the constant related to the amplification rate $r_i$. Since n" is large, the implementations have the following approximation $var(n_{1i}'/n') = p_{1i}p_{2i} / [n'' \cdot (1+r_i)/2]$. The best beta distribution that models DNA extraction and PCR is then Beta([n''$\cdot$ (1+ $r_i$)/2 - 1]$p_{1i}$, [n''$\cdot$ (1+ $r_i$)/2 - 1]$p_{2i}$). Notice this is close to the beta distribution for cfDNA/gDNA extraction Beta((n''-1)$p_{1i}$, (n''-1)$p_{2i}$), yet the variance is now larger. For a typical PCR reaction with $r_i = 0.8$ to 0.95, the implementations have n'' $\cdot$ (1+ $r_i$)/2 = 0.9 $\cdot$ n'' to 0.975 $\cdot$ n''.

The full multiple-loci likelihood function for cfDNA-PCR-Seq model is:

$$L(\beta, n'', r, \lambda, \pi; n_1, n_2) = \Pi_i \Sigma_{g1ig2i} BB(n_{1i} | n_i, n'' \cdot (1+r)/2 \cdot p_{1i}(g_{1i}, g_{2i}, \lambda, \beta), n'' \cdot (1+r)/2 \cdot p_{2i}(g_{1i}, g_{2i}, \lambda, \beta)) \cdot P(g_{1i}, g_{2i}|\pi)$$

### *Baseline Method: NaiveLM or KGT.NaiveLM*

A conventional method for quantifying DNA fractions of contributors uses a basic linear regression formulation, which does not use the same probabilistic model or cost functions described above. Instead, its cost function is expressed as:

$E = [r - p]^T \cdot [r - p]$, where r is the allele fraction vector, $p = g/2 \bullet \beta$ is the expected allele fraction vector, $g$ is the genotype matrix, and $\beta$ is the contributor DNA fraction vector. The naive method is only applicable when all base lines are known.

### Method for Estimating Contributor Nucleic Acid Fractions and Their Confidence Intervals

### *Numerical Optimization for Estimating Contributor DNA Fractions*

The contributor DNA fraction $\beta$ is estimated as the value that maximize the full likelihood function $L(n_1, n_2| \beta)$. As mentioned above, although DNA is referred to in this and other examples, RNA and other nucleic acid molecules may be processed and analyzed similarly. Also, although the examples refer to nucleic acid mixture samples, the sample may include only a single contributor's nucleic acid, in which case the contributor fraction would be estimated as 1 or within a margin of error from 1.

During the calculation of $L(n_1, n_2| \beta)$, multiple small probabilities values are multiplied. To avoid numerical underflowing when multiplying small probabilities, the implementations perform all summation and multiplications on log scale. The sum of small probability on log scale is performed as following: 1) obtain the max of the log probabilities as $x_{max}$; 2) subtract all the log probabilities by the max; 3) exponentiate and then sum the resulting values; 4) log transform the resulting sum; 5) add back the max of the log probabilities. $log(exp(x_1 - x_{max}) + exp(x_2 - x_{max}) + ... + exp(x_n - x_{max})) + x_{max}$.

To ensure positive contributor fractions within 0 to 1, the logit transformation $\beta = 1/(1+e^{-\eta})$ is used.

A novel numerical optimization computer strategy that seamlessly integrating iterative grid search with Broyden-Fletcher-Goldfarb-Shanno (BFGS) quasi-Newton method is implemented as described below.

Step 1: A grid initialization method generates even grids in N-1 dimensional space, where N is the number of contributors. In applications with only two contributors, to ensure global optimization and avoiding local optimums, the full likelihood function is initialized with $\beta_0 = 1/(1+e^{-\eta_0})$, where $\eta_0$ is the value among -10, -9.9, -9.8, .... , -0.1, 0 that maximizes $L(n_1, n_2| \beta_0 = 1/(1+e^{-\eta_0}))$ for two contributor cases. In applications with for multi-contributor cases, $\beta$ is transformed using softmax, and then initialized over a high dimensional grid.

Step 2: An exhaustive search on the grid is performed to identified mixture fractions that minimizes -log2(L).

Step 3: Initializing using the identified mixture fractions, numerical optimization of $\eta$ is then performed using Broyden-Fletcher-Goldfarb-Shanno (BFGS) quasi-Newton method to minimize -log2(L). Record the optimized mixture fraction as well as the convergence.

Step 4: Hessian matrix of -log2(L) is computed using numerical differentiation on the identified mixture fractions.

Step 5: Errors and confidence interval around computed mixture fractions is determined based on the inverse of the hessian matrix. Meanwhile, determine if the hessian matrix is positive semi-definite.

Step 6: If BFGS optimization did not converge or if the hessian matrix is not positive semi-definite, then the procedure is configured for a next iteration of optimization. Otherwise optimization complete.

Step 7: When next iteration of optimization is to be performed, a finer N-1 dimensional grid is constructed covering $2^{N-1}$ original grids around the previously determined $\eta$, which corresponds to the estimated mixture fractions. The procedure then loops back to step 2 for next iteration of grid search and BFGS optimization.

The totality of these steps cannot be performed by human experts manually or in their heads. Instead, one or more computers are required to perform these steps.

### *Iterative Strategy for Model S with Known Genotypes (KGT.IterLM)*

In some implementations, the single-locus likelihood function comprises a binomial distribution and the multiple-loci likelihood function is as follows: $L(\beta, \lambda, \pi ; n_1, n_2) = \Pi_i [\Sigma g_i\, BN(n_{1i} | n_i, \cdot p(g_i, \lambda, \beta)) \bullet P(g_i | \pi)]$

In some implementations, the contributors include two contributors and the likelihood function is: $L(\beta, \lambda, \pi ; n_1, n_2) = \Pi_i \Sigma_{g1i, g2i}\, BN(n_{1i}| n_i, p_{1i}(g_{1i}, g_{2i}, \lambda, \beta)) \cdot P(g_{1i}, g_{2i}|\pi)$

where $L(\beta, \lambda, \pi ; n_1, n_2)$ is the likelihood of observing allele count vectors $n_1$ to $n_2$ for alleles 1 and 2 given parameters $\beta$ and $\pi$; $p_{1i}(g_{1i}, g_{2i}, \lambda, \beta)$ is a probability parameter, taken as $p_1$' from Table 3, indicating a probability of allele *1* at locus *i* based on the two contributors' genotypes $(g_{1i}, g_{2i})$; and $P(g_{1i}, g_{2i}|\pi)$ is a prior joint probability of observing the two contributors' genotypes given a population allele frequency $(\pi)$.

In some implementations, the genotypes of all contributors are known, and the likelihood functions are expressed as $L(\beta, \lambda; n_1, n_2) = \Pi_i n_i!/(n_{1i}!n_{2i}!)\, p_1^{n1i}p_2^{n2i}$, where $p_{ai} = \Sigma_{d=0...D-1}\, g_{dai} \bullet \beta_d / [\Sigma_{d=0...D-1}\, \beta_d \bullet (\Sigma_{a=1,2}\, g_{dai})]$. If all markers are on somatic chromosomes, then $p_{ai} = 1/2 \bullet \Sigma_{d=0...D-1}\, g_{dai} \bullet \beta_d$. In matrix notation, this is $p \leftarrow g/2 \bullet \beta$.

The iterative weighted linear regression method is developed by constructing a cost function that has the same gradient as that of $log[L(\beta; n_1, n_2)]$ in each iteration when $\beta = \beta_0$:

$$E = 1/2 \;\;\bullet\; \Sigma_i\, n_i/[p_{1i}(\boldsymbol{\beta_0}) \;\;\bullet\; (1-p_{1i}(\boldsymbol{\beta_0}))] \;\;\bullet\; (n_{1i}/n_i - p_{1i}(\boldsymbol{\beta}))^2.$$

In matrix notation, this is $E = 1/2 \bullet (r - p)^T \bullet W^2 \bullet (r - p)$, where $W = diag([n/(p_0 \bullet (1 - p_0))]^{1/2})$ is a diagonal matrix, and $p_0 = g/2 \bullet \beta_0$.

Iterative weighted linear regression is carried out by executing the following steps, given inputs: *r, n, g, and* $\lambda$

Step 1. Initialize $\beta$ as a uniform length D probability vector $\beta \leftarrow [1/D]_D$

Step 2. Compute error correction of genotype matrix $g$: $G \leftarrow [(1- \lambda)\, g + \lambda\, (2-g)]/2$

Step 3: Repeat Step a - Step e until convergence

Step a:. Update expected allele 1 fraction using the previous computed contributor fraction: $p \leftarrow G \bullet \beta$

Step b. Compute the weights for weighted regression $W \leftarrow diag([n/(p \bullet (1 - p))]^{1/2})$

Step c. Solve the weighted linear regression: $\beta \leftarrow (W \bullet G)^{-1} \bullet (W \bullet r)$

Step d. Ensure non-negativity: for each contributor i, $\beta_i \leftarrow max(\beta_i, 0)$

Step e. Normalization to probability vector: $\beta \leftarrow \beta /\Sigma_i \beta_i$ - normalization

### *Estimate the Confidence Interval*

The lower bound of the confidence interval of the estimates are determined based on the Cramer-Rao inequality: $var(\theta_{ML}) \geq 1/I(\theta_{ML})$, where $\theta_{ML}$ is the maximum likelihood estimate of parameter $\theta$, and $I(\theta_{ML})$ is fisher's information at $\theta_{ML}$. Based on this, one can estimate the variance of $\beta$ and c in the above described likelihood functions. The standard error is estimated as sqrt(1/H) following the Cramér-Rao bound, where H is the Hessian matrix which can be approximated and is estimated in the BFGS - quasi-Newton method.

We use the following reparameterizations during the numerical optimization to estimate $\beta$ and c,

$$\beta = 1/(1+e^{-\eta}),$$

$$c = e^{\kappa}.$$

Let $I(\eta)$ and $I(\kappa)$ be the Fisher's information under parameterization $\eta$ and $\kappa$, then the Fisher's information of the original parameters are

$$I(\beta) = I(\eta) \ (1/(\beta(1-\beta))^2$$

$$I(c) = I(k) \ (1/c)^2.$$

Hence the implementations have the following transformation on top of the numerical optimization method for estimating stand deviations,

$$std(\beta) = std(\eta) \cdot \beta \cdot (1-\beta)$$

$$std(\beta) = std(\eta) \cdot c.$$

**Samples**

Samples used herein contain nucleic acids that are "cell-free" (e.g., cfDNA) or cell-bound (e.g., cellular DNA). Cell-free nucleic acids, including cell-free DNA, can be obtained by various methods known in the art from biological samples including but not limited to plasma, serum, and urine (see, e.g., Fan et al., Proc Natl Acad Sci 105:16266-16271 [2008]; Koide et al., Prenatal Diagnosis 25:604-607 [2005]; Chen et al., Nature Med. 2: 1033-1035 [1996]; Lo et al., Lancet 350: 485-487 [1997]; Botezatu et al., Clin Chem. 46: 1078-1084, 2000; and Su et al., J Mol. Diagn. 6: 101-107 [2004]). To separate cell-free DNA from cells in a sample, various methods including, but not limited to fractionation, centrifugation (e.g., density gradient centrifugation), DNA-specific precipitation, or high-throughput cell sorting and/or other separation methods can be used. Commercially available kits for manual and automated separation of cfDNA are available (Roche Diagnostics, Indianapolis, IN, Qiagen, Valencia, CA, Macherey-Nagel, Duren, DE). Biological samples comprising cfDNA have been used in assays to determine the presence or absence of chromosomal abnormalities, e.g., trisomy 21, by sequencing assays that can detect chromosomal aneuploidies and/or various polymorphisms.

In various embodiments the DNA present in the sample can be enriched specifically or non-specifically prior to use (e.g., prior to preparing a sequencing library). Non-specific enrichment of sample DNA refers to the whole genome amplification of the genomic DNA fragments of the sample that can be used to increase the level of the sample DNA prior to preparing a DNA sequencing library. Non-specific enrichment can be the selective enrichment of one of the two genomes present in a sample that comprises more than one genome. For example, non-specific enrichment can be selective of the cancer genome in a plasma sample, which can be obtained by known methods to increase the relative proportion of cancer to normal DNA in a sample. Alternatively, non-specific enrichment can be the non-selective amplification of both genomes present in the sample. For example, non-specific amplification can be of cancer and normal DNA in a sample comprising a mixture of DNA from the cancer and normal genomes. Methods for whole genome amplification are known in the art. Degenerate oligonucleotide-primed PCR (DOP), primer extension PCR technique (PEP) and multiple displacement amplification (MDA) are examples of whole genome amplification methods. In some embodiments, the sample comprising the mixture of cfDNA from different genomes is unenriched for cfDNA of the genomes present in the mixture. In other embodiments, the sample comprising the mixture of cfDNA from different genomes is non-specifically enriched for any one of the genomes present in the sample.

The sample comprising the nucleic acid(s) to which the methods described herein are applied typically comprises a biological sample ("test sample"), e.g., as described above. In some embodiments, the nucleic acid(s) to be analyzed is purified or isolated by any of a number of well-known methods.

Accordingly, in certain embodiments the sample comprises or consists of a purified or isolated polynucleotide, or it can comprise samples such as a tissue sample, a biological fluid sample, a cell sample, and the like. Suitable biological fluid samples include, but are not limited to blood, plasma, serum, sweat, tears, sputum, urine, sputum, ear flow, lymph, saliva, cerebrospinal fluid, ravages, bone marrow suspension, vaginal flow, trans-cervical lavage, brain fluid, ascites, milk, secretions of the respiratory, intestinal and genitourinary tracts, amniotic fluid, milk, and leukophoresis samples. In some embodiments, the sample is a sample that is easily obtainable by non-invasive procedures, e.g., blood, plasma, serum, sweat, tears, sputum, urine, sputum, ear flow, saliva or feces. In certain embodiments the sample is a peripheral blood sample, or the plasma and/or serum fractions of a peripheral blood sample. In other embodiments, the biological sample is a swab or smear, a biopsy specimen, or a cell culture. In another embodiment, the sample is a mixture of two or more biological samples, e.g., a biological sample can comprise two or more of a biological fluid sample, a tissue sample, and a cell culture sample. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

In certain embodiments, samples can be obtained from sources, including, but not limited to, samples from different individuals, samples from different developmental stages of the same or different individuals, samples from different diseased individuals (e.g., individuals with cancer or suspected of having a genetic disorder), normal individuals, samples obtained at different stages of a disease in an individual, samples obtained from an individual subjected to different treatments for a disease, samples from individuals subjected to different environmental factors, samples from individuals with predisposition to a pathology, samples individuals with exposure to an infectious disease agent (e.g., HIV), and the like.

In one illustrative, but non-limiting embodiment, the sample is a donee sample that is obtained from a donee of an organ transplant, such as a plasma sample from a donee, which includes cfDNA originating from the donee and cfDNA originating from a tissue or organ transplanted from the donor. In this instance, the sample can be analyzed using the methods described herein to quantify donee and donor DNA portions. The donee sample can be a tissue sample, a biological fluid sample, or a cell sample. A biological fluid includes, as non-limiting examples, blood, plasma, serum, sweat, tears, sputum, urine, sputum, ear flow, lymph, saliva, cerebrospinal fluid, ravages, bone marrow suspension, vaginal flow, transcervical lavage, brain fluid, ascites, milk, secretions of the respiratory, intestinal and genitourinary tracts, and leukophoresis samples.

In another illustrative, but non-limiting embodiment, the donee sample is a mixture of two or more biological samples, e.g., the biological sample can comprise two or more of a biological fluid sample, a tissue sample, and a cell culture sample. In some embodiments, the sample is a sample that is easily obtainable by non-invasive procedures, e.g., blood, plasma, serum, sweat, tears, sputum, urine, milk, sputum, ear flow, saliva and feces. In some embodiments, the biological sample is a peripheral blood sample, and/or the plasma and serum fractions thereof. In other embodiments, the biological sample is a swab or smear, a biopsy specimen, or a sample of a cell culture. As disclosed above, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

In certain embodiments samples can also be obtained from in vitro cultured tissues, cells, or other polynucleotide-containing sources. The cultured samples can be taken from sources including, but not limited to, cultures (e.g., tissue or cells) maintained in different media and conditions (e.g., pH, pressure, or temperature), cultures (e.g., tissue or cells) maintained for different periods of length, cultures (e.g., tissue or cells) treated with different factors or reagents (e.g., a drug candidate, or a modulator), or cultures of different types of tissue and/or cells.

Methods of isolating nucleic acids from biological sources are well known and will differ depending upon the nature of the source. One of skill in the art can readily isolate nucleic acid(s) from a source as needed for the method described herein. In some instances, it can be advantageous to fragment the nucleic acid molecules in the nucleic acid sample. Fragmentation can be random, or it can be specific, as achieved, for example, using restriction endonuclease digestion. Methods for random fragmentation are well known in the art, and include, for example, limited DNAse digestion, alkali treatment and physical shearing. In one embodiment, sample nucleic acids are obtained from as cfDNA, which is not subjected to fragmentation.

## Sequencing Library Preparation

In one embodiment, the methods described herein can utilize next generation sequencing technologies (NGS), that allow multiple samples to be sequenced individually as genomic molecules (i.e., singleplex sequencing) or as pooled samples comprising indexed genomic molecules (e.g., multiplex sequencing) on a single sequencing run. These methods can generate up to several hundred million reads of DNA sequences. In various embodiments the sequences of genomic nucleic acids, and/or of indexed genomic nucleic acids can be determined using, for example, the Next Generation Sequencing Technologies (NGS) described herein. In various embodiments analysis of the massive amount of sequence data obtained using NGS can be performed using one or more processors as described herein.

In various embodiments the use of such sequencing technologies does not involve the preparation of sequencing libraries.

However, in certain embodiments the sequencing methods contemplated herein involve the preparation of sequencing libraries. In one illustrative approach, sequencing library preparation involves the production of a random collection of adapter-modified DNA fragments (e.g., polynucleotides) that are ready to be sequenced. Sequencing libraries of polynucleotides can be prepared from DNA or RNA, including equivalents, analogs of either DNA or cDNA, for example, DNA or cDNA that is complementary or copy DNA produced from an RNA template, by the action of reverse transcriptase. The polynucleotides may originate in double-stranded form (e.g., dsDNA such as genomic DNA fragments, cDNA, PCR amplification products, and the like) or, in certain embodiments, the polynucleotides may originated in single-stranded form (e.g., ssDNA, RNA, etc.) and have been converted to dsDNA form. By way of illustration, in certain embodiments, single stranded mRNA molecules may be copied into double-stranded cDNAs suitable for use in preparing a sequencing library. The precise sequence of the primary polynucleotide molecules is generally not material to the method of library preparation, and may be known or unknown. In one embodiment, the polynucleotide molecules are DNA molecules. More particularly, in certain embodiments, the polynucleotide molecules represent the entire genetic complement of an organism or substantially the entire genetic complement of an organism, and are genomic DNA molecules (e.g., cellular DNA, cell free DNA (cfDNA), etc.), that typically include both intron sequence and exon sequence (coding sequence), as well as noncoding regulatory sequences such as promoter and enhancer sequences. In certain embodiments, the primary polynucleotide molecules comprise human genomic DNA molecules, e.g., cfDNA molecules present in peripheral blood of a pregnant subject.

Preparation of sequencing libraries for some NGS sequencing platforms is facilitated by the use of polynucleotides comprising a specific range of fragment sizes. Preparation of such libraries typically involves the fragmentation of large polynucleotides (e.g. cellular genomic DNA) to obtain polynucleotides in the desired size range.

Fragmentation can be achieved by any of a number of methods known to those of skill in the art. For example, fragmentation can be achieved by mechanical means including, but not limited to nebulization, sonication and hydroshear. However mechanical fragmentation typically cleaves the DNA backbone at C-O, P-O and C-C bonds resulting in a heterogeneous mix of blunt and 3'- and 5'-overhanging ends with broken C-O, P-O and/ C-C bonds (see, e.g., Alnemri and Liwack, J Biol. Chem 265:17323-17333 [1990]; Richards and Boyer, J Mol Biol 11:327-240 [1965]) which may need to be repaired as they may lack the requisite 5'-phosphate for the subsequent enzymatic reactions, e.g., ligation of sequencing adaptors, that are required for preparing DNA for sequencing.

In contrast, cfDNA, typically exists as fragments of less than about 300 base pairs and consequently, fragmentation is not typically necessary for generating a sequencing library using cfDNA samples.

Typically, whether polynucleotides are forcibly fragmented (e.g., fragmented in vitro), or naturally exist as fragments, they are converted to blunt-ended DNA having 5'-phosphates and 3'-hydroxyl. Standard protocols, e.g., protocols for sequencing using, for example, the Illumina platform as described elsewhere herein, instruct users to end-repair sample DNA, to purify the end-repaired products prior to dA-tailing, and to purify the dA-tailing products prior to the adaptor-ligating steps of the library preparation.

Various embodiments of methods of sequence library preparation described herein obviate the need to perform one or more of the steps typically mandated by standard protocols to obtain a modified DNA product that can be sequenced by NGS. An abbreviated method (ABB method), a 1-step method, and a 2-step method are examples of methods for preparation of a sequencing library, which can be found in patent application 13/555,037 filed on July 20, 2012.

## Sequencing Methods

As indicated above, the prepared samples (e.g., Sequencing Libraries) are sequenced as part of the procedure for quantifying and deconvolving DNA mixture samples. Any of a number of sequencing technologies can be utilized.

Some sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies include, but are not limited to, the SMRT™ technology of Pacific Biosciences, the ION TORRENT™ technology, and nanopore sequencing developed for example, by Oxford Nanopore Technologies.

While the automated Sanger method is considered as a 'first generation' technology, Sanger sequencing including the automated Sanger sequencing, can also be employed in the methods described herein. Additional suitable sequencing methods include, but are not limited to nucleic acid imaging technologies, e.g., atomic force microscopy (AFM) or transmission electron microscopy (TEM). Illustrative sequencing technologies are described in greater detail below.

In one illustrative, but non-limiting, embodiment, the methods described herein comprise obtaining sequence information for the nucleic acids in a test sample, e.g., cfDNA in a donee sample including donor DNA and donee DNA, cfDNA or cellular DNA in a subject being screened for a cancer, and the like, using Illumina's sequencing-by-synthesis and reversible terminator-based sequencing chemistry (e.g. as described in Bentley et al., Nature 6:53-59 [2009]). Template DNA can be genomic DNA, e.g., cellular DNA or cfDNA. In some embodiments, genomic DNA from isolated cells is used as the template, and it is fragmented into lengths of several hundred base pairs. In other embodiments, cfDNA is used as the template, and fragmentation is not required as cfDNA exists as short fragments. For example fetal cfDNA circulates in the bloodstream as fragments approximately 170 base pairs (bp) in length (Fan et al., Clin Chem 56:1279-1286 [2010]), and no fragmentation of the DNA is required prior to sequencing. Circulating tumor DNA also exist in short fragments, with a size distribution peaking at about 150-170bp. Illumina's sequencing technology relies on the attachment of fragmented genomic DNA to a planar, optically transparent surface on which oligonucleotide anchors are bound. Template DNA is end-repaired to generate 5'-phosphorylated blunt ends, and the polymerase activity of Klenow fragment is used to add a single A base to the 3' end of the blunt phosphorylated DNA fragments. This addition prepares the DNA fragments for ligation to oligonucleotide adapters, which have an overhang of a single T base at their 3' end to increase ligation efficiency. The adapter oligonucleotides are complementary to the flow-cell anchor oligos (not to be confused with the anchor/anchored reads in the analysis of repeat expansion). Under limiting-dilution conditions, adapter-modified, single-stranded template DNA is added to the flow cell and immobilized by hybridization to the anchor oligos. Attached DNA fragments are extended and bridge amplified to create an ultra-high density sequencing flow cell with hundreds of millions of clusters, each containing about 1,000 copies of the same template. In one embodiment, the randomly fragmented genomic DNA is amplified using PCR before it is subjected to cluster amplification. Alternatively, an amplification-free (e.g., PCR free) genomic library preparation is used, and the randomly fragmented genomic DNA is enriched using the cluster amplification alone (Kozarewa et al., Nature Methods 6:291-295 [2009]). The templates are sequenced using a robust four-color DNA sequencing-by-synthesis technology that employs reversible terminators with removable fluorescent dyes. High-sensitivity fluorescence detection is achieved using laser excitation and total internal reflection optics. Short sequence reads of about tens to a few hundred base pairs are aligned against a reference genome and unique mapping of the short sequence reads to the reference genome are identified using specially developed data analysis pipeline software. After completion of the first read, the templates can be regenerated in situ to enable a second read from the opposite end of the fragments. Thus, either single-end or paired end sequencing of the DNA fragments can be used.

Various embodiments of the disclosure may use sequencing by synthesis that allows paired end sequencing. In some embodiments, the sequencing by synthesis platform by Illumina involves clustering fragments. Clustering is a process in which each fragment molecule is isothermally amplified. In some embodiments, as the example described here, the fragment has two different adaptors attached to the two ends of the fragment, the adaptors allowing the fragment to hybridize with the two different oligos on the surface of a flow cell lane. The fragment further includes or is connected to two index sequences at two ends of the fragment, which index sequences provide labels to identify different samples in multiplex sequencing. In some sequencing platforms, a fragment to be sequenced is also referred to as an insert.

In some implementation, a flow cell for clustering in the Illumina platform is a glass slide with lanes. Each lane is a glass channel coated with a lawn of two types of oligos. Hybridization is enabled by the first of the two types of oligos on the surface. This oligo is complementary to a first adapter on one end of the fragment. A polymerase creates a compliment strand of the hybridized fragment. The double-stranded molecule is denatured, and the original template strand is washed away. The remaining strand, in parallel with many other remaining strands, is clonally amplified through bridge application.

In bridge amplification, a strand folds over, and a second adapter region on a second end of the strand hybridizes with the second type of oligos on the flow cell surface. A polymerase generates a complimentary strand, forming a double-stranded bridge molecule. This double-stranded molecule is denatured resulting in two single-stranded molecules tethered to the flow cell through two different oligos. The process is then repeated over and over, and occurs simultaneously for millions of clusters resulting in clonal amplification of all the fragments. After bridge amplification, the reverse strands are cleaved and washed off, leaving only the forward strands. The 3' ends are blocked to prevent unwanted priming.

After clustering, sequencing starts with extending a first sequencing primer to generate the first read. With each cycle, fluorescently tagged nucleotides compete for addition to the growing chain. Only one is incorporated based on the sequence of the template. After the addition of each nucleotide, the cluster is excited by a light source, and a characteristic fluorescent signal is emitted. The number of cycles determines the length of the read. The emission wavelength and the signal intensity determine the base call. For a given cluster all identical strands are read simultaneously. Hundreds of millions of clusters are sequenced in a massively parallel manner. At the completion of the first read, the read product is washed away.

In the next step of protocols involving two index primers, an index 1 primer is introduced and hybridized to an index 1 region on the template. Index regions provide identification of fragments, which is useful for de-multiplexing samples in a multiplex sequencing process. The index 1 read is generated similar to the first read. After completion of the index 1 read, the read product is washed away and the 3' end of the strand is de-protected. The template strand then folds over and binds to a second oligo on the flow cell. An index 2 sequence is read in the same manner as index 1. Then an index 2 read product is washed off at the completion of the step.

After reading two indices, read 2 initiates by using polymerases to extend the second flow cell oligos, forming a double-stranded bridge. This double-stranded DNA is denatured, and the 3' end is blocked. The original forward strand is cleaved off and washed away, leaving the reverse strand. Read 2 begins with the introduction of a read 2 sequencing primer. As with read 1, the sequencing steps are repeated until the desired length is achieved. The read 2 product is washed away. This entire process generates millions of reads, representing all the fragments. Sequences from pooled sample libraries are separated based on the unique indices introduced during sample preparation. For each sample, reads of similar stretches of base calls are locally clustered. Forward and reversed reads are paired creating contiguous sequences. These contiguous sequences are aligned to the reference genome for variant identification.

The sequencing by synthesis example described above involves paired end reads, which is used in many of the embodiments of the disclosed methods. Paired end sequencing involves two reads from the two ends of a fragment. When a pair of reads are mapped to a reference sequence, the base-pair distance between the two reads can be determined, which distance can then be used to determine the length of the fragments from which the reads were obtained. In some instances, a fragment straddling two bins would have one of its pair-end read aligned to one bin, and another to an adjacent bin. This gets rarer as the bins get longer or the reads get shorter. Various methods may be used to account for the bin-membership of these fragments. For instance, they can be omitted in determining fragment size frequency of a bin; they can be counted for both of the adjacent bins; they can be assigned to the bin that encompasses the larger number of base pairs of the two bins; or they can be assigned to both bins with a weight related to portion of base pairs in each bin.

Paired end reads may use insert of different length (i.e., different fragment size to be sequenced). As the default meaning in this disclosure, paired end reads are used to refer to reads obtained from various insert lengths. In some instances, to distinguish short-insert paired end reads from long-inserts paired end reads, the latter is also referred to as mate pair reads. In some embodiments involving mate pair reads, two biotin junction adaptors first are attached to two ends of a relatively long insert (e.g., several kb). The biotin junction adaptors then link the two ends of the insert to form a circularized molecule. A sub-fragment encompassing the biotin junction adaptors can then be obtained by further fragmenting the circularized molecule. The sub-fragment including the two ends of the original fragment in opposite sequence order can then be sequenced by the same procedure as for short-insert paired end sequencing described above. Further details of mate pair sequencing using an Illumina platform is shown in an online publication at the following URL: res|.|illumina|.|com/documents/products/technotes/technote_nextera_matepair_data_processing. Additional information about paired end sequencing can be found in US Patent No. 7601499 and US Patent Publication No. 2012/0,053,063, with regard to materials on paired end sequencing methods and apparatuses.

After sequencing of DNA fragments, sequence reads of predetermined length, e.g., 100 bp, are mapped or aligned to a known reference genome. The mapped or aligned reads and their corresponding locations on the reference sequence are also referred to as tags. In one embodiment, the reference genome sequence is the NCBI36/hg18 sequence, which is available on the world wide web at genome dot ucsc dot edu/cgi-bin/hgGateway?org=Human&db=hg18&hgsid=166260105). Alternatively, the reference genome sequence is the GRCh37/hg19, which is available on the World Wide Web at genome dot ucsc dot edu/cgi-bin/hgGateway. Other sources of public sequence information include GenBank, dbEST, dbSTS, EMBL (the European Molecular Biology Laboratory), and the DDBJ (the DNA Databank of Japan). A number of computer programs are available for aligning sequences, including but not limited to BLAST (Altschul et al., 1990), BLITZ (MPsrch) (Sturrock & Collins, 1993), FASTA (Person & Lipman, 1988), BOWTIE (Langmead et al., Genome Biology 10:R25.1-R25.10 [2009]), or ELAND (Illumina, Inc., San Diego, CA, USA). In one embodiment, one end of the clonally expanded copies of the plasma cfDNA molecules is sequenced and processed by bioinformatics alignment analysis for the Illumina Genome Analyzer, which uses the Efficient Large-Scale Alignment of Nucleotide Databases (ELAND) software.

In one illustrative, but non-limiting, embodiment, the methods described herein comprise obtaining sequence information for the nucleic acids in a test sample, e.g., cfDNA in a donee sample including donee and donor DNA, cfDNA or cellular DNA in a subject being screened for a cancer, and the like, using single molecule sequencing technology of the Helicos True Single Molecule Sequencing (tSMS) technology (e.g. as described in Harris T.D. et al., Science 320:106-109 [2008]). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. In certain embodiments the templates can be at a density of about 100 million templates/cm2. The flow cell is then loaded into an instrument, e.g., HeliScope™ sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are discerned by imaging the flow cell surface. After imaging, a cleavage step removes the fluorescent label, and the process is repeated with other fluorescently labeled nucleotides until the desired read length is achieved. Sequence information is collected with each nucleotide addition step. Whole genome sequencing by single molecule sequencing technologies excludes or typically obviates PCR-based amplification in the preparation of the sequencing libraries, and the methods allow for direct measurement of the sample, rather than measurement of copies of that sample.

In another illustrative, but non-limiting embodiment, the methods described herein comprise obtaining sequence information for the nucleic acids in the test sample, e.g., cfDNA in a donee test sample including donee and donor DNA, cfDNA or cellular DNA in a subject being screened for a cancer, and the like, using the 454 sequencing (Roche) (e.g. as described in Margulies, M. et al. Nature 437:376-380 [2005]). 454 sequencing typically involves two steps. In the first step, DNA is sheared into fragments of approximately 300-800 base pairs, and the fragments are blunt-ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (e.g., picoliter-sized wells). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi), which is released upon nucleotide addition. PPi is converted to ATP by ATP sulfurylase in the presence of adenosine 5' phosphosulfate. Luciferase uses ATP to convert luciferin to oxyluciferin, and this reaction generates light that is measured and analyzed.

In another illustrative, but non-limiting, embodiment, the methods described herein comprises obtaining sequence information for the nucleic acids in the test sample, e.g., cfDNA in a donee test sample, cfDNA or cellular DNA in a subject being screened for a cancer, and the like, using the SOLiD™ technology (Applied Biosystems). In SOLiD™ sequencing-by-ligation, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

In another illustrative, but non-limiting, embodiment, the methods described herein comprise obtaining sequence information for the nucleic acids in the test sample, e.g., cfDNA in a donee test sample, cfDNA or cellular DNA in a subject being screened for a cancer, and the like, using the single molecule, real-time (SMRT™) sequencing technology of Pacific Biosciences. In SMRT sequencing, the continuous incorporation of dye-labeled nucleotides is imaged during DNA synthesis. Single DNA polymerase molecules are attached to the bottom surface of individual zero-mode wavelength detectors (ZMW detectors) that obtain sequence information while phospholinked nucleotides are being incorporated into the growing primer strand. A ZMW detector comprises a confinement structure that enables observation of incorporation of a single nucleotide by DNA polymerase against a background of fluorescent nucleotides that rapidly diffuse in an out of the ZMW (e.g., in microseconds). It typically takes several milliseconds to incorporate a nucleotide into a growing strand. During this time, the fluorescent label is excited and produces a fluorescent signal, and the fluorescent tag is cleaved off. Measurement of the corresponding fluorescence of the dye indicates which base was incorporated. The process is repeated to provide a sequence.

In another illustrative, but non-limiting embodiment, the methods described herein comprise obtaining sequence information for the nucleic acids in the test sample, e.g., cfDNA in a maternal or donee test sample, cfDNA or cellular DNA in a subject being screened for a cancer, and the like, using nanopore sequencing (e.g. as described in Soni GV and Meller A. Clin Chem 53: 1996-2001 [2007]). Nanopore sequencing DNA analysis techniques are developed by a number of companies, including, for example, Oxford Nanopore Technologies (Oxford, United Kingdom), Sequenom, NABsys, and the like. Nanopore sequencing is a single-molecule sequencing technology whereby a single molecule of DNA is sequenced directly as it passes through a nanopore. A nanopore is a small hole, typically of the order of 1 nanometer in diameter. Immersion of a nanopore in a conducting fluid and application of a potential (voltage) across it results in a slight electrical current due to conduction of ions through the nanopore. The amount of current that flows is sensitive to the size and shape of the nanopore. As a DNA molecule passes through a nanopore, each nucleotide on the DNA molecule obstructs the nanopore to a different degree, changing the magnitude of the current through the nanopore in different degrees. Thus, this change in the current as the DNA molecule passes through the nanopore provides a read of the DNA sequence.

In another illustrative, but non-limiting, embodiment, the methods described herein comprises obtaining sequence information for the nucleic acids in the test sample, e.g., cfDNA in a donee test sample, cfDNA or cellular DNA in a subject being screened for a cancer, and the like, using the chemical-sensitive field effect transistor (chemFET) array (e.g., as described in U.S. Patent Application Publication No. 2009/0026082). In one example of this technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be discerned as a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

In another embodiment, the present method comprises obtaining sequence information for the nucleic acids in the test sample, e.g., cfDNA in a donee test sample, using transmission electron microscopy (TEM). The method, termed Individual Molecule Placement Rapid Nano Transfer (IMPRNT), comprises utilizing single atom resolution transmission electron microscope imaging of high-molecular weight (150kb or greater) DNA selectively labeled with heavy atom markers and arranging these molecules on ultra-thin films in ultra-dense (3nm strand-to-strand) parallel arrays with consistent base-to-base spacing. The electron microscope is used to image the molecules on the films to determine the position of the heavy atom markers and to extract base sequence information from the DNA. The method is further described in PCT patent publication WO 2009/046445. The method allows for sequencing complete human genomes in less than ten minutes.

In another embodiment, the DNA sequencing technology is the Ion Torrent single molecule sequencing, which pairs semiconductor technology with a simple sequencing chemistry to directly translate chemically encoded information (A, C, G, T) into digital information (0, 1) on a semiconductor chip. In nature, when a nucleotide is incorporated into a strand of DNA by a polymerase, a hydrogen ion is released as a byproduct. Ion Torrent uses a high-density array of micro-machined wells to perform this biochemical process in a massively parallel way. Each well holds a different DNA molecule. Beneath the wells is an ion-sensitive layer and beneath that an ion sensor. When a nucleotide, for example a C, is added to a DNA template and is then incorporated into a strand of DNA, a hydrogen ion will be released. The charge from that ion will change the pH of the solution, which can be detected by Ion Torrent's ion sensor. The sequencer-essentially the world's smallest solid-state pH meter-calls the base, going directly from chemical information to digital information. The Ion personal Genome Machine (PGM™) sequencer then sequentially floods the chip with one nucleotide after another. If the next nucleotide that floods the chip is not a match. No voltage change will be recorded and no base will be called. If there are two identical bases on the DNA strand, the voltage will be double, and the chip will record two identical bases called. Direct detection allows recordation of nucleotide incorporation in seconds.

In another embodiment, the present method comprises obtaining sequence information for the nucleic acids in the test sample, e.g., cfDNA in a donee test sample, using sequencing by hybridization. Sequencing-by-hybridization comprises contacting the plurality of polynucleotide sequences with a plurality of polynucleotide probes, wherein each of the plurality of polynucleotide probes can be optionally tethered to a substrate. The substrate might be flat surface comprising an array of known nucleotide sequences. The pattern of hybridization to the array can be used to determine the polynucleotide sequences present in the sample. In other embodiments, each probe is tethered to a bead, e.g., a magnetic bead or the like. Hybridization to the beads can be determined and used to identify the plurality of polynucleotide sequences within the sample.

In some embodiments of the methods described herein, the mapped sequence tags comprise sequence reads of about 20bp, about 25bp, about 30bp, about 35bp, about 40bp, about 45bp, about 50bp, about 55bp, about 60bp, about 65bp, about 70bp, about 75bp, about 80bp, about 85bp, about90bp, about 95bp, about 100bp, about 110bp, about 120bp, about 130, about 140bp, about 150bp, about 200bp, about 250bp, about 300bp, about 350bp, about 400bp, about 450bp, or about 500bp. It is expected that technological advances will enable single-end reads of greater than 500bp enabling for reads of greater than about 1000bp when paired end reads are generated. In one embodiment, the mapped sequence tags comprise sequence reads that are 36bp. Mapping of the sequence tags is achieved by comparing the sequence of the tag with the sequence of the reference to determine the chromosomal origin of the sequenced nucleic acid (e.g. cfDNA) molecule, and specific genetic sequence information is not needed. A small degree of mismatch (0-2 mismatches per sequence tag) may be allowed to account for minor polymorphisms that may exist between the reference genome and the genomes in the mixed sample.

A plurality of sequence tags are typically obtained per sample. In some embodiments, at least about $1 \times 10^5$ sequence tags comprising between 75bp read are obtained from mapping the reads to the reference genome per sample.

The accuracy required for correctly quantifying DNA mixture samples, is predicated on the variation of the number of sequence tags that map to the reference genome among samples within a sequencing run (inter-run variability), and the variation of the number of sequence tags that map to the reference genome in different sequencing runs (inter-run variability). Other variations can result from using different protocols for the extraction and purification of the nucleic acids, the preparation of the sequencing libraries, and the use of different sequencing platforms.

**Apparatus and System for Deconvolving and Quantifying Mixtures of Nucleic Acid from Multiple Sources**

Analysis of the sequencing data and the diagnosis derived therefrom are typically performed using various computer programs. Therefore, certain embodiments employ processes involving data stored in or transferred through one or more computer systems or other processing systems. Embodiments disclosed herein also relate to apparatus for performing these operations. This apparatus may be specially constructed for the required purposes, or it may be a general-purpose computer (or a group of computers) selectively activated or reconfigured by a computer program and/or data structure stored in the computer. In some embodiments, a group of processors performs some or all of the recited analytical operations collaboratively (e.g., via a network or cloud computing) and/or in parallel. A processor or group of processors for performing the methods described herein may be of various types including microcontrollers and microprocessors such as programmable devices (e.g., CPLDs and FPGAs) and non-programmable devices such as gate array ASICs or general purpose microprocessors.

In addition, certain embodiments relate to tangible and/or non-transitory computer readable media or computer program products that include program instructions and/or data (including data structures) for performing various computer-implemented operations. Examples of computer-readable media include, but are not limited to, semiconductor memory devices, magnetic media such as disk drives, magnetic tape, optical media such as CDs, magneto-optical media, and hardware devices that are specially configured to store and perform program instructions, such as read-only memory devices (ROM) and random access memory (RAM). The computer readable media may be directly controlled by an end user or the media may be indirectly controlled by the end user. Examples of directly controlled media include the media located at a user facility and/or media that are not shared with other entities. Examples of indirectly controlled media include media that is indirectly accessible to the user via an external network and/or via a service providing shared resources such as the "cloud." Examples of program instructions include both machine code, such as produced by a compiler, and files containing higher level code that may be executed by the computer using an interpreter.

In various embodiments, the data or information employed in the disclosed methods and apparatus is provided in an electronic format. Such data or information may include reads and tags derived from a nucleic acid sample, counts or densities of such tags that align with particular regions of a reference sequence (e.g., that align to a chromosome or chromosome segment), reference sequences (including reference sequences providing solely or primarily polymorphisms), calls such as SNV or aneuploidy calls, counseling recommendations, diagnoses, and the like. As used herein, data or other information provided in electronic format is available for storage on a machine and transmission between machines. Conventionally, data in electronic format is provided digitally and may be stored as bits and/or bytes in various data structures, lists, databases, etc. The data may be embodied electronically, optically, etc.

One embodiment provides a computer program product for generating an output indicating the presence or absence of an SNV or aneuploidy associated with a cancer, in a test sample. The computer product may contain instructions for performing any one or more of the above-described methods for determining a chromosomal anomaly. As explained, the computer product may include a non-transitory and/or tangible computer readable medium having a computer executable or compilable logic (e.g., instructions) recorded thereon for enabling a processor to quantify DNA mixture samples. In one example, the computer product comprises a computer readable medium having a computer executable or compilable logic (e.g., instructions) recorded thereon for enabling a processor to quantify DNA mixture samples.

The sequence information from the sample under consideration may be mapped to chromosome reference sequences to identify a number of sequence tags for each of any one or more chromosomes of interest. In various embodiments, the reference sequences are stored in a database such as a relational or object database, for example.

It should be understood that it is not practical, or even possible in most cases, for an unaided human being to perform the computational operations of the methods disclosed herein. For example, mapping a single 30 bp read from a sample to any one of the human chromosomes might require years of effort without the assistance of a computational apparatus.

The methods disclosed herein can be performed using a system for quantifying DNA mixture samples. The system comprising: (a) a sequencer for receiving nucleic acids from the test sample providing nucleic acid sequence information from the sample; (b) a processor; and (c) one or more computer-readable storage media having stored thereon instructions for execution on said processor to carry out a method for quantifying DNA mixture samples.

In some embodiments, the methods are instructed by a computer-readable medium having stored thereon computer-readable instructions for carrying out a method for quantifying DNA mixture samples. Thus one embodiment provides a computer program product comprising one or more computer-readable non-transitory storage media having stored thereon computer-executable instructions that, when executed by one or more processors of a computer system, cause the computer system to implement a method for quantifying DNA mixture samples. The method includes: (a) extracting nucleic acid molecules from the nucleic acid sample; (b) amplifying the extracted nucleic acid molecules; (c) sequencing the amplified nucleic acid molecules using a nucleic acid sequencer to produce nucleic acid sequence reads; (d) mapping, by the one or more processors, the nucleic acid sequence reads to one or more polymorphism loci on a reference sequence; (e) determining, using the mapped nucleic acid sequence reads and by the one or more processors, allele counts of nucleic acid sequence reads for one or more alleles at the one or more polymorphism loci; and (f) quantifying, using a probabilistic mixture model and by the one or more processors, one or more fractions of nucleic acid of the one or more contributors in the nucleic acid sample, wherein using the probabilistic mixture model comprises applying a probabilistic mixture model to the allele counts of nucleic acid sequence reads, and wherein the probabilistic mixture model uses probability distributions to model the allele counts of nucleic acid sequence reads at the one or more polymorphism loci, the probability distributions accounting for errors in the nucleic acid sequence read sequences and counts.

In some embodiments, the instructions may further include automatically recording information pertinent to the method in a patient medical record for a human subject providing the donee test sample. The patient medical record may be maintained by, for example, a laboratory, physician's office, a hospital, a health maintenance organization, an insurance company, or a personal medical record website. Further, based on the results of the processor-implemented analysis, the method may further involve prescribing, initiating, and/or altering treatment of a human subject from whom the donee test sample was taken. This may involve performing one or more additional tests or analyses on additional samples taken from the subject.

Disclosed methods can also be performed using a computer processing system which is adapted or configured to perform a method for quantifying DNA mixture samples. One embodiment provides a computer processing system, which is adapted or configured to perform a method as described herein. In one embodiment, the apparatus comprises a sequencing device adapted or configured for sequencing at least a portion of the nucleic acid molecules in a sample to obtain the type of sequence information described elsewhere herein. The apparatus may also include components for processing the sample. Such components are described elsewhere herein.

Sequence or other data, can be input into a computer or stored on a computer readable medium either directly or indirectly. In one embodiment, a computer system is directly coupled to a sequencing device that reads and/or analyzes sequences of nucleic acids from samples. Sequences or other information from such tools are provided via interface in the computer system. Alternatively, the sequences processed by system are provided from a sequence storage source such as a database or other repository. Once available to the processing apparatus, a memory device or mass storage device buffers or stores, at least temporarily, sequences of the nucleic acids. In addition, the memory device may store tag counts for various chromosomes or genomes, etc. The memory may also store various routines and/or programs for analyzing the presenting the sequence or mapped data. Such programs/routines may include programs for performing statistical analyses, etc.

In one example, a user provides a sample into a sequencing apparatus. Data is collected and/or analyzed by the sequencing apparatus, which is connected to a computer. Software on the computer allows for data collection and/or analysis. Data can be stored, displayed (via a monitor or other similar device), and/or sent to another location. The computer may be connected to the internet which is used to transmit data to a handheld device utilized by a remote user (e.g., a physician, scientist or analyst). It is understood that the data can be stored and/or analyzed prior to transmittal. In some embodiments, raw data is collected and sent to a remote user or apparatus that will analyze and/or store the data. Transmittal can occur via the internet, but can also occur via satellite or other connection. Alternately, data can be stored on a computer-readable medium and the medium can be shipped to an end user (e.g., via mail). The remote user can be in the same or a different geographical location including, but not limited to a building, city, state, country or continent.

In some embodiments, the methods also include collecting data regarding a plurality of polynucleotide sequences (e.g., reads, tags and/or reference chromosome sequences) and sending the data to a computer or other computational system. For example, the computer can be connected to laboratory equipment, e.g., a sample collection apparatus, a nucleotide amplification apparatus, a nucleotide sequencing apparatus, or a hybridization apparatus. The computer can then collect applicable data gathered by the laboratory device. The data can be stored on a computer at any step, e.g., while collected in real time, prior to the sending, during or in conjunction with the sending, or following the sending. The data can be stored on a computer-readable medium that can be extracted from the computer. The data collected or stored can be transmitted from the computer to a remote location, e.g., via a local network or a wide area network such as the internet. At the remote location various operations can be performed on the transmitted data as described below.

Among the types of electronically formatted data that may be stored, transmitted, analyzed, and/or manipulated in systems, apparatus, and methods disclosed herein are the following:

Reads obtained by sequencing nucleic acids in a test sample

Tags obtained by aligning reads to a reference genome or other reference sequence or sequences

The reference genome or sequence

Allele counts - Counts or numbers of tags for each allele and regions of a reference genome or other reference sequences

Determined contributor nucleic acid fractions and the associated confidence intervals

Diagnoses (clinical condition associated with the calls)

Recommendations for further tests derived from the calls and/or diagnoses

Treatment and/or monitoring plans derived from the calls and/or diagnoses

These various types of data may be obtained, stored transmitted, analyzed, and/or manipulated at one or more locations using distinct apparatus. The processing options span a wide spectrum. At one end of the spectrum, all or much of this information is stored and used at the location where the test sample is processed, e.g., a doctor's office or other clinical setting. In other extreme, the sample is obtained at one location, it is processed and optionally sequenced at a different location, reads are aligned and calls are made at one or more different locations, and diagnoses, recommendations, and/or plans are prepared at still another location (which may be a location where the sample was obtained).

In various embodiments, the reads are generated with the sequencing apparatus and then transmitted to a remote site where they are processed to produce calls. At this remote location, as an example, the reads are aligned to a reference sequence to produce tags, which are counted and assigned to chromosomes or segments of interest. Also at the remote location, the doses are used to generate calls.

Among the processing operations that may be employed at distinct locations are the following:

Sample collection

Sample processing preliminary to sequencing

Sequencing

Analyzing sequence data and quantifying DNA mixture samples

Diagnosis

Reporting a diagnosis and/or a call to patient or health care provider

Developing a plan for further treatment, testing, and/or monitoring

Executing the plan

Counseling

Any one or more of these operations may be automated as described elsewhere herein. Typically, the sequencing and the analyzing of sequence data and quantifying DNA mixture samples will be performed computationally. The other operations may be performed manually or automatically.

Examples of locations where sample collection may be performed include health practitioners' offices, clinics, patients' homes (where a sample collection tool or kit is provided), and mobile health care vehicles. Examples of locations where sample processing prior to sequencing may be performed include health practitioners' offices, clinics, patients' homes (where a sample processing apparatus or kit is provided), mobile health care vehicles, and facilities of DNA analysis providers. Examples of locations where sequencing may be performed include health practitioners' offices, clinics, health practitioners' offices, clinics, patients' homes (where a sample sequencing apparatus and/or kit is provided), mobile health care vehicles, and facilities of DNA analysis providers. The location where the sequencing takes place may be provided with a dedicated network connection for transmitting sequence data (typically reads) in an electronic format. Such connection may be wired or wireless and have and may be configured to send the data to a site where the data can be processed and/or aggregated prior to transmission to a processing site. Data aggregators can be maintained by health organizations such as Health Maintenance Organizations (HMOs).

The analyzing and/or deriving operations may be performed at any of the foregoing locations or alternatively at a further remote site dedicated to computation and/or the service of analyzing nucleic acid sequence data. Such locations include for example, clusters such as general purpose server farms, the facilities of a DNA analysis service business, and the like. In some embodiments, the computational apparatus employed to perform the analysis is leased or rented. The computational resources may be part of an internet accessible collection of processors such as processing resources colloquially known as the cloud. In some cases, the computations are performed by a parallel or massively parallel group of processors that are affiliated or unaffiliated with one another. The processing may be accomplished using distributed processing such as cluster computing, grid computing, and the like. In such embodiments, a cluster or grid of computational resources collective form a super virtual computer composed of multiple processors or computers acting together to perform the analysis and/or derivation described herein. These technologies as well as more conventional supercomputers may be employed to process sequence data as described herein. Each is a form of parallel computing that relies on processors or computers. In the case of grid computing these processors (often whole computers) are connected by a network (private, public, or the Internet) by a conventional network protocol such as Ethernet. By contrast, a supercomputer has many processors connected by a local high-speed computer bus.

In certain embodiments, the diagnosis is generated at the same location as the analyzing operation. In other embodiments, it is performed at a different location. In some examples, reporting the diagnosis is performed at the location where the sample was taken, although this need not be the case. Examples of locations where the diagnosis can be generated or reported and/or where developing a plan is performed include health practitioners' offices, clinics, internet sites accessible by computers, and handheld devices such as cell phones, tablets, smart phones, etc. having a wired or wireless connection to a network. Examples of locations where counseling is performed include health practitioners' offices, clinics, internet sites accessible by computers, handheld devices, etc.

In some embodiments, the sample collection, sample processing, and sequencing operations are performed at a first location and the analyzing and deriving operation is performed at a second location. However, in some cases, the sample collection is collected at one location (e.g., a health practitioner's office or clinic) and the sample processing and sequencing is performed at a different location that is optionally the same location where the analyzing and deriving take place.

In various embodiments, a sequence of the above-listed operations may be triggered by a user or entity initiating sample collection, sample processing and/or sequencing. After one or more these operations have begun execution the other operations may naturally follow. For example, the sequencing operation may cause reads to be automatically collected and sent to a processing apparatus which then conducts, often automatically and possibly without further user intervention, the sequence analysis and quantifying DNA mixture samples. In some implementations, the result of this processing operation is then automatically delivered, possibly with reformatting as a diagnosis, to a system component or entity that processes reports the information to a health professional and/or patient. As explained such information can also be automatically processed to produce a treatment, testing, and/or monitoring plan, possibly along with counseling information. Thus, initiating an early stage operation can trigger an end to end sequence in which the health professional, patient or other concerned party is provided with a diagnosis, a plan, counseling and/or other information useful for acting on a physical condition. This is accomplished even though parts of the overall system are physically separated and possibly remote from the location of, e.g., the sample and sequence apparatus.

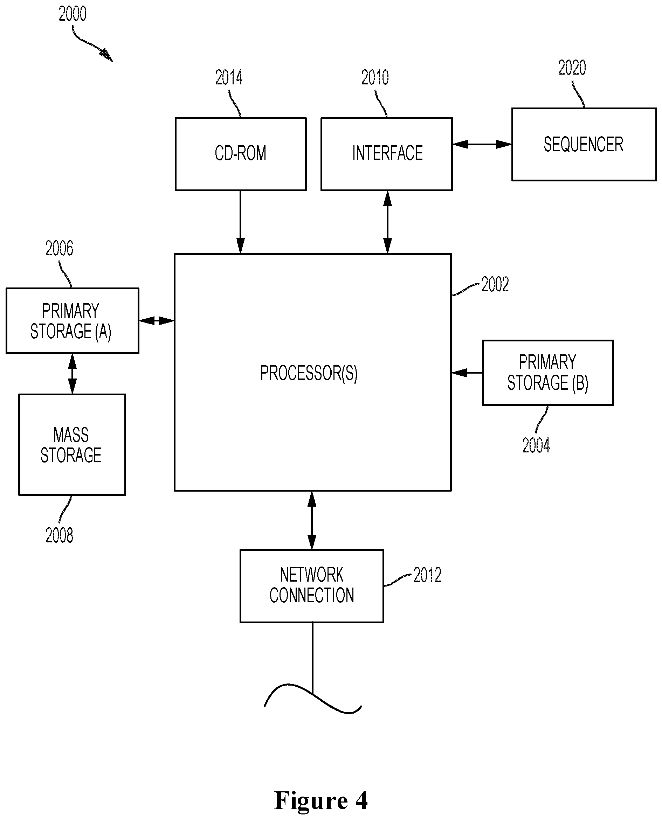

Figure 4 illustrates, in simple block format, a typical computer system that, when appropriately configured or designed, can serve as a computational apparatus according to certain embodiments. The computer system 2000 includes any number of processors 2002 (also referred to as central processing units, or CPUs) that are coupled to storage devices including primary storage 2006 (typically a random access memory, or RAM), primary storage 2004 (typically a read only memory, or ROM). CPU 2002 may be of various types including microcontrollers and microprocessors such as programmable devices (e.g., CPLDs and FPGAs) and non-programmable devices such as gate array ASICs or general-purpose microprocessors. In the depicted embodiment, primary storage 2004 acts to transfer data and instructions uni-directionally to the CPU and primary storage 2006 is used typically to transfer data and instructions in a bi-directional manner. Both of these primary storage devices may include any suitable computer-readable media such as those described above. A mass storage device 2008 is also coupled bi-directionally to primary storage 2006 and provides additional data storage capacity and may include any of the computer-readable media described above. Mass storage device 2008 may be used to store programs, data and the like and is typically a secondary storage medium such as a hard disk. Frequently, such programs, data and the like are temporarily copied to primary memory 2006 for execution on CPU 2002. It will be appreciated that the information retained within the mass storage device 2008, may, in appropriate cases, be incorporated in standard fashion as part of primary storage 2004. A specific mass storage device such as a CD-ROM 2014 may also pass data uni-directionally to the CPU or primary storage.

CPU 2002 is also coupled to an interface 2010 that connects to one or more input/output devices such as such as a nucleic acid sequencer (2020), video monitors, track balls, mice, keyboards, microphones, touch-sensitive displays, transducer card readers, magnetic or paper tape readers, tablets, styluses, voice or handwriting recognition peripherals, USB ports, or other well-known input devices such as, of course, other computers. Finally, CPU 2002 optionally may be coupled to an external device such as a database or a computer or telecommunications network using an external connection as shown generally at 2012. With such a connection, it is contemplated that the CPU might receive information from the network, or might output information to the network in the course of performing the method steps described herein.

In some implementations, a nucleic acid sequencer (2020) may be communicatively linked to the CPU 2002 via the network connection 2012 instead of or in addition to via the interface 2010.

In one embodiment, a system such as computer system 2000 is used as a data import, data correlation, and querying system capable of performing some or all of the tasks described herein. Information and programs, including data files can be provided via a network connection 2012 for access or downloading by a researcher. Alternatively, such information, programs and files can be provided to the researcher on a storage device.

In a specific embodiment, the computer system 2000 is directly coupled to a data acquisition system such as a microarray, high-throughput screening system, or a nucleic acid sequencer (2020) that captures data from samples. Data from such systems are provided via interface 2010 for analysis by system 2000. Alternatively, the data processed by system 2000 are provided from a data storage source such as a database or other repository of relevant data. Once in apparatus 2000, a memory device such as primary storage 2006 or mass storage 2008 buffers or stores, at least temporarily, relevant data. The memory may also store various routines and/or programs for importing, analyzing and presenting the data, including sequence reads, UMIs, codes for determining sequence reads, collapsing sequence reads and correcting errors in reads, etc.

In certain embodiments, the computers used herein may include a user terminal, which may be any type of computer (e.g., desktop, laptop, tablet, etc.), media computing platforms (e.g., cable, satellite set top boxes, digital video recorders, etc.), handheld computing devices (e.g., PDAs, e-mail clients, etc.), cell phones or any other type of computing or communication platforms.

In certain embodiments, the computers used herein may also include a server system in communication with a user terminal, which server system may include a server device or decentralized server devices, and may include mainframe computers, mini computers, super computers, personal computers, or combinations thereof. A plurality of server systems may also be used without departing from the scope of the present invention. User terminals and a server system may communicate with each other through a network. The network may comprise, e.g., wired networks such as LANs (local area networks), WANs (wide area networks), MANs (metropolitan area networks), ISDNs (Intergrated Service Digital Networks), etc. as well as wireless networks such as wireless LANs, CDMA, Bluetooth, and satellite communication networks, etc. without limiting the scope of the present invention.

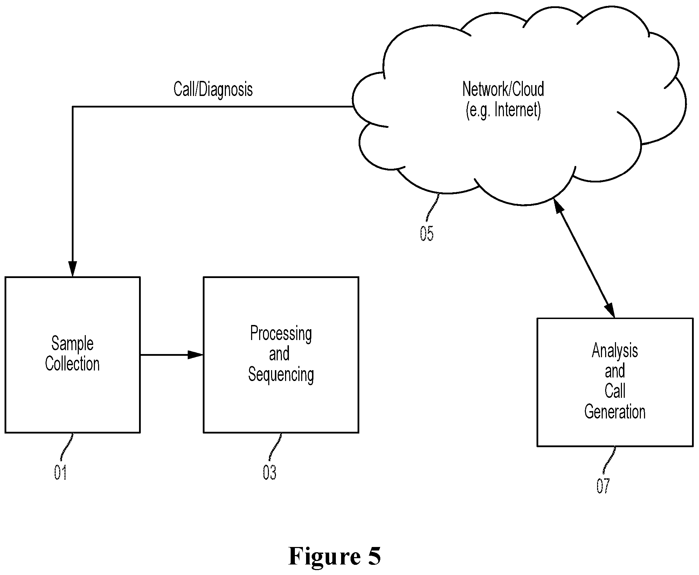

Figure 5 shows one implementation of a dispersed system for producing a call or diagnosis from a test sample. A sample collection location 01 is used for obtaining a test sample from a patient such as a pregnant female or a putative cancer patient. The samples then provided to a processing and sequencing location 03 where the test sample may be processed and sequenced as described above. Location 03 includes apparatus for processing the sample as well as apparatus for sequencing the processed sample. The result of the sequencing, as described elsewhere herein, is a collection of reads which are typically provided in an electronic format and provided to a network such as the Internet, which is indicated by reference number 05 in Figure 5.

The sequence data is provided to a remote location 07 where analysis and call generation are performed. This location may include one or more powerful computational devices such as computers or processors. After the computational resources at location 07 have completed their analysis and generated a call from the sequence information received, the call is relayed back to the network 05. In some implementations, not only is a call generated at location 07 but an associated diagnosis is also generated. The call and or diagnosis are then transmitted across the network and back to the sample collection location 01 as illustrated in Figure 5. As explained, this is simply one of many variations on how the various operations associated with generating a call or diagnosis may be divided among various locations. One common variant involves providing sample collection and processing and sequencing in a single location. Another variation involves providing processing and sequencing at the same location as analysis and call generation.

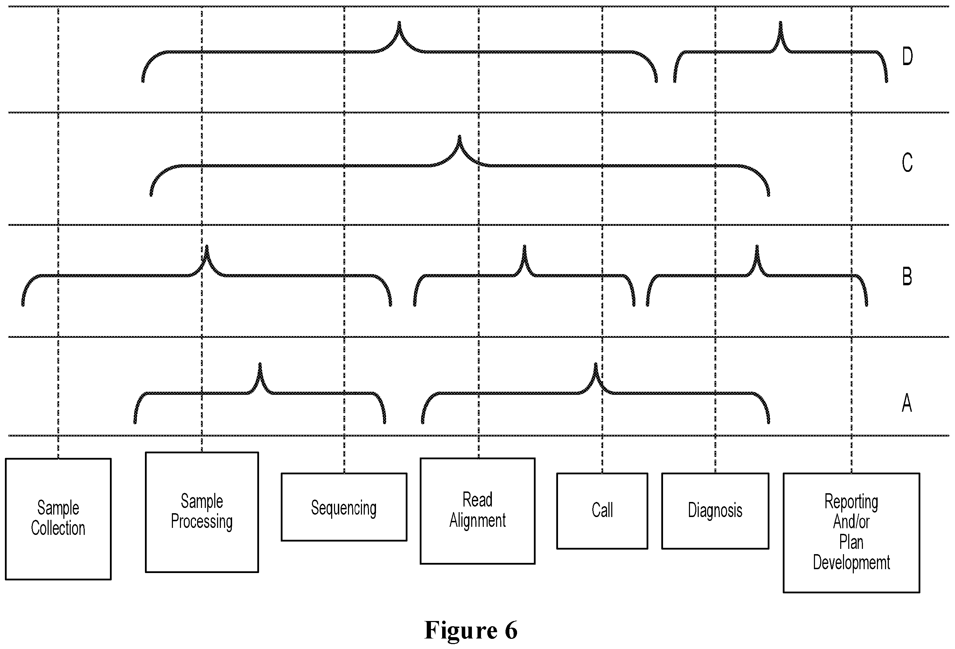

Figure 6 elaborates on the options for performing various operations at distinct locations. In the most granular sense depicted in Figure 6, each of the following operations is performed at a separate location: sample collection, sample processing, sequencing, read alignment, calling, diagnosis, and reporting and/or plan development.

In one embodiment that aggregates some of these operations, sample processing and sequencing are performed in one location and read alignment, calling, and diagnosis are performed at a separate location. See the portion of Figure 6 identified by reference character A. In another implementation, which is identified by character B in Figure 6, sample collection, sample processing, and sequencing are all performed at the same location. In this implementation, read alignment and calling are performed in a second location. Finally, diagnosis and reporting and/or plan development are performed in a third location. In the implementation depicted by character C in Figure 6, sample collection is performed at a first location, sample processing, sequencing, read alignment, calling,, and diagnosis are all performed together at a second location, and reporting and/or plan development are performed at a third location. Finally, in the implementation labeled D in Figure 6, sample collection is performed at a first location, sample processing, sequencing, read alignment, and calling are all performed at a second location, and diagnosis and reporting and/or plan management are performed at a third location.

One embodiment provides a system for analyzing cell-free DNA (cfDNA) for simple nucleotide variants associated with tumors, the system including a sequencer for receiving a nucleic acid sample and providing nucleic acid sequence information from the nucleic acid sample; a processor; and a machine readable storage medium comprising instructions for execution on said processor, the instructions comprising: code for mapping the nucleic acid sequence reads to one or more polymorphism loci on a reference sequence; code for determining, using the mapped nucleic acid sequence reads, allele counts of nucleic acid sequence reads for one or more alleles at the one or more polymorphism loci; and code for quantifying, using a probabilistic mixture model, one or more fractions of nucleic acid of the one or more contributors in the nucleic acid sample, wherein using the probabilistic mixture model comprises applying a probabilistic mixture model to the allele counts of nucleic acid sequence reads, and the probabilistic mixture model uses probability distributions to model the allele counts of nucleic acid sequence reads at the one or more polymorphism loci, the probability distributions accounting for errors in the nucleic acid sequence reads.

In some embodiments of any of the systems provided herein, the sequencer is configured to perform next generation sequencing (NGS). In some embodiments, the sequencer is configured to perform massively parallel sequencing using sequencing-by-synthesis with reversible dye terminators. In other embodiments, the sequencer is configured to perform sequencing-by-ligation. In yet other embodiments, the sequencer is configured to perform single molecule sequencing.

## EXPERIMENTAL

### Example 1

This example uses data obtained from actual DNA mixture samples to illustrate that some implementations can provide higher accuracy and reliability, as well as lower empirical bias, in quantifying DNA mixture samples, than conventional technologies that do not use the probabilistic approaches disclosed herein.

The DNA mixture samples included two DNA from genomes (contributors), and the minor fractions are 0.1%, 0.2%, 0.4%, and 2% in different samples. Some samples included 3 ng of input DNA, and others included 10 ng. The samples were processed in two experimental procedures labeled as Nack or Nack2 to indicate two primer designs, where the numbers of target loci are different for the two designs. Some samples were processed using the MiSeq sequencing platform and some using the MiniSeq platform.

The sample data were analyzed using three different methods. Table 8 shows the average of coefficient of variance (CV, defined as standard_deviation_of_predictions / true_fraction) values over multiple mixture fractions and the average of coefficient of variation + bias (CVB, commonly denoted as CV(RMSD) and defined as RMSD/true_fraction) values over multiple mixture fractions for the three different methods using various samples and experimental procedures. The first method applies a probabilistic model including a binomial distribution for modeling sequencing errors. The first method corresponds to some implementations descried as the Seq Model above. The data for the first method (Seq) are shown in the third row of Table 8. The second method applies a probabilistic mixture model including probability distributions accounting for DNA extraction errors, PCR amplification errors, and sequencing errors. The second method corresponds to some implementations descried as the Extraction-PCR-Seq Model above. The data for the second method (EPS) are shown in the fourth row of Table 8.

The third method corresponds to the baseline method NaiveLM or also called KGT.NaiveLM as described above. It determines DNA fractions of contributors using a basic linear regression formulation. The data for the third method (NaiveLM) are shown in the fifth row of Table 8.

It is worth noting that the genotype information of the contributors was not used to quantify the contributor fractions in the Seq or EPS method, but it was used in the NaiveLM method. Despite the fact that the Seq method and the EPS method did not need to use the genotype information of the contributors, they produced more reliable results as indicated by the smaller coefficient of variation values than the NaiveLM method. Moreover, the Seq method and the EPS method had lower bias as indicated by the smaller CVB values than the NaiveLM method. The best results among the three methods are bolded in Table 8. In short, the two methods using probabilistic mixture models produced more reliable, accurate, and less biased results than the linear regression method.

**Table 8. CV and CVB performance metrics for two of disclosed methods (Seq and EPS) compared to baseline method (NaiveLM) on four different datasets.**

| Method | Genotype | Nack (3ng, 10ng) | | Nack2 (3ng, 10ng) | | Nack2 MiniSeq (3ng, 10ng) | | Nack2 Validation (3ng, 10ng) | |
|---|---|---|---|---|---|---|---|---|---|
| | | CV | CVB | CV | CVB | CV | CVB | CV | CVB |
| Seq | Not used | 0.151 | 0.796 | 0.214 | 0.688 | 0.175 | 0.414 | 0.21 | 0.488 |
| EPS | Not used | **0.139** | **0.207** | **0.126** | **0.193** | **0.125** | **0.216** | **0.165** | **0.253** |

(continued)

| Method | Genotype | Nack (3ng, 10ng) | | Nack2 (3ng, 10ng) | | Nack2 MiniSeq (3ng, 10ng) | | Nack2 Validation (3ng, 10ng) | |
|---|---|---|---|---|---|---|---|---|---|
| | | CV | CVB | CV | CVB | CV | CVB | CV | CVB |
| NaiveLM | Used | 0.771 | 1.117 | 0.889 | 1.388 | 3.818 | 1.03 | 8.83 | 1.407 |

### Example 2

There are multiple free parameters, such as the average length of DNA template, average length of amplicon, human genome molecular weight, that are used together with the input DNA amount to estimate the effective input DNA amount and read counts. Justified adjustment of these parameters may ensure less biases and robust prediction performance. This example investigates how the average length of DNA template affects the performance of the various methods described above for quantifying DNA mixtures.

This example uses mock cfDNA (mcfDNA) to mimic real cfDNA. In order to obtain a proper correcting factor for real cfDNA, we need to 1) generate the similar standard mixtures using real cfDNA extracted from two individuals; 2) perform gDNA spike in experiments over real cfDNA mixtures.

#### *Source Genomes*

mcfDNA: mcfDNA from one of the tested cell lines, for which Nack4 target sites do not have CNV for the cell line.

cfDNA: cfDNA from a healthy person but not maternal cfDNA

gDNA: gDNA from one of the tested cell line or a normal cell line

#### *Mixture Composition Design*

Mixture 1: 75% cfDNA or mcfDNA, 25% gDNA

Mixture 2: 50% cfDNA or mcfDNA, 50% gDNA

Mixture 3: 25% cfDNA or mcfDNA, 75% gDNA

Mixture 4: 10% cfDNA or mcfDNA, 90% gDNA

Each with 3 replicates.

#### *Mixing Strategy*

1. cfDNA and gDNA templates are quantified;

2. cfDNA and gDNA templates are mixed at 3:1, 1:1, 1:3, 1:9 ratios;

3. PCR over the mixed templates.

The resulting mixtures and their compositions are shown in Table 9.

**Table 9. Mock and real DNA mixtures and estimated mixture fractions reflecting the impact of cfDNA/mcfDNA length on their relative effectiveness as PCR templates. There are three replicates for each type of mixture.**

| Mixing Fraction | mcfDNA + gDNA | cfDNA + gDNA |
|---|---|---|
| Mixture 1 (75%) | 82.9%, 84.0% , 84.9% | 68.6%, 68.8%, 69.1% |
| Mixture 3 (25%) | 14.2%, 14.5%, 14.4% | 20.5%, 20.2%, 20.0% |
| Mixture 4 (10%) | 5.37%, 5.51%, 5.40% | 8.94%, 8.86%, 9.09% |

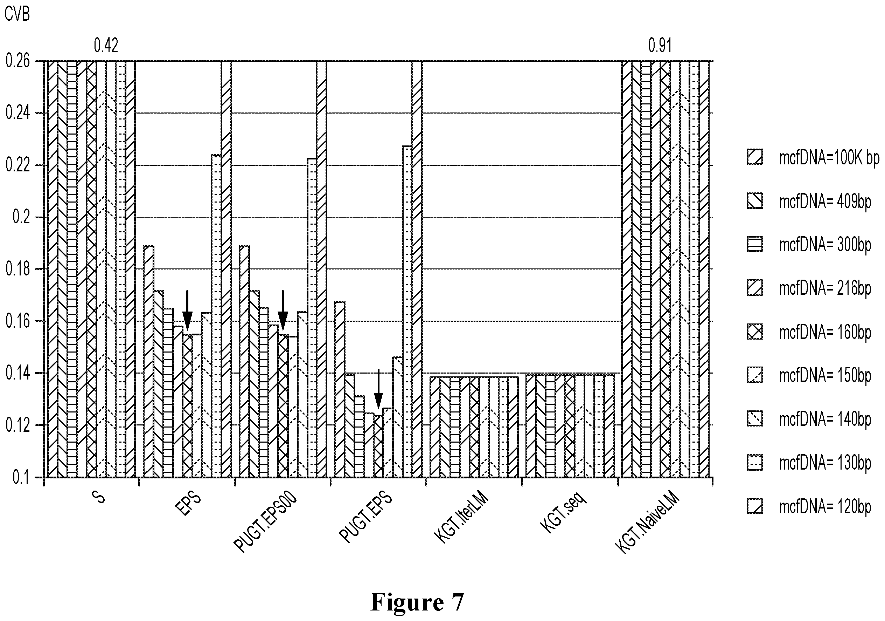

Figure 7 shows the CVB performance of various methods each under different choices of cfDNA length parameter. The following lengths: 120bp, 130bp, 140bp, 150bp, 160bp, 216bp, 300bp, 409bp, and 100k bp are evaluated. Different shades of bars indicate different mcfDNA lengths.

The different methods are labeled as follows.

S: probabilistic model accounting for errors due to sequencing. Not using baseline genomes as input. (without knowing D and R genome)

EPS: probabilistic model accounting for errors due to DNA extraction, PCR, and sequencing. Not using baseline genomes as input.

PUGT.EPS00: generic implementation of EPS model allowing both known, unknown, and partially known baselines. Not using baseline genomes as input.

PUGT.EPS: generic implementation of EPS model allowing both known, unknown, and partially known baselines. Using baseline genomes as input.

KGT.IterLM: Iterative Linear Model. Using baseline genomes as input.

KGT.Seq: probabilistic model accounting for errors due to sequencing. Using baseline genomes as input.

KGT.NaiveLM: Baseline method, the naive linear model with known genotype. Using baseline genomes as input.

At default DNA length parameter of 160bp, the EPS models have the best performance (indicated by arrows), both when the baseline genomes are available and not available.

Moreover, the quantification performances of the EPS methods remain outstanding even when practitioners perturb the DNA length parameter from 160bp to 120bp or 216bp. This indicates robustness of the methods to the cfDNA length parameter. The range is comfortably wider than the parameters used in the implementations described above: 160bp for mcfDNA, and 165bp for cfDNA.

The performance ranking among different methods is:

PUGT.EPS (using baseline genomes) > KGT.seq or KGT.IterLM (using baseline genomes) > PUGT.EPS or EPS (not using baseline genomes) > S (not using baseline genomes) > KGT.Naive (using baseline genomes).

Notably, the three EPS methods have markedly lower CVB than the naive linear model with known genotype, indicating that the EPS methods have improved accuracy and reduced bias over conventional linear model methods. Note that conventional methods are only applicable to mixture samples with known baselines genomes.

Furthermore, under default DNA length parameters, methods described in this disclosure have lower limit of bland (LOB) and higher analytical sensitivity than the method using conventional linear model. As shown in Table 10, the limit of blank (LOB) is below 0.1% for four methods disclosed, but the conventional, naive linear model method's LOB is at 0.42%.

**Table 10. LOB of Different Methods**

| Method | S | PUGT.EPS00 | PUGT.EPS | KGT.seq | KGT.NaiveLM |
|---|---|---|---|---|---|
| LOB | 0.05% | 0.08% | 0.06% | 0.03% | 0.42% |

## Example 3

This example uses data obtained from mock cfDNA (mcfDNA) and actual genomic DNA (gDNA) to investigate the sensitivities of some of the disclosed methods, and compare them to a known method KIMERDx that uses a qPCR technique.

Table 11 shows the LOQ of two probabilistic models labeled as follows.

EPS: probabilistic model accounting for errors due to DNA extraction, PCR, and sequencing. Not using baseline genomes as input.

PUGT.EPS: generic implementation of EPS model allowing both known, unknown, and partially known baselines. Using baseline genomes as input.

LOQ, or limit of quantification, is a measure of quantification sensitivity. It is defined as the minimal donor fraction that can be determined at no greater than 20% coefficient of variation (CV).

Under mcfDNA conditions (top two rows of data in Table 11), which mimic cfDNA samples from solid organ transplant patients, DNA mixture samples of two contributors were generated. Each sample included 3 ng of DNA. The probabilistic methods PUGT.EPS (using baseline genotypes) and EPS (without using baseline genotypes from pre-transplant recipient and donor) were applied to 5 samples x 3 replicates. Both probabilistic methods achieved LOQ of <=0.2% when using only 3 ng of input DNA, indicating high sensitivity for both disclosed methods.

Under a gDNA condition (third row of data in Table 11), which mimics blood gDNA samples from bone marrow transplant patients, DNA mixture samples of two contributors were generated. Each sample includes 10 ng of DNA. The PUGT.EPS method was used to analyze 5 samples x 3 replicates. The PUGT.EPS method achieved an LOQ of <=0.1% when using 10 ng of input DNA, which, as expected, is lower than the LOQ in the mcfDNA conditions using 3ng of input DNA.

Under another gDNA condition (four row of data in Table 11), DNA mixture samples of five contributors were generated. Each sample includes 10 ng total amount of DNA. The PUGT.EPS method was used to analyze 5 samples x 3 replicates. The PUGT.EPS method achieved an LOQ of <=0.35%. Even for such a difficult condition with five contributors, the method achieved a great LOQ significantly lower than 1%.

**Table 11. Sensitivity of Disclosed Methods**

| Sample Type | LOQ | Sample Size | Method |
|---|---|---|---|
| mcfDNA 3ng, 2 contributors | 0.2% | 5 samples x 3 | PUGT.EPS |
| mcfDNA 3ng, 2 contributors | 0.2% | 5 samples x 3 | EPS (no baseline) |
| gDNA 10ng, 2 contributors | 0.1% | 5 samples x 3 | PUGT.EPS |
| gDNA 10ng, 5 contributors | 0.35% | 4 samples x 3 | PUGT.EPS |

Table 12 shows the sensitivity (LOQ) values of a KIMERDx method that uses a qPCR technique on mixture samples of only two contributors. The KIMERDx method was used to analyze different quantity of input gDNA. To achieve 0.1% of LOQ, it requires 66 ng of input gDNA. In comparison, the PUGT.EPS method only requires <=10ng of input DNA to achieve the same level of sensitivity. With 10ng input gDNA, KIMERDx would achieve an LOQ of 0.7% compared to <0.1% for PUGT.EPS.

**Table 12. Sensitivity of qPCR KIMERDx Method**

| LOQ | # Cells | Input DNA (ng) |
|---|---|---|
| 0.05% | 20000 | 132 |
| 0.1% | 10000 | 66 |
| 1% | 1000 | 7 |
| 2% | 500 | 3 |
| 5% | 200 | 1 |

Therefore, this example illustrates that the disclosed probabilistic methods required significantly less input DNA to achieve a same level of sensitivity compared to the state of art method. Conversely, the disclose methods achieves a significantly higher sensitivity at low input DNA amount. Due to their improved sensitivity, the methods may allow for faster sample processing, require less reagent and improve accuracy of DNA mixture quantification.

Existing chimerism assays do *not* work for solid organ transplant monitoring, which our methods are designed for. The disclosed methods improve the sensitivity of DNA mixture quantification, which is particularly beneficial in applications where the input DNA quantity is limited, which covers all solid organ transplant cases. Solid organ transplant monitoring using cfDNA is challenging because the amount of cfDNA extracted from a typical blood sample is typically < 10ng, much lower than the amount of extractable gDNA. Meanwhile, cfDNA is much less effective as PCR template compared to gDNA of the same amount.

Existing methods also do not work for transplant with more than one donor, for which the methods we disclosed

51

still achieved high sensitivity. Transplants with more than one donor occur frequently for bone marrow transplants, and are also commonly seen in organ transplant with blood transfusion and in patients with prior organ transplants .

## Example 4

Conventional methods of chimerism analysis utilize capillary electrophoresis (CE) fragment analysis or quantitative polymerase chain reaction (qPCR) analysis of short tandem repeats (STRs) or small insertions and deletions (Indels). There are a number of drawbacks associated with these methods including limit of quantification, dynamic range, number of targets, workflow, analysis, and reproducibility. An alternative approach to these conventional methods utilizes next-generation sequencing (NGS) targeting hundreds of SNPs to quantitatively assess chimerism with low limit of quantification, broad dynamic range, simple workflow, automated analysis, and robust reproducibility.

### *Conventional Chimerism Analysis Using CE*

#### *Targets: STRs*

STRs are loci found throughout the genome. They are comprised of short sequences, usually between 2 and 8 nucleotides and most commonly 4, that are repeated tandemly (e.g. *gata* tandemly repeated as *gatagatagatagatagata*). The number of repeats varies between 4 and 40 repeats making a typical STR less than 400 total nucleotides in length. The number of repeats is highly variable within the human population. These two characteristics of STRs, relatively short total length and high variability, have made them attractive targets for human identification in forensic science. The short length is important for poor quality forensic samples because amplification of larger regions is difficult with these types of samples. The high variability in the population is an attractive feature because a relatively small number are needed for positive identification. While more than 100 STRs have been well characterized in the human genome, most applications use less than 30.

#### *Assay Design*

PCR primers are designed in the conserved flanking regions surrounding the STR. Primers can be multiplexed with each of the four fluorophores containing 4 to 7 STRs of varying lengths. This means that the multiplexes support between 10 and 21 unique STRs. The CE system measures the relative fluorescence units and the elapse time to detection to generate an electropherogram for each STR. Most labs utilize the full multiplexes for generating pre-transplant baseline genotypes for the recipient and the donor. The pre-transplant genotypes are compared to one another to select informative markers, markers in which the recipient and donor have unique alleles. The chimerism samples may be run with the entire multiplex or with individual singleplex assays for the informative STRs. Singleplex assays generally provide the highest level of sensitivity, but many labs prefer to run the multiplex assay.

#### *Workflow*

- DNA is extracted from peripheral blood, bone marrow, or cell lineages isolated with magnetic beads or by flow cytometry.

- PCR amplification of the target STRs is performed including fluorescent tagging.

- Separation and detection of the STR-PCR amplicons is performed with electrophoresis, most frequently a CE instrument. The CE system measures the relative fluorescence units and the elapse time to detection to generate electropherograms for each allele present in the sample.

- The person performing the analysis reviews the electropherograms for each informative marker to determine the relative frequency of the donor to the recipient. In cases with multiple informative markers, the average frequency is usually taken as the final measure of chimerism after taking into account variable performance of the different markers.

From extracted DNA to data analysis takes about seven hours with about 2 hours of that hands-on time. The analysis of the data is highly variable and takes from 15 minutes to two hours to analyze a single chimerism sample depending on the number of informative markers, the variability between the markers, and the complexity of the stutter peak subtraction.

### Limitations

There are three primary limitations to CE analysis of STR regions for chimerism analysis.

First, the electropherogram peaks alone are often difficult to analyze and percent chimerism from multiple peaks within the same sample frequently vary 10-15%. As a consequence of this variability, analysis can often take hours for a single sample and the results are still semiquantitative.

Second, limit of quantification (LOQ), often referred to as limit of detection (LOD) or sensitivity, ranges from 1-5% with this methodology. This broad range exists because each STR will have its own LOQ depending on the PCR enzyme stutter or "slippage" on the STR and variable performance of the fluorophores.

Third, although more than 100 STR targets are well characterized in the genome, including more than 21 STRs in an assay has not been reliable. This is because multiplexing that many specific primer pools into a single assay is very difficult to make robust and reliable. Therefore, chimerism mixtures from closely related individuals may have difficulty identifying informative markers and cases with many donors may be very difficult to analyze.

These limitations can be significant in clinical use. For example, an actual chimerism result of 99% will be reported as 100%.

### ***Conventional Chimerism Analysis Using qPCR***

### Targets: Indels

An indel is an insertion or deletion of 1 to 10,000 nucleotide bases. Millions of indels have been discovered in the human genome making it the second largest contributor to human genome variability after SNPs. Similar to STRs, many indels are short and can be easily amplified even from highly degraded DNA and small amounts of DNA. In addition, there is a wide variety of indels available in different lengths, different allele frequencies, and they are broadly distributed throughout the genome. These features of indels make them attractive targets for human identification and chimerism analysis.

### Assay Design

PCR primers are designed to amplify the indel and are designed as singleplex, small multiplexes (~3 targets), or large multiplexes (30-40 targets). It has been shown that 30-40 appropriately selected indels are needed to distinguish individuals from one another. With the commercially available kits, pre-transplant donor and recipient baseline samples are run through 30 to 40 indel targets in either 3-indel multiplexes or individual indels laid out on a 96-well plate. This step identifies informative targets in which the donor and recipient have different alleles. A minimum of two informative targets are then selected for each donor-recipient pair to be used for chimerism analysis.

Each indel is targeted by a set of fluorescently labeled primers that hybridize the DNA of interest. As the amplicon undergoes PCR cycling, the increasing fluorescence is proportional to the quantity of amplicon present. The quantification is determined by the number of PCR cycles required to reach the threshold cycle (Ct) value. The informative markers are usually selected to amplify the genome of the minor contributor, usually the recipient in the case of stem cell transplantation. The quantity is then determined by comparing the Ct values of the post-transplant sample, the matched pre-transplant baseline, and the reference control sample.

### Workflow

- DNA is extracted from peripheral blood, bone marrow, or cell lineages isolated with magnetic beads or by flow cytometry.
- Purified DNA is quantified and diluted as needed to achieve target concentrations.
- Baseline genotyping is performed by testing both the donor and recipient pre-transplant samples for every target indel in the system. In the small multiplex system this includes 10 individual reactions of 2-3 indel targets per reaction. In the singleplex system, this requires 46 individual reactions with a single indel target in each reaction. Each baseline sample run must also include a positive control and a no template control. This means that the small multiplex system can fit 8 baseline samples on a 96-well plate and the singleplex system can fit 2 per plate.
- 10ng of baseline DNA is added to each reaction well (100ng total for small multiplex and 460ng for singleplex)
- PCR Master Mix is prepared and added to each reaction well.

- Amplification primers are added to the appropriate wells (8x10 for the small multiplex and 2x46 for the singleplex)
- Plates are sealed, vortexed, centrifuged, and loaded onto the qPCR instrument.
- Results are loaded into the application-specific software.
- Recipient and donor baselines are compared in the software and informative markers are selected for chimerism analysis. Usually two informative targets are selected for each transplant recipient/donor pair.
- For each target to be amplified, the pre-transplant baseline sample from the minor contributor must be run in triplicate, each post-transplant chimerism sample must be run in triplicate, a positive control for every two reaction wells, and a no template control for each target. In other words, to perform a single post-transplant chimerism analysis 60ng (6 wells) of reference DNA must be run, 60ng (6 wells) of pre-transplant baseline DNA must be run, and 60ng (6 wells) of post-transplant chimerism DNA must be run. This is a total of 21 wells to generate data from 2 targets.
- PCR Master Mix is prepared and added to each reaction well.
- Amplification primers are added to the appropriate wells (7 wells per sample - 3 pre-transplant, 3 post-transplant, and 1 no template control)
- Plates are sealed, vortexed, centrifuged, and loaded onto the qPCR instrument.
- Results are loaded into the application-specific software.

From extracted DNA to genotyping data for informative marker selection takes about 3 total hour with one and half hours hands-on. After selection of informative markers and DNA extraction from chimerism samples, an addition 3 hours and one and a half hours of hands-on time is needed for generation of the chimerism data.

*Limitations*

There are three primary limitations of qPCR-based chimerism analysis of indel targets.

First, each chimerism analysis requires 60ng of pre-transplant recipient baseline sample. This is in addition to the 100-500ng of baseline DNA required for the initial genotyping. For programs frequently performing chimerism analysis, the pre-transplant baseline samples may be depleted, limiting the ability to run this assay for long periods of times.

Second, the requirement to run the chimerism analyses as singleplex reactions complicates the overall system requiring dozens of unique assays to be held in inventory. In addition, the cost of each reaction usually limits the analysis to only two targets per donor-recipient pair and these targets are likely to be different for each donor-recipient pair, making the setup susceptible to error.

Third, while the LOQ for qPCR is very low, the dynamic range of qPCR-based chimerism suffers and chimerism predictions when the minor contributor is greater than 30% are not reliable.

### Novel Chimerism Analysis by NGS

*Targets: SNPs*

SNPs are single nucleotide positions in which variation is present to a measurable degree within the human population or within specific populations. *dbSNP* is a database of SNPs managed by the National Center for Biotechnology Information (NCBI) and it currently contains more than 170 million human SNPs with nearly 25 million of them validated. This means that SNPs are responsible for the vast majority of variability within the human population averaging one SNP per 1,000 nucleotide bases. SNPs can be biallelic (two observed alleles), triallelic (three observed alleles), or tetra-allelic (four observed alleles). A single base variant can be considered a SNP when the minor allele has a frequency of at least 1% in a random set of individuals in a population. SNPs are excellent targets for chimerism analysis because of their low mutation rate, small amplicon size, and compatibility with high-throughput sequencing technology.

*Assay Design*

SNPs are selected to be biallelic with roughly 50/50 allele frequency within various populations around the world. In addition, SNPs having low mutation rates and no linkage disequilibrium with the SNP pool are selected. Finally, SNPs were assessed for design-ability, both in terms of minimizing primer-primer interaction and uniformity in PCR amplification and in sequencing coverage. The total number of SNPs is determined based on power to discriminate between first-degree relatives from all populations around the world.

A single PCR step amplifies the DNA, isolates the amplicons of interest, and incorporates flowcell adapters (inverse oligonucleotide sequences to those on the Illumina flowcells allowing the sample amplicons to bind to the flowcell), sequencing primers (oligonucleotide sequences that serve as initiation sites for the Illumina sequencing by synthesis (SBS) process), and index barcode sequences (oligonucleotide sequences that allow multiple samples to be run simultaneously).

The NGS system sequences each amplicon hundreds to thousands of times. In pre-transplant baseline samples, this information is used to genotype each contributor. In post-transplant chimerism samples, the reads counts for each nucleotide at a SNP location can be used with or without the baseline genotypes to accurately estimate the percent chimerism of each contributor, up to five total contributors.

*Workflow*

- DNA is extracted from peripheral blood, bone marrow, or cell lineages isolated with magnetic beads or by flow cytometry.
- Purified DNA is quantified and diluted as needed to achieve target concentrations.
- Unique index barcodes are added to each sample DNA.
- Master mix is added to every sample, mixed, sealed, and centrifuged.
- PCR amplification is performed.
- All samples are pooled into a single well and then a PCR clean-up is performed.
- The cleaned pool is quantified, diluted, and denatured.
- The final pool, also called a library, is loaded onto the sequencer and sequencing is initiated.
- Sequencing data is imported into the chimerism-specific analysis software for automated quality control and chimerism analysis.

From extracted DNA to loading of the sequencer takes less than 3 hours with less than 2 hours of hands-on time. The sequencing run requires 9 to 13 hours depending on the number of samples being run simultaneously. Once sequencing data are collected, the analysis of the data does not require manual intervention, allowing automation of the analysis and reducing human errors.

*Limitations*

There is one primary limitation of NGS-based chimerism analysis using SNPs: compared to CE and qPCR-based chimerism analysis, NGS-based sample processing and sequencing take longer, although the hands-on time is equivalent. The NGS-based library preparation can be completed in the afternoon with the sequencing completed overnight. This allows 24-hour turnaround for samples received in the morning. However, because sequencing may be multiplexed, this method can combine multiple samples for sequencing, thereby improving the overall efficiency of sample processing.

*Summary*

NGS-based chimerism analysis using SNP targets is an efficient, accurate, and reliable method to overcome many of the limitations associated with conventional methods of chimerism analysis. The results are truly quantitative and can be automatically generated without the need for laborious human review of electropherograms and stutter subtractions. The NGS-based chimerism analysis has a broad dynamic range with low LOQ and no performance degradation at high levels of mixed chimerism. More than 200 SNP targets are used with the NGS system and they are multiplexed into a single reaction. This allows for utility with more than one donor and with donor-recipient pairs that are very closely related. The indexing capabilities and throughput of the NGS system allow for baseline and chimerism samples to be run simultaneously, only one assay and kit to be stored in inventory, and low potential for human error in the workflow.

**Example 5**

This example shows that some implementations improve over conventional methods because of the throughput of the NGS sequencer, the assay design with incredibly high uniformity, and the use of SNPs as targets. The disclosed methods can analyze far more targets than conventional methods, which are limited to <30 targets. The process allows multiplex many samples to boost efficiency. The methods are quantitative, and can all be done cost-effectively.

One experiment compares the performance of the methods in some implementations with baseline genomes known or unknown. Table 12 shows the DNA quantifications for four samples with different recipient portions for three baseline conditions (both baselines known, both baselines unknown, and recipient known but donor unknown). The results show that the methods can be performed with and without baselines with similar performance at different recipient portions. When baselines are known, the methods tend to produce results with smaller confidence intervals (and higher reliability).

**Table 12.** DNA Quantifications with Known and Unknown Baselines

| | | Both Baselines Known | | | Both Baselines Unknown | | | Recipient Known Donor Unknown | | |
|---|---|---|---|---|---|---|---|---|---|---|
| | | % recipient | Low 95% Ct | High 95% Ct | % recipient | Low 95% Ct | High 95% Ct | % recipient | Low 95% Ct | High 95% Ct |
| | Sample 1 | 0.7% | 0.6% | 0.8% | 0.7% | 0.5% | 0.9% | 0.7% | 0.6% | 0.8% |
| | Sample 2 | 5.8% | 5.4% | 6.2% | 5.7% | 5.2% | 6.2% | 5.8% | 5.4% | 6.2% |
| | Sample 3 | 12.3% | 12.0% | 12.6% | 12.3% | 11.8% | 12.7% | 12.3% | 12% | 12.6% |
| | Sample 4 | 38.6% | 38.1% | 39.0% | 38.7% | 38.0% | 39.3% | 38.6% | 38.1% | 39.0% |

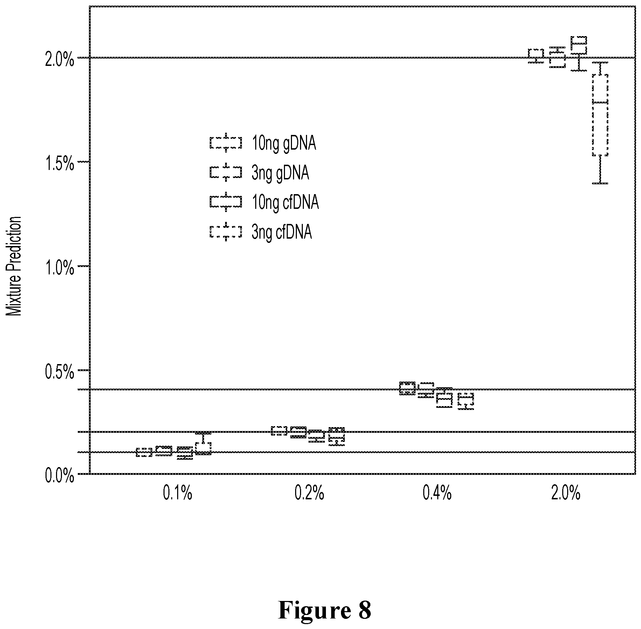

Figure 8 compares DNA portion determined by some implementations (Y axis) and actual DNA portions (X axis). The horizontal lines indicate the values of actual portions. The chimerism sample includes cfDNA mixtures that are mock cfDNA provided by Horizon Discovery (Catalog No. 12498714289). As the figure shows, the predicted minor contributor portion are quite close to the actual minor contributor portion at 0.1%, 0.2%, 0.4%, and 2%.

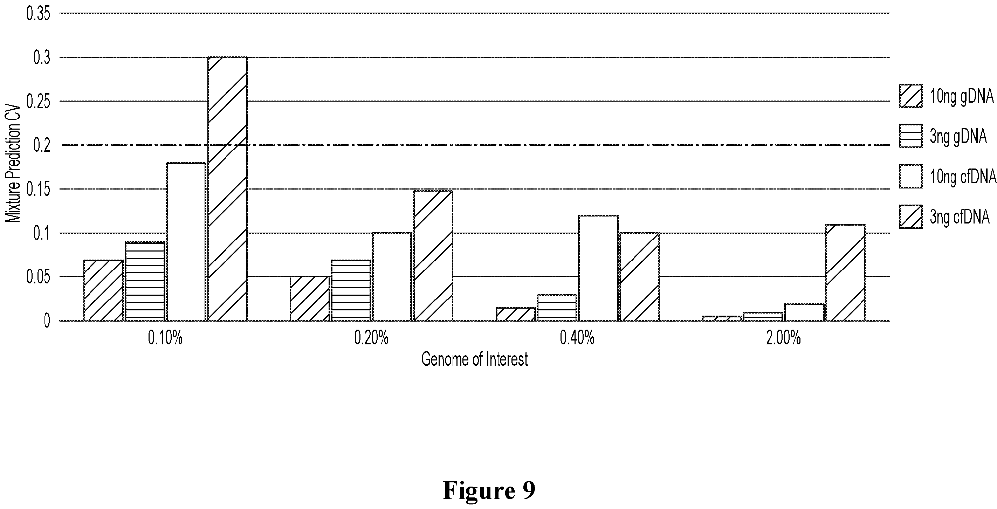

Figure 9 shows the coefficient of variance (CV) of 16 conditions for determining the limit of quantification (LOQ) for some implementations. LOQ is defined as the lowest concentration at which an analyte can be reliably detected at which the imprecision (CV) is less than 20%. This measurement takes into account both analytical sensitivity (i.e., limit of detection) and reproducibility (i.e., precision). The four different groups of bars represent different minor contributor fractions of 0.1%, 0.2%, 0.4%, and 2%. The four bars in a group represent, from left to right, four input DNA conditions: 10ng of gDNA, 3 ng of gDNA, 10ng of cf DNA, and 3 ng of cfDNA. At each minor contributor fraction, there is a consistent pattern as expected -samples of smaller amounts lead to higher CV, and cfDNA lead to higher CV.

All but one condition (0.1% minor contributor fraction, 3ng of cfDNA) can detect an analyte with an imprecision (CV) less than 20%. In other words, the one condition (3ng of cfDNA) has an LOQ of 0.2%, while the rest of the conditions have an LOQ of 0.1%.

Table 13 summarizes the data above. It clearly shows that all four input DNA conditions have LOQ values smaller than 0.2%, and all but the most challenging input condition (3 ng cfDNA) have LOQ of 0.1%.

**Table 13.** Limit of Quantification for some Implementations with Different DNA Input

| Input DNA | Limit of Quantification |
|---|---|
| 10 ng cfDNA | <0.1% |
| 3 ng cfDNA | <0.2% |
| 10 ng gDNA | <0.1% |
| 3 ng gDNA | <0.1% |

## ***Discussions***

Conventional chimerism methods using qPCR or CE technologies sacrifice ease of use, number of targets, sensitivity, or dynamic range.

The implemented methods multiplex samples in a single assay. This enables pre-transplant baseline samples and post-transplant chimerism samples to be run using the same assay and side by side on the same sequencing run. The methods can be performed with and without baselines with near identical performance.

qPCR and CE chimerism methods may provide some level of multiplexing for the pre-transplant baseline samples, but these methods have performance degradation for post-transplant chimerism quantification when targets are multiplexed. This means that baseline and chimerism samples must be run separately and the entire system may requires at least a dozen unique assays.

While qPCR is sensitive for microchimerism detection, it lacks the dynamic range to be reliable for mixed chimerism. CE-based chimerism analysis offers a broad dynamic range for mixed chimerism detection, but lacks the sensitivity for microchimerism. The disclosed methods provide both a reliable low limit of quantification (LOQ) and a broad dynamic range, enabling one solution that can cover all different types of chimerism.

**Claims**

1. A method, implemented at a computer system that includes one or more processors and system memory, of quantifying a nucleic acid sample comprising nucleic acid of two or more contributors, the method comprising:

    (a) extracting nucleic acid molecules from the nucleic acid sample;
    (b) amplifying the extracted nucleic acid molecules;
    (c) sequencing the amplified nucleic acid molecules using a nucleic acid sequencer to produce nucleic acid sequence reads;
    (d) mapping, by the one or more processors, the nucleic acid sequence reads to one or more polymorphism loci on a reference sequence;
    (e) determining, using the mapped nucleic acid sequence reads and by the one or more processors, allele counts of nucleic acid sequence reads for one or more alleles at the one or more polymorphism loci; and
    (f) quantifying, using a probabilistic mixture model and by the one or more processors, one or more fractions of nucleic acid of the two or more contributors in the nucleic acid sample, wherein using the probabilistic mixture model comprises applying a probabilistic mixture model to the allele counts of nucleic acid sequence reads, and wherein the probabilistic mixture model uses probability distributions to model the allele counts of nucleic acid sequence reads at the one or more polymorphism loci, the probability distributions accounting for errors in the nucleic acid sequence reads,

    wherein the two or more contributors comprise one or more donors of a transplant and a donee of the transplant, and wherein the nucleic acid sample comprises a sample obtained from the donee.

2. The method of claim 1, further comprising, determining, using the probabilistic mixture model and by the one or more processors, one or more genotypes of the two or more contributors at the one or more polymorphism loci, or further comprising, determining, using the one or more fractions of nucleic acid of the two or more contributors, a risk of one contributor (a donee) rejecting a tissue or an organ transplanted from another contributor (a donor), or wherein the transplant comprises an allogeneic or xenogeneic transplant.

3. The method of claim 1, wherein the nucleic acid molecules comprise DNA molecules or RNA molecules, or wherein the extracted nucleic acid molecules comprise cell-free nucleic acid or cellular DNA.

4. The method of claim 1, wherein the one or more polymorphism loci comprise one or more biallelic polymorphism loci, or wherein the one or more alleles at the one or more polymorphism loci comprise one or more single nucleotide polymorphism (SNP) alleles.

5. The method of claim 1, wherein the probabilistic mixture model uses a single-locus likelihood function to model allele counts at a single polymorphism locus, the single-locus likelihood function comprising

$$M(n_{1i}, n_{2i} \mid p_{1i}, \theta)$$

wherein

$n_{1i}$ is the allele count of allele 1 at locus i,
$n_{2i}$ is the allele count of allele 2 at locus i,
$p_{1i}$ is an expected fraction of allele 1 at locus i, and
$\theta$ comprises one or more model parameters,
optionally wherein $p_{1i}$ is modeled as a function of:

    (i) genotypes of the contributors at locus $i$, or $g_i = (g_{11i},..., g_{D1i})$, which is a vector of copy number of allele 1 at locus $i$ in contributors 1... D;
    (ii) read count errors resulting from the sequencing operation in (c), or $\lambda$; and
    (iii) fractions of nucleic acid of contributors in the nucleic acid sample, or $\beta = (\beta_1, ..., \beta_D)$, wherein D is the number of contributors,

    further optionally wherein

$$p_{1i} = p(g_i, \lambda, \boldsymbol{\beta}) \leftarrow [(1-\lambda)\, g_i + \lambda\, (2-g_i)] \,/\, 2 \bullet \boldsymbol{\beta},$$

where $\bullet$ is vector dot product operator, optionally wherein $p_{1i}$ is obtained using the $p_1'$ values in Table 3.

6. The method of claim 5, wherein zero, one or more genotypes of the contributors are unknown, optionally wherein (f) comprises marginalizing over a plurality of possible combinations of genotypes to enumerate the probability parameter $p_{1i}$, or further comprising determining a genotype configuration at each of the one or more polymorphism loci, the genotype configuration comprising two alleles for each of the one or more contributors.

7. The method of claim 5, wherein the single-locus likelihood function comprise a first binomial distribution.

8. The method of claim 7, wherein the first binomial distribution is expressed as follows:

$$n_{1i} \sim BN(n_i, p_{1i})$$

wherein

$n_{1i}$ is an allele count of nucleic acid sequence reads for allele 1 at locus $i$; and
$n_i$ is a total read count at locus $i$, which equals to a total genome copy numbers $n''$.

9. The method of claim 8, wherein (f) comprises maximizing a multiple-loci likelihood function calculated from a plurality of single-locus likelihood functions, optionally wherein (f) comprises:

calculating a plurality of multiple-loci likelihood values using a plurality of potential fraction values and a multiple-loci likelihood function of the allele counts of nucleic acid sequence reads determined in (e);
identifying one or more potential fraction values associated with a maximum multiple-loci likelihood value; and
quantifying the one or more fractions of nucleic acid of the one or more contributors in the nucleic acid sample as the identified potential fraction value.

10. The method of claim 9, wherein the multiple-loci likelihood function comprises:

$$L(\boldsymbol{\beta}, \theta, \lambda, \pi ; n_1, n_2) = \Pi_i \, [\Sigma g_i \, M(n_{1i}, n_{2i} \mid p(g_i, \lambda, \boldsymbol{\beta}), \theta) \bullet P(g_i \mid \pi)]$$

wherein

$L(\beta, \theta, \lambda, \pi ; n_1, n_2)$ is the likelihood of observing allele count vectors $n_1$ and $n_2$ for alleles 1 and 2;
$p(g_i, \lambda, \boldsymbol{\beta})$ is the expected fraction or probability of observing allele 1 at locus $i$ based on the contributors' genotypes $g_i$ at locus i;
$P(g_i|\pi)$ is the prior probability of observing the genotypes $g_i$ at locus i given a population allele frequency ($\pi$); and, $\Sigma g_i$ denotes summing over a plurality of possible combinations of genotypes of the contributors, optionally wherein the multiple-loci likelihood function comprises:

$$L(\boldsymbol{\beta}, \lambda, \pi ; n_1, n_2) = \Pi_i \, [\Sigma g_i \, BN(n_{1i} \mid n_i, \cdot p(g_i, \lambda, \boldsymbol{\beta})) \bullet P(g_i \mid \pi)],$$

preferably wherein the likelihood function comprises:

$$L(\boldsymbol{\beta}, \lambda, \pi ; n_1, n_2) = \Pi_i \, \Sigma_{g1ig2i} \, BN(n_{1i} \mid n_i, p_{1i}(g_{1i}, g_{2i}, \lambda, \boldsymbol{\beta})) \cdot P(g_{1i}, g_{2i} \mid \pi)$$

wherein

$L(\beta, \lambda, \pi ; n_1, n_2)$ is the likelihood of observing allele count vectors $n_1$ to $n_2$ for alleles 1 and 2 given parameters $\beta$ and $\pi$ ;
$p_{1i}(g_{1i}, g_{2i}, \lambda, \beta)$ is a probability parameter, taken as $p_1'$ from Table 3, indicating a probability of allele $1$ at locus $i$ based on the two contributors' genotypes ($g_{1i}, g_{2i}$); and $P(g_{1i}, g_{2i}|\pi)$ is a prior joint probability of observing the two contributors' genotypes given a population allele frequency ($\pi$), more preferably wherein the prior joint probability is calculated using marginal distributions $P(g_{1i}|\pi)$ and $P(g_{2i}|\pi)$ that satisfy the Hardy-Weinberg equilibrium, more preferably wherein the prior joint probability is calculated using genetic relationship between the two contributors.

11. The method of claim 10, wherein the probabilistic mixture model accounts for nucleic acid molecule copy number errors resulting from extracting the nucleic acid molecules performed in (a), as well as the read count errors resulting from the sequencing operation in (c), optionally wherein the probabilistic mixture model uses a second binomial distribution to model allele counts of the extracted nucleic acid molecules for alleles at the one or more polymorphism loci.

12. The method of claim 11, wherein the second binomial distribution is expressed as follows:

$$n_{1i}'' \sim BN(n_i'', p_{1i})$$

wherein

$n_{1i}''$ is an allele count of extracted nucleic acid molecules for allele *1* at locus *i*;
$n_i''$ is a total nucleic acid molecule count at locus *i*; and
$p_{iu}$ is a probability parameter indicating the probability of allele *1* at locus *i*, optionally
wherein the first binomial distribution is conditioned on an allele fraction $n_{1i}''/n_i''$, further optionally wherein the first binomial distribution is re-parameterized as follows:

$$n_{1i} \sim BN(n_i, n_{1i}''/n_i'')$$

wherein

$n_{1i}$ is an allele count of nucleic acid sequence reads for allele *1* at locus *i*;
$n_i''$ is a total number of nucleic acid molecules at locus *i*, which equals to a total genome copy numbers $n''$;
$n_i$ is a total read count at locus *i*; and
$n_{1i}''$ is a number of extracted nucleic acid molecules for allele *1* at locus *i*.

13. The method of claim 12, wherein the probabilistic mixture model uses a first beta distribution to approximate a distribution of $n_{1i}''/n''$, optionally wherein the first beta distribution has a mean and a variance that match a mean and a variance of the second binomial distribution, or wherein locus *i* is modeled as biallelic and the first beta distribution is expressed as follows:

$$n_{i1}''/n'' \sim Beta((n''-1)p_{1i}, (n''-1)p_{2i})$$

wherein

$p_{1i}$ is a probability parameter indicating the probability of a first allele at locus *i*; and
$p_{2i}$ is a probability parameter indicating the probability of a second allele at locus *i*.

14. The method of claim 13, wherein (f) comprises combining the first binomial distribution, modeling sequencing read counts, and the first beta distribution, modeling extracted nucleic acid molecule number, to obtain the single-locus likelihood function of $n_{1i}$ that follows a first beta-binomial distribution, preferably wherein the first beta-binomial distribution has the form:

$$n_{1i} \sim BB(n_i, (n''-1) \cdot p_{1i}, (n''-1) \cdot p_{2i}),$$

or an alternative approximation:

$$n_{1i} \sim BB(n_i, n'' \cdot p_{1i}, n'' \cdot p_{2i}),$$

more preferably wherein the multiple-loci likelihood function comprises:

$$L(\beta, n'', \lambda, \pi \; ; n_1, n_2) = \Pi i \; [\Sigma g_i \; BB(n_{1i} \mid n_i, (n''-1) \cdot p_{1i}, (n''-1) \cdot p_{2i}) \cdot P(g_i \mid \pi)]$$

wherein $L(\beta, n'', \lambda, \pi ; n_1, n_2)$ is the likelihood of observing allele count vectors $n_1$ and $n_2$ for alleles 1 and 2 at all loci, and $p_{1i} = \boldsymbol{p}(g_i, \lambda, \beta)$, $p_{2i} = 1 - p_{1i}$,
more preferably wherein the multiple-loci likelihood function comprises:

$$L(\beta, n'', \lambda, \pi \; ; n_1, n_2) = \Pi_i \Sigma_{g_{1i}g_{2i}} BB(n_{1i}, n_{2i} \mid n_i, (n''-1) \cdot p_{1i}(g_{1i}, g_{2i}, \lambda, \beta), (n''-1)p_{2i}(g_{1i}, g_{2i}, \lambda, \beta)) \cdot P(g_{1i}, g_{2i} \mid \pi)$$

wherein $L(\beta, n'', \lambda, \pi ; n_1, n_2)$ is the likelihood of observing an allele count vector for the first allele of all loci ($n_1$) and an allele count vector for the second allele of all loci ($n_2$) given parameters $\beta$, $n''$, $\lambda$, and $\pi$;
$p_{1i}(g_{1i}, g_{2i}, \lambda, \beta)$ is a probability parameter, taken as $p_1'$ from Table 3, indicating a probability of allele 1 at locus $i$ based on the two contributors' genotypes ($g_{1i}, g_{2i}$);
$p_{2i}(g_{1i}, g_{2i}, \lambda, \beta)$ is a probability parameter, taken as $p_2'$ from Table 3, indicating a probability of allele 2 at locus $i$ based on the two contributors' genotypes ($g_{1i}, g_{2i}$); and
$P(g_{1i}, g_{2i} \mid \pi)$ is a prior joint probability of observing the first contributor's genotype for the first allele ($g_{1i}$) and the second contributor's genotype for the first allele ($g_{2i}$) at locus $i$ given a population allele frequency ($\pi$).

15. The method of claim 12, wherein (f) comprises estimating the total extracted genome copy number $n''$ from a mass of the extracted nucleic acid molecules, optionally wherein the estimated total extracted genome copy number $n''$ is adjusted according to fragment size of the extracted nucleic acid molecules.

16. The method of claim 10, wherein the probabilistic mixture model accounts for nucleic acid molecule number errors resulting from amplifying the nucleic acid molecules performed in (b), as well as the read count errors resulting from the sequencing operation in (c), optionally wherein the amplification process of (b) is modeled as follows:

$$x_{t+1} = x_t + y_{t+1}$$

wherein

$x_{t+1}$ is the nucleic acid copies of a given allele after cycle $t+1$ of amplification;
$x_t$ is the nucleic acid copies of a given allele after cycle $t$ of amplification;
$y_{t+1}$ is the new copies generated at cycle $t+1$, and it follows a binomial distribution $y_{t+1} \sim BN(x_t, r_{t+1})$; and
$r_{t+1}$ is the amplification rate for cycle $t+1$.

17. The method of claim 16, wherein the probabilistic mixture model uses a second beta distribution to model allele fractions of the amplified nucleic acid molecules for alleles at the one or more polymorphism loci, optionally wherein locus $i$ is biallelic and the second beta distribution is expressed as follows:

$$n_{1i}'/(n_{1i}' + n_{2i}') \sim Beta(n'' \cdot \rho_i \cdot p_{1i}, n'' \cdot \rho_i \cdot p_{2i})$$

wherein

$n_{1i}'$ is an allele count of amplified nucleic acid molecules for a first allele at locus $i$;
$n_{2i}'$ is an allele count of amplified nucleic acid molecules for a second allele at locus $i$;
$n''$ is a total nucleic acid molecule count at any locus;
$\rho_i$ is a constant related to an average amplification rate r;
$p_{1i}$ is the probability of the first allele at locus $i$; and
$p_{2i}$ is the probability of the second allele at locus $i$, further optionally wherein $\rho_i$ is $(1+r)/(1-r) / [1-(1+r)^{-t}]$, and r is the average amplification rate per cycle, or wherein $\rho_i$ is approximated as $(1+r)/(1-r)$.

18. The method of claim 17, wherein (f) comprises combining the first binomial distribution and the second beta distribution to obtain the single-locus likelihood function for $n_{1i}$ that follows a second beta-binomial distribution, optionally wherein the second beta-binomial distribution has the form:

$$n_{1i} \sim BB(n_i, n'' \cdot \rho_i \cdot p_{1i}, n'' \cdot \rho_i \cdot p_{2i})$$

wherein

$n_{1i}$ is an allele count of nucleic acid sequence reads for the first allele at locus $i$;

$p_{1i}$ is a probability parameter indicating the probability of a first allele at locus $i$; and

$p_{2i}$ is a probability parameter indicating the probability of a second allele at locus $i$, further optionally wherein (f) comprises, by assuming the one or more polymorphism loci have a same amplification rate, re-parameterizing the second beta-binomial distribution as:

$$n_{1i} \sim BB(n_i,\ n'' \cdot (1+r)/(1-r) \cdot p_{1i},\ n'' \cdot (1+r)/(1-r) \cdot p_{2i})$$

wherein $r$ is an amplification rate, further optionally wherein the multiple-loci likelihood function comprises:

$$L(\beta, n'', r, \lambda, \pi\ ;\ n_1, n_2) = \Pi i\ [\Sigma g_i\ BB(n_{1i}\ |\ n_i,\ n'' \cdot (1+r)/(1-r) \cdot p_{1i},\ n'' \cdot (1+r)/(1-r) \cdot p_{2i}) \bullet P(g_i\ |\ \pi)],\ \text{or}$$

wherein the multiple-loci likelihood function comprises:

$$L(\beta, n'', r, \lambda, \pi\ ;\ n_1, n_2) =$$
$$\Pi i \Sigma_{g1ig2i}\ [BB(n_{1i}\ |\ n_i,\ n'' \cdot (1+r)/(1-r) \cdot p_{1i}(g_{1i}, g_{2i}, \lambda, \beta),\ n'' \cdot (1+r)/(1-r) \cdot p_{2i}(g_{1i}, g_{2i}, \lambda,$$
$$\beta)) \cdot P(g_{1i}, g_{2i}|\pi)]$$

wherein $L(\beta, n'', r, \lambda, \pi\ ;\ n_1, n_2)$ is the likelihood of observing an allele count vector for the first allele of all loci ($n_1$) and an allele count vector for the second allele of all loci ($n_2$) given parameters $\beta, n'', r, \lambda,$ and $\pi$.

19. The method of claim 18, wherein (f) comprises, by defining a relative amplification rate of each polymorphism locus to be proportional to a total reads of the locus, re-parameterizing the second beta-binomial distribution as:

$$n_{1i} \sim BB(n_i,\ c' \cdot n_i \cdot p_{1i},\ c' \cdot n_i \cdot p_{2i})$$

wherein

$c'$ is a parameter to be optimized; and

$n_i$ is the total reads at locus $i$, optionally wherein the multiple-loci likelihood function comprises:

$$L(\beta, n'', c', \lambda, \pi\ ;\ n_1, n_2) = \Pi i\ [\Sigma g_i\ BB(n_{1i}|\ n_i,\ c' \cdot n_i \cdot p_{1i},\ c' \cdot n_i \cdot p_{2i}) \bullet P(g_i\ |\ \pi)]$$

20. The method of claim 10, wherein the probabilistic mixture model accounts for nucleic acid molecule number errors resulting from extracting the nucleic acid molecules performed in (a) and amplifying the nucleic acid molecules performed in (b), as well as the read count errors resulting from the sequencing operation in (c), optionally wherein the probabilistic mixture model uses a third beta distribution to model allele fractions of the amplified nucleic acid molecules for alleles at the one or more polymorphism loci, accounting for the sampling errors resulting from extracting the nucleic acid molecules performed in (a) and amplifying the nucleic acid molecules performed in (b), further optionally wherein locus $i$ is biallelic and the third beta distribution has the form of:

$$n_{1i}'/(n_{1i}' + n_{2i}') \sim Beta(n'' \cdot (1+r_i)/2 \cdot p_{1i},\ n'' \cdot (1+r_i)/2 \cdot p_{2i})$$

wherein

$n_{1i}'$ is an allele count of amplified nucleic acid molecules for a first allele at locus $i$;

$n_{2i}'$ is an allele count of amplified nucleic acid molecules for a second allele at locus $i$;

$n''$ is a total nucleic acid molecule count;

$r_i$ is the average amplification rate for locus i;

$p_{1i}$ is the probability of the first allele at locus $i$; and

$p_{2i}$ is a probability of the second allele at locus $i$.

21. The method of claim 20, wherein (f) comprises combining the first binomial distribution and the third beta distribution to obtain the single-locus likelihood function of $n_{1i}$ that follows a third beta-binomial distribution, optionally wherein the third beta-binomial distribution has the form:

$$n_{1i} \sim BB(n_i, n'' \cdot (1+ r_i)/2 \cdot p_{1i}, n'' \cdot (1+ r_i)/2 \cdot p_{2i})$$

wherein $r_i$ is an amplification rate, further optionally wherein the multiple-loci likelihood function comprises:

$$L(\beta, n'', r, \lambda, \pi ; n_1, n_2) =$$

$$\Pi i \, [\Sigma g_i \, BB(n_{1i} \mid n_i, n'' \cdot (1+ r )/2 \cdot p_{1i}, n'' \cdot (1+ r )/2 \cdot p_{2i}) \cdot \boldsymbol{P}(g_i \mid \pi)],$$

wherein r is an amplification rate assumed to be equal for all loci.

22. The method of claim 21, wherein the multiple-loci likelihood function comprises:

$$L(\beta, n'', r, \lambda, \pi ; n_1, n_2) = \Pi_i \Sigma_{g_1 i g_2 i} BB(n_{1i} \mid n_i, n'' \cdot (1+ r )/2 \cdot p_{1i}(g_{1i}, g_{2i}, \lambda, \beta), n'' \cdot (1+ r )/2$$
$$\cdot p_{2i}(g_{1i}, g_{2i}, \lambda, \beta)) \cdot P(g_{1i}, g_{2i} \mid \pi)$$

wherein $L(n_1, n_2 \mid \beta, n'', r, \lambda, \pi)$ is the likelihood of observing allele counts for the first allele vector $n_1$ and an allele count for the second allele vector $n_2$ given parameters $\beta, n'', r, \lambda$, and $\pi$.

23. The method of claim 1, further comprising: (g) estimating one or more confidence intervals of the one or more fractions of nucleic acid of the two or more contributors using the hessian matrix of the log-likelihood using numerical differentiation.

24. The method of claim 1, wherein the mapping of (d) comprises using computer hashing and computer dynamic programming, optionally wherein the mapping of (d) comprises identifying, by the one or more processors using computer hashing and computer dynamic programing, reads among the nucleic acid sequence reads matching any sequence of a plurality of unbiased target sequences, wherein the plurality of unbiased target sequences comprises subsequences of the reference sequence and sequences that differ from the subsequences by a single nucleotide, optionally wherein the plurality of unbiased target sequences comprises five categories of sequences encompassing each polymorphic site of a plurality of polymorphic sites:

   (i) a reference target sequence that is a sub-sequence of the reference sequence, the reference target sequence having a reference allele with a reference nucleotide at the polymorphic site;
   (ii) alternative target sequences each having an alternative allele with an alternative nucleotide at the polymorphic site, the alternative nucleotide being different from the reference nucleotide;
   (iii) mutated reference target sequences comprising all possible sequences that each differ from the reference target sequence by only one nucleotide at a site that is not the polymorphic site;
   (iv) mutated alternative target sequences comprising all possible sequences that each differ from an alternative target sequence by only one nucleotide at a site that is not the polymorphic site; and
   (v) unexpected allele target sequences each having an unexpected allele different from the reference allele and the alternative allele, and each having a sequence different from the previous four categories of sequences, optionally further comprising estimating a sequencing error rate $\lambda$ at the variant site base on a frequency of observing the unexpected allele target sequences of (v),
   optionally wherein (e) comprises using the identified reads and their matching unbiased target sequences to determine allele counts of the nucleic acid sequence reads for the alleles at the one or more polymorphism loci, or wherein the plurality of unbiased target sequences comprises sequences that are truncated to have the same length as the nucleic acid sequence reads, or wherein the plurality of unbiased target sequences comprises sequences stored in one or more hash tables, and the reads are identified using the hash tables.

25. The method of claim 1, wherein the quantifying employs (i) a computer optimization method combining multi-iteration grid searching and a BFGS - quasi-Newton method, or an iterative weighted linear regression, and (ii) a numerical differentiation method.

26. A system for quantifying a nucleic acid sample comprising nucleic acid of two or more contributors, the system comprising:

   (a) a sequencer configured to (i) receive nucleic acid molecules extracted from the nucleic acid sample, (ii) amplify the extracted nucleic acid molecules, and (iii) sequence the amplified nucleic acid molecules under conditions that produce nucleic acid sequence reads; and
   (b) a computer comprising one or more processors configured to:

   map the nucleic acid sequence reads to one or more polymorphism loci on a reference sequence;
   determine, using the mapped nucleic acid sequence reads, allele counts of nucleic acid sequence reads for one or more alleles at the one or more polymorphism loci; and
   quantify, using a probabilistic mixture model, one or more fractions of nucleic acid of the two or more contributors in the nucleic acid sample,

   wherein
   using the probabilistic mixture model comprises applying a probabilistic mixture model to the allele counts of nucleic acid sequence reads, and
   the probabilistic mixture model uses probability distributions to model the allele counts of nucleic acid sequence reads at the one or more polymorphism loci, the probability distributions accounting for errors in the nucleic acid sequence reads, and
   the two or more contributors comprise one or more donors of a transplant and a donee of the transplant, and wherein the nucleic acid sample comprises a sample obtained from the donee.

27. The system of claim 26, further comprising a tool for extracting nucleic acid molecules from the nucleic acid sample, or wherein the probability distributions comprise a first binomial distribution as follows:

$$n_{1i} \sim BN(n_i,\, p_{1i})$$

wherein

$n_{1i}$ is an allele count of nucleic acid sequence reads for allele 1 at locus $i$;
$n_i$ is a total read count at locus $i$, which equals to a total genome copy numbers $n''$; and
$p_{1i}$ is a probability parameter indicating the probability of allele 1 at locus $i$.

28. A computer program product comprising a non-transitory machine readable medium storing program code that, when executed by two or more processors of a computer system, causes the computer system to implement a method of quantifying a nucleic acid sample comprising nucleic acid of two or more contributors, said program code comprising:

   code for mapping the nucleic acid sequence reads to one or more polymorphism loci on a reference sequence;
   code for determining, using the mapped nucleic acid sequence reads, allele counts of nucleic acid sequence reads for one or more alleles at the one or more polymorphism loci; and
   code for quantifying, using a probabilistic mixture model, one or more fractions of nucleic acid of the two or more contributors in the nucleic acid sample,

   wherein
   using the probabilistic mixture model comprises applying a probabilistic mixture model to the allele counts of nucleic acid sequence reads, and
   the probabilistic mixture model uses probability distributions to model the allele counts of nucleic acid sequence reads at the one or more polymorphism loci, the probability distributions accounting for errors in the nucleic acid sequence reads, and
   the two or more contributors comprise one or more donors of a transplant and a donee of the transplant, and wherein the nucleic acid sample comprises a sample obtained from the donee.

**Patentansprüche**

1. Verfahren, implementiert in einem Computersystem, das einen oder mehrere Prozessoren und Systemspeicher einschließt, zum Quantifizieren einer Nukleinsäureprobe, die Nukleinsäure von zwei oder mehr Beitragenden umfasst, wobei das Verfahren umfasst:

   (a) Extrahieren von Nukleinsäuremolekülen aus der Nukleinsäureprobe;
   (b) Amplifizieren der extrahierten Nukleinsäuremoleküle;
   (c) Sequenzieren der amplifizierten Nukleinsäuremoleküle unter Verwendung eines Nukleinsäuresequenzierers, um Nukleinsäuresequenz-Reads zu erzeugen;
   (d) Abbilden der Nukleinsäuresequenz-Reads durch den einen oder die mehreren Prozessoren auf einen oder mehrere Polymorphismus-Loci auf einer Referenzsequenz;
   (e) Bestimmen von Allelzahlen von Nukleinsäuresequenz-Reads für ein oder mehrere Allele an dem einen oder den mehreren Polymorphismus-Loci unter Verwendung der abgebildeten Nukleinsäuresequenz-Reads und durch den einen oder die mehreren Prozessoren; und
   (f) Quantifizieren einer oder mehrerer Fraktionen von Nukleinsäure der zwei oder mehr Beitragenden in der Nukleinsäureprobe unter Verwendung eines probabilistischen Mischungsmodells und durch den einen oder die mehreren Prozessoren, wobei das Verwenden des probabilistischen Mischungsmodells das Anwenden eines probabilistischen Mischungsmodells auf die Allelzahlen von Nukleinsäuresequenz-Reads umfasst und wobei das probabilistische Mischungsmodell Wahrscheinlichkeitsverteilungen verwendet, um die Allelzahlen von Nukleinsäuresequenz-Reads an dem einen oder den mehreren Polymorphismus-Loci zu modellieren, wobei die Wahrscheinlichkeitsverteilungen Fehler in den Nukleinsäuresequenz-Reads berücksichtigen,

   wobei die zwei oder mehr Beitragenden einen oder mehrere Spender eines Transplantats und einen Empfänger des Transplantats umfassen und wobei die Nukleinsäureprobe eine Probe umfasst, die von dem Empfänger erlangt wurde.

2. Verfahren nach Anspruch 1, ferner umfassend: Bestimmen von einem oder mehreren Genotypen der zwei oder mehr Beitragenden an dem einen oder den mehreren Polymorphismus-Loci unter Verwendung des probabilistischen Mischungsmodells und durch den einen oder die mehreren Prozessoren, oder ferner umfassend: unter Verwendung der einen oder den mehreren Fraktionen von Nukleinsäure der zwei oder mehr Beitragenden, Bestimmen eines Risikos, dass ein Beitragender (ein Empfänger) ein Gewebe oder ein Organ abstößt, das von einem anderen Beitragenden (einem Spender) transplantiert wurde, oder wobei das Transplantat ein allogenes oder xenogenes Transplantat umfasst.

3. Verfahren nach Anspruch 1, wobei die Nukleinsäuremoleküle DNA-Moleküle oder RNA-Moleküle umfassen oder wobei die extrahierten Nukleinsäuremoleküle zellfreie Nukleinsäure oder zelluläre DNA umfassen.

4. Verfahren nach Anspruch 1, wobei der eine oder die mehreren Polymorphismus-Loci einen oder mehrere biallele Polymorphismus-Loci umfassen oder wobei das eine oder die mehreren Allele an dem einen oder den mehreren Polymorphismus-Loci ein oder mehrere Einzelnukleotidpolymorphismus-(SNP-)Allele umfassen.

5. Verfahren nach Anspruch 1, wobei das probabilistische Mischungsmodell eine Einzellocus-Likelihood-Funktion verwendet, um Allelzahlen an einem einzelnen Polymorphismus-Locus zu modellieren, wobei die Einzellocus-Likelihood-Funktion umfasst:

$$M(n_{1i},\ n_{2i}|p_{1i},\ \theta)$$

wobei:

   $n_{1i}$ die Allelzahl von Allel 1 am Locus $i$ ist,
   $n_{2i}$ die Allelzahl von Allel 2 am Locus $i$ ist,
   $p_{1i}$ eine erwartete Fraktion von Allel 1 am Locus $i$ ist, und
   $\theta$ einen oder mehrere Modellparameter umfasst,
   optional wobei $p_{1i}$ als eine Funktion von Folgendem modelliert wird:

      (i) Genotypen der Beitragenden am Locus $i$ oder $g_i = (g_{11i},..., g_{D1i})$, das ein Vektor der Kopienanzahl von Allel 1 am Locus *i* in den Beitragenden 1 ... *D* ist,

(ii) Read-Zählungsfehler, die aus der Sequenzierungsoperation in (c) resultieren, oder $\lambda$; und

(iii) Nukleinsäurefraktionen von Beitragenden in der Nukleinsäureprobe oder $\beta = (\beta_1, ..., \beta_D)$, wobei *D* die Anzahl der Beitragenden ist,

ferner optional wobei:

$$p_{1i} = p(g_i, \lambda, \beta) \leftarrow [(1 - \lambda)\, g_i + \lambda\, (2 - g_i)] / 2 \cdot \beta,$$

worin • der Skalarprodukt-Operator ist, optional wobei $p_{1i}$ unter Verwendung der $p_1'$-Werte in Tabelle 3 erlangt wird.

6. Verfahren nach Anspruch 5, wobei null, ein oder mehrere Genotypen der Beitragenden unbekannt sind, optional wobei (f) umfasst: Marginalisieren über eine Vielzahl von möglichen Kombinationen von Genotypen, um den Wahrscheinlichkeitsparameter $p_{1i}$ durchzuzählen, oder ferner umfassend: Bestimmen einer Genotypkonfiguration an jedem des einen oder der mehreren Polymorphismus-Loci, wobei die Genotypkonfiguration zwei Allele für jeden des einen oder der mehreren Beitragenden umfasst.

7. Verfahren nach Anspruch 5, wobei die Einzellocus-Likelihood-Funktion eine erste Binomialverteilung umfasst.

8. Verfahren nach Anspruch 7, wobei die erste Binomialverteilung wie folgt ausgedrückt wird:

$$n_{1i} \sim BN(n_i, p_{1i})$$

wobei:

$n_{1i}$ eine Allelzahl von Nukleinsäuresequenz-Reads für Allel 1 am Locus *i* ist; und

$n_i$ eine Gesamtzahl der Reads am Locus *i* ist, die gleich einer Gesamtanzahl der Genomkopien $n''$ ist.

9. Verfahren nach Anspruch 8, wobei (f) umfasst: Maximieren einer Mehrfachloci-Likelihood-Funktion, die aus einer Vielzahl von Einzellocus-Likelihood-Funktionen berechnet wird, optional wobei (f) umfasst:

Berechnen einer Vielzahl von Mehrfachloci-Likelihood-Werten unter Verwendung einer Vielzahl von potenziellen Fraktionswerten und einer Mehrfachloci-Likelihood-Funktion der Allelzahlen von Nukleinsäuresequenz-Reads, die in (e) bestimmt wurden;

Identifizieren von einem oder mehreren potenziellen Fraktionswerten, die mit einem maximalen Mehrfachloci-Likelihood-Wert assoziiert sind; und

Quantifizieren der einen oder mehreren Nukleinsäurefraktionen des einen oder der mehreren Beitragenden in der Nukleinsäureprobe als den identifizierten potenziellen Fraktionswert.

10. Verfahren nach Anspruch 9, wobei die Mehrfachloci-Likelihood-Funktion umfasst:

$$L(\beta, \theta, \lambda, \pi; n_1, n_2) = \Pi_i\, [\Sigma g_i\, M(n_{1i}, n_{2i} | p(g_i, \lambda, \beta), \theta) \cdot P(g_i | \pi)]$$

wobei:

$L(\beta, \theta, \lambda, \pi; n_1, n_2)$ die Likelihood dafür ist, die Allelzahlvektoren $n_1$ und $n_2$ für die Allele 1 und 2 zu beobachten;

$p(g_i, \lambda, \beta)$ die erwartete Fraktion oder Wahrscheinlichkeit dafür ist, Allel 1 am Locus *i* zu beobachten, und zwar auf der Grundlage der Genotypen $g_i$ der Beitragenden am Locus *i*;

$P(g_i | \pi)$ die vorherige Wahrscheinlichkeit dafür ist, angesichts einer Populationsallelfrequenz ($\pi$) die Genotypen $g_i$ am Locus *i* zu beobachten; und

$\Sigma g_i$ die Summierung über eine Vielzahl von möglichen Kombinationen von Genotypen der Beitragenden bezeichnet, wahlweise wobei die Mehrfachloci-Likelihood-Funktion umfasst:

$$L(\beta, \lambda, \pi; n_1, n_2) = \Pi_i\, [\Sigma g_i\, BN(n_{1i} | n_i, p(g_i, \lambda, \beta)) \cdot P(g_i | \pi)],$$

vorzugsweise wobei die Likelihood-Funktion umfasst:

$$L(\beta, \lambda, \pi; n_1, n_2) = \Pi_i \Sigma_{g1ig2i} BN(n_{1i}|n_i, p_{1i}(g_{1i}, g_{2i}, \lambda, \beta)) \cdot P(g_{1i}, g_{2i}|\pi)$$

wobei:

$L(\beta, \lambda, \pi; n_1, n_2)$ die Likelihood dafür ist, die Allelzahlvektoren $n_1$ bis $n_2$ für die Allele 1 und 2 angesichts der Parameter $\beta$ und $\pi$ zu beobachten;

$p_{1i}(g_{1i}, g_{2i}, \lambda, \beta)$ ein Wahrscheinlichkeitsparameter ist, der als $p_1'$ aus Tabelle 3 entnommen wird und eine Wahrscheinlichkeit des Allels 1 am Locus $i$ auf der Grundlage der Genotypen der beiden Beitragenden ($g_{1i}$, $g_{2i}$) angibt; und

$P(g_{1i}, g_{2i}|\pi)$ eine vorherige gemeinsame Wahrscheinlichkeit dafür ist, angesichts einer Populationsallelfrequenz ($\pi$) die Genotypen der beiden Beitragenden zu beobachten, wobei die vorherige gemeinsame Wahrscheinlichkeit besonders bevorzugt unter Verwendung von Randverteilungen $P(g_{1i}|\pi)$ und $P(g_{2i}|\pi)$ berechnet wird, die das Hardy-Weinberg-Gleichgewicht erfüllen, wobei die vorherige gemeinsame Wahrscheinlichkeit besonders bevorzugt unter Verwendung der genetischen Beziehung zwischen den beiden Beitragenden berechnet wird.

11. Verfahren nach Anspruch 10, wobei das probabilistische Mischungsmodell Fehler in der Kopienanzahl von Nukleinsäuremolekülen, die aus dem in (a) durchgeführten Extrahieren der Nukleinsäuremoleküle resultieren, sowie die aus der Sequenzierungsoperation in (c) resultierenden Read-Zählungsfehler berücksichtigt, optional wobei das probabilistische Mischungsmodell eine zweite Binomialverteilung verwendet, um Allelzahlen der extrahierten Nukleinsäuremoleküle für Allele an dem einen oder den mehreren Polymorphismus-Loci zu modellieren.

12. Verfahren nach Anspruch 11, wobei die zweite Binomialverteilung wie folgt ausgedrückt wird:

$$n_{1i}'' \sim BN(n_i'', p_{1i})$$

wobei:

$n_{1i}''$ eine Allelzahl von extrahierten Nukleinsäuremolekülen für Allel 1 am Locus $i$ ist;
$n_i''$ eine Gesamtanzahl von Nukleinsäuremolekülen am Locus $i$ ist; und
$p_{iu}$ ein Wahrscheinlichkeitsparameter ist, der die Wahrscheinlichkeit des Allels 1 am Locus $i$ angibt, optional: wobei die erste Binomialverteilung von einer Allelfraktion $n_{1i}''/n_i''$ abhängig ist, wobei ferner optional die erste Binomialverteilung wie folgt neu parametrisiert wird:

$$n_{1i} \sim BN(n_i, n_{1i}''/n_i'')$$

wobei:

$n_{1i}$ eine Allelzahl von Nukleinsäuresequenz-Reads für Allel 1 am Locus $i$ ist;
$n_i''$ eine Gesamtanzahl von Nukleinsäuremolekülen am Locus $i$ ist, die gleich einer Gesamt-Genomkopienanzahl n'' ist;
$n_i$ eine Gesamt-Readzahl am Locus $i$ ist; und
$n_{1i}''$ eine Anzahl der extrahierten Nukleinsäuremoleküle für Allel 1 am Locus $i$ ist.

13. Verfahren nach Anspruch 12, wobei das probabilistische Mischungsmodell eine erste Beta-Verteilung verwendet, um eine Verteilung von $n_{1i}''/n''$ anzunähern, optional wobei die erste Beta-Verteilung einen Mittelwert und eine Varianz aufweist, die mit einem Mittelwert und einer Varianz der zweiten Binomialverteilung übereinstimmen, oder wobei der Locus $i$ als biallel modelliert wird und die erste Beta-Verteilung wie folgt ausgedrückt wird:

$$n_{i1}''/n'' \sim Beta((n''-1)p_{1i}, (n''-1)p_{2i})$$

wobei:

$p_{1i}$ ein Wahrscheinlichkeitsparameter ist, der die Wahrscheinlichkeit eines ersten Allels am Locus $i$ angibt; und

$p_{2i}$ ein Wahrscheinlichkeitsparameter ist, der die Wahrscheinlichkeit eines zweiten Allels am Locus $i$ angibt.

14. Verfahren nach Anspruch 13, wobei (f) umfasst: Kombinieren der ersten Binomialverteilung, Modellieren von den Sequenzierungs-Read-Zahlen und der ersten Betaverteilung, Modellieren der Anzahl der extrahierten Nukleinsäuremoleküle, um die Einzellocus-Likelihood-Funktion von $n_{1i}$ zu erlangen, die einer ersten Beta-Binomialverteilung folgt, vorzugsweise wobei die erste Beta-Binomialverteilung die folgende Form aufweist:

$$n_{1i} \sim BB(n_i, (n''-1) \cdot p_{1i}, (n''-1) \cdot p_{2i}),$$

oder eine alternative Näherung:

$$n_{1i} \sim BB(n_i, n'' \cdot p_{1i}, n'' \cdot p_{2i}),$$

wobei besonders bevorzugt die Mehrfachloci-Likelihood-Funktion umfasst:

$$L(\beta, n'', \lambda, \pi; n_1, n_2) = \Pi_i \left[ \Sigma g_i BB(n_{1i}|n_i, (n''-1) \cdot p_{1i}, (n''-1) \cdot p_{2i}) \bullet P(g_i|\pi) \right]$$

wobei $L(\beta, n'', \lambda, \pi; n_1, n_2)$ die Likelihood dafür ist, die Allelzahlvektoren $n_1$ und $n_2$ für die Allele 1 und 2 an allen Loci zu beobachten, und $p_{1i} = p(g_i, \lambda, \beta)$, $p_{2i} = 1 - p_{1i}$,
wobei besonders bevorzugt die Mehrfachloci-Likelihood-Funktion umfasst:

$$L(\beta, n'', \lambda, \pi; n_1, n_2) =$$

$$\Pi_i \Sigma_{g_{1i}g_{2i}} BB(n_{1i}, n_{2i}|n_i, (n''-1) \cdot p_{1i}(g_{1i}, g_{2i}, \lambda, \beta), (n''-1) \cdot p_{2i}(g_{1i}, g_{2i}, \lambda, \beta)) \cdot P(g_{1i}, g_{2i}|\pi)$$

wobei $L(\beta, n'', \lambda, \pi; n_1, n_2)$ die Likelihood dafür ist, einen Allelzahlvektor für das erste Allel aller Loci ($n_1$) und einen Allelzahlvektor für das zweite Allel aller Loci ($n_2$) angesichts den Parametern $\beta$, $n''$, $\lambda$ und $\pi$ zu beobachten;
$p_{1i}(g_{1i}, g_{2i}, \lambda, \beta)$ ein Wahrscheinlichkeitsparameter ist, der als $p_1'$ aus Tabelle 3 entnommen wird und eine Wahrscheinlichkeit für Allel 1 am Locus $i$ auf der Grundlage der Genotypen der beiden Beitragenden ($g_{1i}, g_{2i}$) angibt;
$p_{2i}(g_{1i}, g_{2i}, \lambda, \beta)$ ein Wahrscheinlichkeitsparameter ist, der als $p_2'$ aus Tabelle 3 entnommen wird und eine Wahrscheinlichkeit für Allel 2 am Locus $i$ auf der Grundlage der Genotypen der beiden Beitragenden ($g_{1i}, g_{2i}$) angibt; und
$P(g_{1i}, g_{2i}|\pi)$ eine vorherige gemeinsame Wahrscheinlichkeit dafür ist, den Genotyp des ersten Beitragenden für das erste Allel ($g_{1i}$) und den Genotyp des zweiten Beitragenden für das erste Allel ($g_{2i}$) am Locus $i$ angesichts einer Populationsallelfrequenz ($\pi$) zu beobachten.

15. Verfahren nach Anspruch 12, wobei (f) umfasst: Schätzen der Gesamtanzahl extrahierter Genomkopien $n''$ aus einer Masse der extrahierten Nukleinsäuremoleküle, optional wobei die geschätzte Gesamtanzahl extrahierter Genomkopien $n''$ gemäß der Fragmentgröße der extrahierten Nukleinsäuremoleküle angepasst wird.

16. Verfahren nach Anspruch 10, wobei das probabilistische Mischungsmodell Fehler in der Anzahl der Nukleinsäuremoleküle, die aus dem in (b) durchgeführten Amplifizieren der Nukleinsäuremoleküle resultieren, sowie die aus der Sequenzierungsoperation in (c) resultierenden Read-Zählungsfehler berücksichtigt, optional wobei der Amplifikationsprozess von (b) wie folgt modelliert wird:

$$x_{t+1} = x_t + y_{t+1}$$

wobei:

$x_{t+1}$ die Nukleinsäurekopien eines gegebenen Allels nach dem Zyklus $t+1$ der Amplifikation ist;
$x_t$ die Nukleinsäurekopien eines gegebenen Allels nach dem Zyklus $t$ der Amplifikation ist;
$y_{t+1}$ die im Zyklus $t+1$ erzeugten neuen Kopien ist und einer Binomialverteilung $y_{t+1} \sim BN(x_t, r_{t+1})$ folgt; und
$r_{t+1}$ die Amplifikationsrate für den Zyklus $t+1$ ist.

17. Verfahren nach Anspruch 16, wobei das probabilistische Mischungsmodell eine zweite Beta-Verteilung verwendet, um Allelfraktionen der amplifizierten Nukleinsäuremoleküle für Allele an dem einen oder den mehreren Polymorphismus-Loci zu modellieren, optional wobei der Locus $i$ biallel ist und die zweite Beta-Verteilung wie folgt ausgedrückt wird:

$$n_{1i}'/(n_{1i}' + n_{2i}') \sim Beta(n''{\cdot}\rho_i{\cdot}p_{1i}, \; n''{\cdot}\rho_i{\cdot}p_{2i})$$

wobei:

$n_{1i}'$ eine Allelzahl von amplifizierten Nukleinsäuremolekülen für ein erstes Allel am Locus $i$ ist;
$n_{2i}'$ eine Allelzahl von amplifizierten Nukleinsäuremolekülen für ein zweites Allel am Locus $i$ ist;
$n''$ eine Gesamtanzahl von Nukleinsäuremolekülen an jeglichem Locus ist;
$\rho_i$ eine Konstante ist, die sich auf eine durchschnittliche Amplifikationsrate $r$ bezieht;
$p_{1i}$ die Wahrscheinlichkeit des ersten Allels am Locus $i$ ist; und
$p_{2i}$ die Wahrscheinlichkeit des zweiten Allels am Locus $i$ ist, ferner optional wobei $\rho_i$ $(1+r)/(1-r)$ / $[1-(1+r)^{-t}]$ ist und $r$ die durchschnittliche Amplifikationsrate pro Zyklus ist, oder wobei $\rho_i$ als $(1+r)/(1-r)$ angenähert wird.

18. Verfahren nach Anspruch 17, wobei (f) umfasst: Kombinieren der ersten Binomialverteilung und der zweiten Beta-Verteilung, um die Einzellocus-Likelihood-Funktion für $n_{1i}$ zu erlangen, die einer zweiten Beta-Binomialverteilung folgt, optional wobei die zweite Beta-Binomialverteilung die folgende Form aufweist:

$$n_{1i} \sim BB(n_i, \; n''{\cdot}\rho_i{\cdot}p_{1i}, \; n''{\cdot}\rho_i{\cdot}p_{2i})$$

wobei:

$n_{1i}$ eine Allelzahl der Nukleinsäuresequenz-Reads für das erste Allel am Locus $i$ ist;
$p_{1i}$ ein Wahrscheinlichkeitsparameter ist, der die Wahrscheinlichkeit eines ersten Allels am Locus $i$ angibt; und
$p_{2i}$ ein Wahrscheinlichkeitsparameter ist, der die Wahrscheinlichkeit eines zweiten Allels am Locus $i$ angibt, wobei ferner optional (f) umfasst: unter der Annahme, dass der eine oder die mehreren Polymorphismus-Loci eine gleiche Amplifikationsrate aufweisen, erfolgendes Neuparametrisieren der zweiten Beta-Binomialverteilung als:

$$n_{1i} \sim BB(n_i, \; n''{\cdot}(1+r)/(1-r){\cdot}p_{1i}, \; n''{\cdot}(1+r)/(1-r){\cdot}p_{2i})$$

wobei $r$ eine Amplifikationsrate ist, wobei ferner optional die Mehrfachloci-Likelihood-Funktion umfasst:

$$L(\beta, \; n'', \; r, \; \lambda, \; \pi; \; n_1, n_2) = \Pi_i \; [\Sigma g_i \; BB(n_{1i}|n_i, \; n''{\cdot}(1+r)/(1-r){\cdot}p_{1i}, \; n''{\cdot}(1+r)/(1-r){\cdot}p_{2i}) \; P(g_i|\pi)],$$

wobei die Mehrfachloci-Likelihood-Funktion umfasst:

$$L(\beta, \; n'', \; r, \; \lambda, \; \pi; \; n_1, n_2) =$$

$$\Pi_i\Sigma_{g_{1i}g_{2i}}[BB(n_{1i}|n_i, \; n'' \; (1+r)/(1-r){\cdot}p_{1i}(g_{1i}, \; g_{2i}, \; \lambda, \; \beta), \; n'' \; (1+r)/(1-r){\cdot}p_{2i}(g_{1i}, \; g_{2i}, \; \lambda, \; \beta)){\cdot}P(g_{1i}, \; g_{2i}|\pi)]$$

wobei $L(\beta, n'', r, \lambda, \pi; n_1, n_2)$ die Likelihood dafür ist, einen Allelzahlvektor für das erste Allel aller Loci ($n_1$) und einen Allelzahlvektor für das zweite Allel aller Loci ($n_2$) angesichts den Parametern $\beta, n'', r, \lambda$ und $\pi$ zu beobachten.

19. Verfahren nach Anspruch 18, wobei (f) umfasst: durch Definieren einer relativen Amplifikationsrate jedes Polymorphismus-Locus als proportional zu einer Gesamtzahl der Reads des Locus erfolgendes Neuparametrisieren der zweiten Beta-Binomialverteilung als:

$$n_{1i} \sim BB(n_i, \; c'{\cdot}n_i{\cdot}p_{1i}, \; c'{\cdot}n_i{\cdot}p_{2i})$$

wobei:

> *c'* ein zu optimierender Parameter ist; und
> *n<sub>i</sub>* die Gesamtzahl der Reads am Locus *i* ist, optional wobei die Mehrfachloci-Likelihood-Funktion umfasst:

$$L(\beta, n'', c', \lambda, \pi; n_1, n_2) = \Pi_i \left[ \Sigma g_i \, BB(n_{1i}|n_i, c' \cdot n_i \cdot p_{1i}, c' \cdot n_i \cdot p_{2i}) \cdot P(g_i|\pi) \right]$$

**20.** Verfahren nach Anspruch 10, wobei das probabilistische Mischungsmodell Fehler in der Anzahl der Nukleinsäuremoleküle, die aus dem in (a) durchgeführten Extrahieren der Nukleinsäuremoleküle und dem in (b) durchgeführten Amplifizieren der Nukleinsäuremoleküle resultieren, sowie die aus der Sequenzierungsoperation in (c) resultierenden Read-Zählungsfehler berücksichtigt, optional wobei das probabilistische Mischungsmodell eine dritte Beta-Verteilung verwendet, um Allelfraktionen der amplifizierten Nukleinsäuremoleküle für die Allele an dem einen oder den mehreren Polymorphismus-Loci zu modellieren, wobei die Stichprobenfehler, die aus dem in (a) durchgeführten Extrahieren der Nukleinsäuremoleküle und dem in (b) durchgeführten Amplifizieren der Nukleinsäuremoleküle resultieren, berücksichtigt werden, wobei ferner optional der Locus *i* biallel ist und die dritte Beta-Verteilung die folgende Form aufweist:

$$n_{1i}'/(n_{1i}' + n_{2i}') \sim Beta(n' \cdot (1 + r_i)/2 \cdot p_{1i}, \, n'' \cdot (1 + r_i)/2 \cdot p_{2i})$$

wobei:

> *n<sub>1i</sub>'* eine Allelzahl von amplifizierten Nukleinsäuremolekülen für ein erstes Allel am Locus *i* ist;
> *n<sub>2i</sub>'* eine Allelzahl von amplifizierten Nukleinsäuremolekülen für ein zweites Allel am Locus *i* ist;
> *n''* eine Gesamtzahl der Nukleinsäuremoleküle ist;
> *r<sub>i</sub>* die durchschnittliche Amplifikationsrate für den Locus *i* ist;
> *p<sub>1i</sub>* die Wahrscheinlichkeit des ersten Allels am Locus *i* ist; und
> *p<sub>2i</sub>* eine Wahrscheinlichkeit des zweiten Allels am Locus *i* ist.

**21.** Verfahren nach Anspruch 20, wobei (f) umfasst: Kombinieren der ersten Binomialverteilung und der dritten Beta-Verteilung, um die Einzellocus-Likelihood-Funktion von $n_{1i}$ zu erlangen, die einer dritten Beta-Binomialverteilung folgt, optional wobei die dritte Beta-Binomialverteilung die folgende Form aufweist:

$$n_{1i} \sim BB(n_i, n'' \cdot (1 + r_i)/2 \cdot p_{1i}, \, n'' \cdot (1 + r_i)/2 \cdot p_{2i})$$

wobei *r<sub>i</sub>* eine Amplifikationsrate ist, ferner optional, wobei die Mehrfachloci-Likelihood-Funktion umfasst:

$$L(\beta, n'', r, \lambda, \pi; n_1, n_2) =$$
$$\Pi_i \left[ \Sigma g_i \, BB(n_{1i}|n_i, n'' \cdot (1 + r)/2 \cdot p_{1i}, \, n'' \cdot (1 + r)/2 \cdot p_{2i}) \cdot P(g_i|\pi) \right],$$

wobei *r* eine Amplifikationsrate ist, die als gleich für alle Loci angenommen wird.

**22.** Verfahren nach Anspruch 21, wobei die Mehrfachloci-Likelihood-Funktion umfasst:

$$L(\beta, n'', r, \lambda, \pi; n_1, n_2) = \Pi_i \Sigma_{g_{1i}g_{2i}} BB(n_{1i}|n_i, n'' \cdot (1 + r)/2 \cdot p_{1i}(g_{1i}, g_{2i}, \lambda, \beta), \, n'' \cdot (1 +$$
$$r)/2 \cdot p_{2i}(g_{1i}, g_{2i}, \lambda, \beta)) \cdot P(g_{1i}, g_{2i}|\pi)$$

wobei $L(n_1, n_2|\beta, n'', r, \lambda, \pi)$ die Likelihood dafür ist, Allelzahlen für den ersten Allelvektor $n_1$ und eine Allelzahl für den zweiten Allelvektor $n_2$ angesichts Parametern $\beta, n'', r, \lambda$ und $\pi$ zu beobachten.

**23.** Verfahren nach Anspruch 1, ferner umfassend: (g) Schätzen von einem oder mehreren Konfidenzintervallen der einen oder mehreren Nukleinsäurefraktionen der zwei oder mehr Beitragenden unter Verwendung der Hesse-Matrix der Log-Likelihood unter Verwendung numerischer Differenzierung.

24. Verfahren nach Anspruch 1, wobei das Abbilden von (d) umfasst: Verwenden von Computer-Hashbildung und dynamischer Computerprogrammierung, optional wobei das Abbilden von (d) umfasst: Identifizieren von Reads unter den Nukleinsäuresequenz-Reads, die mit jeglicher Sequenz einer Vielzahl von erwartungstreuen Zielsequenzen übereinstimmen, durch den einen oder die mehreren Prozessoren, die Computer-Hashbildung und dynamische Computerprogrammierung verwenden, wobei die Vielzahl von erwartungstreuen Zielsequenzen Teilsequenzen der Referenzsequenz und Sequenzen umfasst, die sich von den Teilsequenzen durch ein einzelnes Nukleotid unterscheiden, optional wobei die Vielzahl von erwartungstreuen Zielsequenzen fünf Kategorien von Sequenzen umfasst, die jede polymorphe Stelle einer Vielzahl von polymorphen Stellen umfassen:

(i) eine Referenzzielsequenz, die eine Teilsequenz der Referenzsequenz ist, wobei die Referenzzielsequenz ein Referenzallel mit einem Referenznukleotid an der polymorphen Stelle aufweist;
(ii) alternative Zielsequenzen, die jeweils ein alternatives Allel mit einem alternativen Nukleotid an der polymorphen Stelle aufweisen, wobei das alternative Nukleotid sich von dem Referenznukleotid unterscheidet;
(iii) mutierte Referenzzielsequenzen, die alle möglichen Sequenzen umfassen, die sich von der Referenzzielsequenz jeweils nur durch ein Nukleotid an einer Stelle, die nicht die polymorphe Stelle ist, unterscheiden;
(iv) mutierte alternative Zielsequenzen, die alle möglichen Sequenzen umfassen, die sich jeweils von einer alternativen Zielsequenz nur durch ein Nukleotid an einer Stelle, die nicht die polymorphe Stelle ist, unterscheiden; und
(v) unerwartete Allel-Zielsequenzen, die jeweils ein unerwartetes Allel aufweisen, das sich von dem Referenzallel und dem alternativen Allel unterscheidet, und die jeweils eine Sequenz aufweisen, die sich von den vorherigen vier Kategorien von Sequenzen unterscheidet,
optional ferner umfassend: Schätzen einer Sequenzierungsfehlerrate $\lambda$ an der varianten Stelle Grundlage einer Häufigkeit des Beobachtens der unerwarteten Allel-Zielsequenzen von (v),
optional wobei (e) umfasst: Verwenden der identifizierten Reads und ihrer übereinstimmenden erwartungstreuen Zielsequenzen, um Allelzahlen der Nukleinsäuresequenz-Reads für die Allele an dem einen oder den mehreren Polymorphismus-Loci zu bestimmen, oder wobei die Vielzahl von erwartungstreuen Zielsequenzen Sequenzen umfasst, die abgeschnitten wurden, damit sie dieselbe Länge wie die Nukleinsäuresequenz-Reads aufweisen, oder wobei die Vielzahl von erwartungstreuen Zielsequenzen Sequenzen umfasst, die in einer oder mehreren Hash-Tabellen gespeichert sind, und die Reads unter Verwendung der Hash-Tabellen identifiziert werden.

25. Verfahren nach Anspruch 1, wobei das Quantifizieren anwendet: (i) ein Computeroptimierungsverfahren, das eine Mehrfachiteration-Rastersuche und ein BFGS-Quasi-Newton-Verfahren oder eine iterative gewichtete lineare Regression kombiniert, und (ii) ein numerisches Differenzierungsverfahren.

26. System zum Quantifizieren einer Nukleinsäureprobe, die Nukleinsäure von zwei oder mehr Beitragenden umfasst, wobei das System umfasst:

(a) einen Sequenzierer, der dafür konfiguriert ist: (i) Nukleinsäuremoleküle zu empfangen, die aus der Nukleinsäureprobe extrahiert wurden, (ii) die extrahierten Nukleinsäuremoleküle zu amplifizieren und (iii) die amplifizierten Nukleinsäuremoleküle unter Bedingungen zu sequenzieren, die Nukleinsäuresequenz-Reads erzeugen; und
(b) einen Computer, der einen oder mehrere Prozessoren umfasst, die dafür konfiguriert sind:
die Nukleinsäuresequenz-Reads auf einen oder mehrere Polymorphismus-Loci auf einer Referenzsequenz abzubilden;
Allelzahlen von Nukleinsäuresequenz-Reads für ein oder mehrere Allele an dem einen oder den mehreren Polymorphismus-Loci unter Verwendung der abgebildeten Nukleinsäuresequenz-Reads zu bestimmen; und
eine oder mehrere Nukleinsäurefraktionen der zwei oder mehr Beitragenden in der Nukleinsäureprobe unter Verwendung eines probabilistischen Mischungsmodells zu quantifizieren,
wobei:

das Verwenden des probabilistischen Mischungsmodells umfasst: Anwenden eines probabilistischen Mischungsmodells auf die Allelzahlen der Nukleinsäuresequenz-Reads, und
das probabilistische Mischungsmodell Wahrscheinlichkeitsverteilungen verwendet, um die Allelzahlen von Nukleinsäuresequenz-Reads an dem einen oder den mehreren Polymorphismus-Loci zu modellieren, wobei die Wahrscheinlichkeitsverteilungen Fehler in den Nukleinsäuresequenz-Reads berücksichtigen, und
die zwei oder mehr Beitragenden einen oder mehrere Spender eines Transplantats und einen Empfänger des Transplantats umfassen und wobei die Nukleinsäureprobe eine Probe umfasst, die von dem Empfänger erlangt wurde.

**27.** Verfahren nach Anspruch 26, ferner ein Werkzeug zum Extrahieren von Nukleinsäuremolekülen aus der Nukleinsäureprobe umfassend, oder wobei die Wahrscheinlichkeitsverteilungen eine erste Binomialverteilung umfassen, die wie folgt lautet:

$$n_{1i} \sim BN(n_i,\ p_{1i})$$

wobei:

$n_{1i}$ eine Allelzahl der Nukleinsäuresequenz-Reads für Allel 1 am Locus $i$ ist;
$n_i$ eine Gesamtzahl der Reads am Locus $i$ ist, die gleich einer Gesamtanzahl der Genomkopien $n''$ ist; und
$p_{1i}$ ein Wahrscheinlichkeitsparameter ist, der die Wahrscheinlichkeit von Allel 1 am Locus $i$ angibt.

**28.** Computerprogrammprodukt, umfassend ein nichtflüchtiges maschinenlesbares Medium, welches Programmcode speichert, der, wenn er durch zwei oder mehr Prozessoren eines Computersystems ausgeführt wird, das Computersystem veranlasst, ein Verfahren zum Quantifizieren einer Nukleinsäureprobe, die Nukleinsäure von zwei oder mehr Beitragenden umfasst, zu implementieren, wobei der Programmcode umfasst:

Code zum Abbilden der Nukleinsäuresequenz-Reads auf einen oder mehrere Polymorphismus-Loci auf einer Referenzsequenz;
Code zum Bestimmen von Allelzahlen von Nukleinsäuresequenz-Reads für ein oder mehrere Allele an dem einen oder den mehreren Polymorphismus-Loci unter Verwendung der abgebildeten Nukleinsäuresequenz-Reads; und
Code zum Quantifizieren einer oder mehrerer Fraktionen von Nukleinsäure der zwei oder mehr Beitragenden in der Nukleinsäureprobe unter Verwendung eines probabilistischen Mischungsmodells,
wobei:

das Verwenden des probabilistischen Mischungsmodells das Anwenden eines probabilistischen Mischungsmodells auf die Allelzahlen von Nukleinsäuresequenz-Reads umfasst, und
das probabilistische Mischungsmodell Wahrscheinlichkeitsverteilungen verwendet, um die Allelzahlen von Nukleinsäuresequenz-Reads an dem einen oder den mehreren Polymorphismus-Loci zu modellieren, wobei die Wahrscheinlichkeitsverteilungen Fehler in den Nukleinsäuresequenz-Reads berücksichtigen, und
die zwei oder mehr Beitragenden einen oder mehrere Spender eines Transplantats und einen Empfänger des Transplantats umfassen und wobei die Nukleinsäureprobe eine Probe umfasst, die von dem Empfänger erlangt wurde.

## Revendications

**1.** Procédé, mis en œuvre dans un système informatique qui inclut un ou plusieurs processeurs et une ou plusieurs mémoires système, de quantification d'un échantillon d'acide nucléique comprenant l'acide nucléique de deux contributeurs ou plus, le procédé comprenant :

(a) l'extraction de molécules d'acide nucléique à partir de l'échantillon d'acide nucléique ;
(b) l'amplification des molécules d'acide nucléique extraites ;
(c) le séquençage des molécules d'acide nucléique amplifiées en utilisant un séquenceur d'acide nucléique pour produire des lectures de séquences d'acide nucléique ;
(d) la cartographie, par le ou les processeurs, des lectures de séquences d'acide nucléique avec un ou plusieurs loci de polymorphisme sur une séquence de référence ;
(e) la détermination, en utilisant les lectures de séquences d'acide nucléique cartographiées et par le ou les processeurs, des nombres d'allèles de lectures de séquences d'acide nucléique pour un ou plusieurs allèles au niveau du ou des loci de polymorphisme ; et
(f) la quantification, en utilisant un modèle de mélange probabiliste et par le ou les processeurs, d'une ou plusieurs fractions d'acide nucléique des deux contributeurs ou plus dans l'échantillon d'acide nucléique, dans lequel l'utilisation du modèle de mélange probabiliste comprend l'application d'un modèle de mélange probabiliste aux nombres d'allèles de lectures de séquences d'acide nucléique, et dans lequel le modèle de mélange probabiliste utilise des distributions de probabilité pour modéliser les nombres d'allèles des lectures de séquences d'acide nucléique au niveau du ou des loci de polymorphisme, les distributions de probabilité tenant compte d'erreurs dans les lectures de séquences d'acide nucléique,

dans lequel les deux contributeurs ou plus comprennent un ou plusieurs donneurs d'une greffe et un receveur de la greffe, et dans lequel l'échantillon d'acide nucléique comprend un échantillon obtenu à partir du receveur.

2. Procédé selon la revendication 1, comprenant en outre la détermination, en utilisant le modèle de mélange probabiliste et par le ou les processeurs, d'un ou plusieurs génotypes des deux contributeurs ou plus au niveau du ou des loci de polymorphisme, ou comprenant en outre la détermination, en utilisant la ou les fractions d'acide nucléique des deux contributeurs ou plus, d'un risque de rejet par un contributeur (un receveur) d'un tissu ou d'un organe transplanté au départ d'un autre contributeur (d'un donneur), ou dans lequel la transplantation comprend une transplantation allogénique ou xénogénique.

3. Procédé selon la revendication 1, dans lequel les molécules d'acide nucléique comprennent des molécules d'ADN ou d'ARN, ou dans lequel les molécules d'acide nucléique extraites comprennent de l'acide nucléique acellulaire ou de l'ADN cellulaire.

4. Procédé selon la revendication 1, dans lequel le ou les loci de polymorphisme comprennent un ou plusieurs loci de polymorphisme bialléliques, ou dans lequel l'allèle ou les allèles au niveau du ou des loci de polymorphisme comprennent un ou plusieurs allèles de polymorphisme mononucléotidique (SNP).

5. Procédé selon la revendication 1, dans lequel le modèle de mélange probabiliste utilise une fonction de vraisemblance à locus unique pour modéliser les nombres d'allèles en un locus de polymorphisme unique, la fonction de vraisemblance à locus unique comprenant

$$M(n_{1i}, n_{2i} | p_{1i}, \theta)$$

où

$n_{1i}$ est le nombre d'allèles de l'allèle 1 au locus i,
$n_{2i}$ est le nombre d'allèles de l'allèle 2 au locus i,
$p_{1i}$ est une fraction attendue de l'allèle 1 au locus i, et
$\theta$ comprend un ou plusieurs paramètres de modèle,
facultativement où $p_{1i}$ est modélisé sous la forme d'une fonction des suivants :

(i) les génotypes des contributeurs au locus $i$, ou $g_i = (g_{11i}, ..., g_{D1i})$, qui est un vecteur du nombre de copies de l'allèle 1 au locus $i$ chez les contributeurs 1...D ;
(ii) les erreurs dans le nombre de lecture résultant de l'opération de séquençage visée au point (c), ou $\lambda$ ; et
(iii) les fractions d'acide nucléique de contributeurs dans l'échantillon d'acide nucléique, ou $\beta = (\beta_1, ..., \beta_D)$, où $D$ est le nombre de contributeurs,

en outre facultativement où

$$p_{1i} = p(g_i, \lambda, \beta) \leftarrow [(1 - \lambda) g_i + \lambda (2 - g_i)] / 2 \bullet \beta,$$

où • est l'opérateur du produit scalaire vectoriel, facultativement où $p_{1i}$ est obtenu en utilisant les valeurs $p_1$' dans le Tableau 3.

6. Procédé selon la revendication 5, dans lequel zéro, un ou plusieurs génotypes des contributeurs sont inconnus, facultativement dans lequel (f) comprend la marginalisation sur une pluralité de combinaisons possibles de génotypes pour énumérer le paramètre de probabilité $p_{1i}$, ou comprenant en outre la détermination d'une configuration de génotype à chacun du ou des loci de polymorphisme, la configuration de génotype comprenant deux allèles pour chacun du ou des contributeurs.

**7.** Procédé selon la revendication 5, dans lequel la fonction de vraisemblance à locus unique comprend une première distribution binomiale.

**8.** Procédé selon la revendication 7, dans lequel la première distribution binomiale est exprimée comme suit :

$$n_{1i} \sim BN(n_i, p_{1i})$$

où

$n_{1i}$ est un nombre d'allèles de lectures de séquences d'acide nucléique pour l'allèle 1 au locus $i$ ; et
$n_i$ est un nombre de lectures total au locus $i$, qui équivaut à un nombre total de copies du génome $n''$.

**9.** Procédé selon la revendication 8, dans lequel (f) comprend la maximisation d'une fonction de vraisemblance à loci multiples calculée à partir d'une pluralité de fonctions de vraisemblance à locus unique, facultativement dans lequel (f) comprend :

le calcul d'une pluralité de valeurs de vraisemblance à loci multiples en utilisant une pluralité de valeurs de fraction potentielle et une fonction de vraisemblance à loci multiples du nombre d'allèles de lectures de séquences d'acide nucléique déterminées sous (e) ;
l'identification d'une ou plusieurs valeurs de fraction potentielle associées à une valeur de vraisemblance à loci multiples maximale ; et
la quantification de la ou des fractions d'acide nucléique du ou des plusieurs contributeurs dans l'échantillon d'acide nucléique en tant que valeur de fraction potentielle identifiée.

**10.** Procédé selon la revendication 9, dans lequel la fonction de vraisemblance à loci multiples comprend :

$$L(\beta, \theta, \lambda, \pi; n_1, n_2) = \Pi_i \left[ \Sigma g_i \, M(n_{1i}, n_{2i} | p(g_i, \lambda, \beta), \theta) \cdot P(g_i | \pi) \right]$$

où

$L(\beta, \theta, \lambda, \pi; n_1, n_2)$ est la vraisemblance d'observer les vecteurs de nombre d'allèles $n_1$ et $n_2$ pour les allèles 1 et 2 ;
$p(g_i, \lambda, \beta)$ est la fraction ou probabilité attendue d'observer l'allèle 1 au locus $i$ sur la base des génotypes $g_i$ des contributeurs au locus i ;
$P(g_i | \pi)$ est la probabilité antérieure d'observer les génotypes $g_i$ au locus i étant donné une fréquence d'allèles dans la population ($\pi$) ; et,
$\Sigma g_i$ désigne la somme sur une pluralité de combinaisons possibles de génotypes des contributeurs, facultativement où la fonction de vraisemblance à loci multiples comprend :

$$L(\beta, \lambda, \pi; n_1, n_2) = \Pi_i \left[ \Sigma g_i \, BN(n_{1i} | n_i, \cdot p(g_i, \lambda, \beta)) \cdot P(g_i | \pi) \right],$$

de préférence où la fonction de vraisemblance comprend :

$$L(\beta, \lambda, \pi; n_1, n_2) = \Pi_i \Sigma_{g1ig2i} \, BN(n_{1i} | n_i, p_{1i}(g_{1i}, g_{2i}, \lambda, \beta)) \cdot P(g_{1i}, g_{2i} | \pi)$$

où

$L(\beta, \lambda, \pi; n_1, n_2)$ est la vraisemblance d'observer des vecteurs de nombre d'allèles $n_1$ à $n_2$ pour les allèles 1 et 2 étant donné les paramètres $\beta$ et $\pi$ ;
$p_{1i}(g_{1i}, g_{2i}, \lambda, \beta)$ est un paramètre de probabilité, tiré sous forme $p_1'$ du Tableau 3, indiquant une probabilité de l'allèle 1 au locus i sur la base des génotypes des deux contributeurs ($g_{1i}, g_{2i}$) ; et
$P(g_{1i}, g_{2i} | \pi)$ est une probabilité antérieure conjointe d'observer les génotypes des deux contributeurs étant donné une fréquence d'allèles dans la population ($\pi$), de façon particulièrement préférée où la probabilité antérieure conjointe est calculée en utilisant des distributions marginales $P(g_{1i} | \pi)$ et $P(g_{2i} | \pi)$ qui satisfont l'équilibre de Hardy-Weinberg, de façon particulièrement préférée où la probabilité antérieure conjointe est calculée en utilisant la relation génétique entre les deux contributeurs.

**11.** Procédé selon la revendication 10, dans lequel le modèle de mélange probabiliste tient compte d'erreurs du nombre de copies de molécules d'acide nucléique résultant de l'extraction des molécules d'acide nucléique effectuée sous (a), ainsi que des erreurs dans le nombre de lecture résultant de l'opération de séquençage sous (c), facultativement dans lequel le modèle de mélange probabiliste utilise une deuxième distribution binomiale pour modéliser les nombres d'allèles des molécules d'acide nucléique extraites pour des allèles au niveau du ou des loci de polymorphisme.

**12.** Procédé selon la revendication 11, dans lequel la deuxième distribution binomiale est exprimée comme suit :

$$n_{1i}'' \sim BN(n_i'',\ p_{1i})$$

où

$n_{1i}''$ est un nombre d'allèles de molécules d'acide nucléique extraites pour l'allèle 1 au locus $i$ ;
$n_i''$ est un nombre total de molécules d'acide nucléique au locus $i$ ; et
$p_{iu}$ est un paramètre de probabilité indiquant la probabilité de l'allèle 1 au locus $i$, facultativement
où la première distribution binomiale est conditionnée par une fraction d'allèle $n_{1i}''/n_i''$, en outre facultativement où la première distribution binomiale est reparamétrée comme suit :

$$n_{1i} \sim BN(n_i,\ n_{1i}''/n_i'')$$

où

$n_{1i}$ est un nombre d'allèles de lectures de séquences d'acide nucléique pour l'allèle 1 au locus $i$ ;
$n_i''$ est un nombre total de molécules d'acide nucléique au locus $i$, qui équivaut à un nombre total de copies du génome $n''$ ;
$n_i$ est un nombre de lectures total au locus $i$ ; et
$n_{1i}''$ est un nombre de molécules d'acide nucléique extraites pour l'allèle 1 au locus $i$.

**13.** Procédé selon la revendication 12, dans lequel le modèle de mélange probabiliste utilise une première distribution bêta pour approximer une distribution de $n_{1i}''/n''$, facultativement dans lequel la première distribution bêta a une moyenne et une variance qui correspondent à une moyenne et à une variance de la deuxième distribution binomiale, ou dans lequel le locus $i$ est modélisé comme étant biallélique et la première distribution bêta est exprimée comme suit :

$$n_{i1}''/n'' \sim Beta((n''-1)p_{1i},\ (n''-1)p_{2i})$$

où

$p_{1i}$ est un paramètre de probabilité indiquant la probabilité d'un premier allèle au locus $i$ ; et
$p_{2i}$ est un paramètre de probabilité indiquant la probabilité d'un deuxième allèle au locus $i$.

**14.** Procédé selon la revendication 13, dans lequel (f) comprend la combinaison de la première distribution binomiale, la modélisation des nombres de lectures de séquençage, et de la première distribution bêta, la modélisation du nombre de molécules d'acide nucléique extraites, pour obtenir la fonction de vraisemblance à locus unique de $n_{1i}$ qui suit une première distribution bêta-binomiale, de préférence dans lequel la première distribution bêta-binomiale a la forme :

$$n_{1i} \sim BB(n_i,\ (n''-1){\cdot}p_{1i},\ (n''-1){\cdot}p_{2i}),$$

ou une autre approximation :

$$n_{1i} \sim BB(n_i,\ n''{\cdot}p_{1i},\ n''{\cdot}p_{2i}),$$

plus préférablement où la fonction de vraisemblance à loci multiples comprend :

$$L(\beta, n'', \lambda, \pi; n_1, n_2) = \Pi i \left[ \Sigma g_i \, BB(n_{1i}|n_i, (n''-1) \cdot p_{1i}, (n''-1) \cdot p_{2i}) \cdot P(g_i|\pi) \right]$$

où $L(\beta, n'', \lambda, \pi; n_1, n_2)$ est la vraisemblance d'observer des vecteurs de nombre d'allèles $n_1$ et $n_2$ pour les allèles $1$ et $2$ à l'ensemble des loci, et $p_{1i} = p(g_i, \lambda, \beta)$, $p_{2i} = 1 - p_{1i}$,
de façon particulièrement préférée où la fonction de vraisemblance à loci multiples comprend :

$$L(\beta, n'', \lambda, \pi; n_1, n_2) =$$

$$\Pi_i \Sigma_{g_{1i}g_{2i}} BB(n_{1i}, n_{2i}|n_i, (n''-1) \cdot p_{1i}(g_{1i}, g_{2i}, \lambda, \beta), (n''-1) \cdot p_{2i}(g_{1i}, g_{2i}, \lambda, \beta)) \cdot P(g_{1i}, g_{2i}|\pi)$$

où $L(\beta, n'', \lambda, \pi; n_1, n_2)$ est la vraisemblance d'observer un vecteur de nombre d'allèles pour le premier allèle de l'ensemble des loci $(n_1)$ et un vecteur de nombre d'allèles pour le deuxième allèle de l'ensemble des loci $(n_2)$ étant donné les paramètres $\beta$, $n''$, $\lambda$, et $\pi$;
$p_{1i}(g_{1i}, g_{2i}, \lambda, \beta)$ est un paramètre de probabilité, tiré sous forme $p_1'$ du Tableau 3, indiquant une probabilité de l'allèle 1 au locus $i$ sur la base des génotypes des deux contributeurs $(g_{1i}, g_{2i})$ ;
$p_{2i}(g_{1i}, g_{2i}, \lambda, \beta)$ est un paramètre de probabilité, tiré sous forme $p_2'$ du Tableau 3, indiquant une probabilité de l'allèle 2 au locus $i$ sur la base des génotypes des deux contributeurs $(g_{1i}, g_{2i})$ ; et
$P(g_{1i}, g_{2i}|\pi)$ est une probabilité antérieure conjointe d'observer le génotype du premier contributeur pour le premier allèle $(g_{1i})$ et le génotype du deuxième contributeur pour le premier allèle $(g_{2i})$ au locus $i$ étant donné une fréquence d'allèles dans la population $(\pi)$.

15. Procédé selon la revendication 12, dans lequel (f) comprend l'estimation du nombre total de copies du génome extrait $n''$ à partir d'une masse des molécules d'acide nucléique extraites, facultativement dans lequel le nombre total de copies du génome extrait estimé $n''$ est ajusté selon la taille de fragments des molécules d'acide nucléique extraites.

16. Procédé selon la revendication 10, dans lequel le modèle de mélange probabiliste tient compte d'erreurs dans le nombre de molécules d'acide nucléique résultant de l'amplification des molécules d'acide nucléique réalisée sous (b), ainsi que d'erreurs dans le nombre de lecture résultant de l'opération de séquençage sous (c), facultativement dans lequel le processus d'amplification de (b) est modélisé comme suit :

$$x_{t+1} = x_t + y_{t+1}$$

où

$x_{t+1}$ est le nombre de copies d'acide nucléique d'un allèle donné après le cycle d'amplification $t+1$ ;
$x_t$ est le nombre de copies d'acide nucléique d'un allèle donné après le cycle d'amplification $t$ ;
$y_{t+1}$ est les nouvelles copies générées au cycle $t+1$, et suit une distribution binomiale $y_{t+1} \sim BN(x_t, r_{t+1})$ ; et
$r_{t+1}$ est le taux d'amplification pour le cycle $t+1$.

17. Procédé selon la revendication 16, dans lequel le modèle de mélange probabiliste utilise une deuxième distribution bêta pour modéliser des fractions d'allèles des molécules d'acide nucléique amplifiées pour des allèles au niveau du ou des loci de polymorphisme, facultativement dans lequel le locus $i$ est biallélique et la deuxième distribution bêta est exprimée comme suit :

$$n_{1i}'/(n_{1i}' + n_{2i}') \sim Beta(n'' \cdot \rho_i \cdot p_{1i}, n'' \cdot \rho_i \cdot p_{2i})$$

où

$n_{1i}'$ est un nombre d'allèles de molécules d'acide nucléique amplifiées pour un premier allèle au locus $i$ ;
$n_{2i}'$ est un nombre d'allèles de molécules d'acide nucléique amplifiées pour un deuxième allèle au locus $i$ ;
$n''$ est un nombre total de molécules d'acide nucléique en un quelconque locus ;
$\rho_i$ est une constante liée à un taux d'amplification moyen r ;
$p_{1i}$ est la probabilité du premier allèle au locus $i$ ; et
$p_{2i}$ est la probabilité du deuxième allèle au locus $i$, en outre facultativement où $\rho_i$ est $(1+r)/(1-r) / [1-(1+r)^{-t}]$, et r est le taux d'amplification moyen par cycle, ou où $\rho_i$ est approximé comme étant $(1+r)/(1-r)$.

**18.** Procédé selon la revendication 17, dans lequel (f) comprend la combinaison de la première distribution binomiale et de la deuxième distribution bêta pour obtenir la fonction de vraisemblance à un seul locus pour $n_{1i}$ qui suit une deuxième distribution bêta-binomiale, facultativement où la deuxième distribution bêta-binomiale a la forme :

$$n_{1i} \sim BB(n_i, n''{\cdot}\rho_i{\cdot}p_{1i}, n''{\cdot}\rho_i{\cdot}p_{2i})$$

où

$n_{1i}$ est un nombre d'allèles de lectures de séquences d'acide nucléique pour le premier allèle au locus $i$ ;
$p_{1i}$ est un paramètre de probabilité indiquant la probabilité d'un premier allèle au locus $i$ ; et
$p_{2i}$ est un paramètre de probabilité indiquant la probabilité d'un deuxième allèle au locus $i$, en outre facultativement où (f) comprend, en supposant que le ou les loci de polymorphisme ont un même taux d'amplification, le reparamétrage de la deuxième distribution bêta-binomiale comme suit :

$$n_{1i} \sim BB(n_i, n''{\cdot}(1+r)/(1-r){\cdot}p_{1i}, n''{\cdot}(1+r)/(1-r){\cdot}p_{2i})$$

où $r$ est un taux d'amplification, en outre facultativement où la fonction de vraisemblance à loci multiples comprend :

$$L(\beta, n'', r, \lambda, \pi; n_1, n_2) = \Pi_i [\Sigma_{g_i} BB(n_{1i}|n_i, n''{\cdot}(1+r)/(1-r){\cdot}p_{1i}, n''{\cdot}(1+r)/(1-r){\cdot}p_{2i}){\cdot}P(g_i|\pi)],$$

ou
où la fonction de vraisemblance à loci multiples comprend :

$$L(\beta, n'', r, \lambda, \pi; n_1, n_2) =$$
$$\Pi_i \Sigma_{g1ig2i} [BB(n_{1i}|n_i, n'' (1+r)/(1-r){\cdot}p_{1i}(g_{1i}, g_{2i}, \lambda, \beta), n'' (1+r)/(1-r){\cdot}p_{2i}(g_{1i}, g_{2i}, \lambda, \beta)){\cdot}P(g_{1i}, g_{2i}|\pi)]$$

où $L(\beta, n'', r, \lambda, \pi; n_1, n_2)$ est la vraisemblance d'observer un vecteur de nombre d'allèles pour le premier allèle de l'ensemble des loci $(n_1)$ et un vecteur de nombre d'allèles pour le deuxième allèle de l'ensemble des loci $(n_2)$ étant donné les paramètres $\beta, n'', r, \lambda$, et $\pi$.

**19.** Procédé selon la revendication 18, dans lequel (f) comprend, en définissant un taux d'amplification relatif de chaque locus de polymorphisme comme étant proportionnel à un total de lectures du locus, le reparamétrage de la deuxième distribution bêta-binomiale comme suit :

$$n_{1i} \sim BB(n_i, c'{\cdot}n_i{\cdot}p_{1i}, c'{\cdot}n_i{\cdot}p_{2i})$$

où

$c'$ est un paramètre à optimiser ; et
$n_i$ est le total des lectures au locus $i$, facultativement où la fonction de vraisemblance à loci multiples comprend :

$$L(\beta, n'', c', \lambda, \pi; n_1, n_2) = \Pi_i [\Sigma_{g_i} BB(n_{1i}|n_i, c'{\cdot}n_i{\cdot}p_{1i}, c'{\cdot}n_i{\cdot}p_{2i}){\cdot}P(g_i|\pi)]$$

**20.** Procédé selon la revendication 10, dans lequel le modèle de mélange probabiliste tient compte d'erreurs dans le nombre de molécules d'acide nucléique résultant de l'extraction des molécules d'acide nucléique effectuée sous (a) et de l'amplification des molécules d'acide nucléique effectuée sous (b), ainsi que d'erreurs dans le nombre de lecture résultant de l'opération de séquençage sous (c), facultativement, dans lequel le modèle de mélange probabiliste utilise une troisième distribution bêta pour modéliser des fractions d'allèles des molécules d'acide nucléique amplifiées pour les allèles au niveau du ou des loci de polymorphisme, en tenant compte des erreurs d'échantillonnage résultant de l'extraction des molécules d'acide nucléique effectuée sous (a) et de l'amplification des molécules d'acide nucléique effectuée sous (b), en outre facultativement dans lequel le locus $i$ est biallélique et la troisième distribution bêta a la forme suivante :

$$n_{1i}'/(n_{1i}' + n_{2i}') \sim Beta(n'' \cdot (1 + r_i)/2 \cdot p_{1i}, \, n'' \cdot (1 + r_i)/2 \cdot p_{2i})$$

où

$n_{1i}'$ est un nombre d'allèles de molécules d'acide nucléique amplifiées pour un premier allèle au locus $i$ ;
$n_{2i}'$ est un nombre d'allèles de molécules d'acide nucléique amplifiées pour un deuxième allèle au locus $i$ ;
$n''$ est un nombre total de molécules d'acide nucléique ;
$r_i$ est le taux d'amplification moyen pour le locus i ;
$p_{1i}$ est la probabilité du premier allèle au locus $i$ ; et
$p_{2i}$ est une probabilité du deuxième allèle au locus $i$.

21. Procédé selon la revendication 20, dans lequel (f) comprend la combinaison de la première distribution binomiale et de la troisième distribution bêta pour obtenir la fonction de vraisemblance à un seul locus de $n_{1i}$ qui suit une troisième distribution bêta-binomiale, facultativement dans lequel la troisième distribution bêta-binomiale a la forme :

$$n_{1i} \sim BB(n_i, \, n'' \cdot (1 + r_i)/2 \cdot p_{1i}, \, n'' \cdot (1 + r_i)/2 \cdot p_{2i})$$

où $r_i$ est un taux d'amplification, en outre facultativement où la fonction de vraisemblance à loci multiples comprend :

$$L(\beta, \, n'', \, r, \, \lambda, \, \pi; \, n_1, \, n_2) =$$
$$\Pi i \, [\Sigma g_i \, BB(n_{1i}|n_i, \, n'' \cdot (1 + r)/2 \cdot p_{1i}, \, n'' \cdot (1 + r)/2 \cdot p_{2i}) \cdot P(g_i|\pi)],$$

où r est un taux d'amplification supposé égal pour l'ensemble des loci.

22. Procédé selon la revendication 21, dans lequel la fonction de vraisemblance à loci multiples comprend :

$$L(\beta, \, n'', \, r, \, \lambda, \, \pi; \, n_1, \, n_2) = \Pi i \, \Sigma_{g1ig2i} \, BB(n_{1i}|n_i, \, n'' \cdot (1 + r)/2 \cdot p_{1i}(g_{1i}, \, g_{2i}, \, \lambda, \, \beta), \, n'' \cdot (1 + r)/2 \cdot p_{2i}(g_{1i}, \, g_{2i}, \, \lambda, \, \beta)) \cdot P(g_{1i}, \, g_{2i}|\pi)$$

où $L(n_1, n_2|\beta, n'', r, \lambda, \pi)$ est la vraisemblance d'observer des nombres d'allèles pour le vecteur de premier allèle $n_1$ et un nombre d'allèles pour le vecteur de deuxième allèle $n_2$ étant donné les paramètres $\beta, n'', r, \lambda,$ et $\pi$.

23. Procédé selon la revendication 1, comprenant en outre : (g) l'estimation d'un ou plusieurs intervalles de confiance de la ou des fractions d'acide nucléique des deux contributeurs ou plus en utilisant la matrice hessienne de la vraisemblance logarithmique en utilisant la différenciation numérique.

24. Procédé selon la revendication 1, dans lequel la cartographie de (d) comprend l'utilisation du hachage informatique et de la programmation dynamique informatique, facultativement dans lequel la cartographie de (d) comprend l'identification, par le ou les processeurs utilisant le hachage informatique et la programmation dynamique informatique, de lectures parmi les lectures de séquences d'acide nucléique correspondant à une quelconque séquence d'une pluralité de séquences cibles non biaisées, dans lequel la pluralité de séquences cibles non biaisées comprend des sous-séquences de la séquence de référence et des séquences qui diffèrent des sous-séquences d'un seul nucléotide, facultativement dans lequel la pluralité de séquences cibles non biaisées comprend cinq catégories de séquences englobant chaque site polymorphe d'une pluralité de sites polymorphes :

(i) une séquence cible de référence qui est une sous-séquence de la séquence de référence, la séquence cible de référence ayant un allèle de référence avec un nucléotide de référence sur le site polymorphe ;
(ii) d'autres séquences cibles ayant chacune un autre allèle avec un autre nucléotide sur le site polymorphe, l'autre nucléotide étant différent du nucléotide de référence ;
(iii) des séquences cibles de référence mutées comprenant l'ensemble des séquences possibles qui diffèrent chacune de la séquence cible de référence par un seul nucléotide sur un site qui n'est pas le site polymorphe ;
(iv) d'autres séquences cibles alternatives mutées comprenant l'ensemble des séquences possibles qui diffèrent chacune d'une autre séquence cible par un seul nucléotide sur un site qui n'est pas le site polymorphe ; et
(v) des séquences cibles d'allèles inattendues ayant chacune un allèle inattendu différent de l'allèle de référence et de l'autre allèle, et ayant chacune une séquence différente des quatre catégories de séquences précédentes, comprenant en outre facultativement l'estimation d'un taux d'erreur de séquençage $\lambda$ au site de variante base d'une fréquence d'observation des séquences cibles d'allèles inattendues de (v),
facultativement dans lequel (e) comprend l'utilisation des lectures identifiées et de leurs séquences cibles non biaisées correspondantes pour déterminer des nombres d'allèles des lectures de séquences d'acide nucléique pour les allèles au niveau du ou des loci de polymorphisme, ou dans lequel la pluralité de séquences cibles non biaisées comprend des séquences tronquées pour avoir la même longueur que les lectures de séquences d'acide nucléique, ou dans lequel la pluralité de séquences cibles non biaisées comprend des séquences stockées dans une ou plusieurs tables de hachage, et les lectures sont identifiées en utilisant les tables de hachage.

25. Procédé selon la revendication 1, dans lequel la quantification emploie (i) une méthode d'optimisation informatique combinant une recherche sur grille à itérations multiples et une méthode BFGS-quasi-Newton, ou une régression linéaire pondérée itérative, et (ii) une méthode de différenciation numérique.

26. Système de quantification d'un échantillon d'acide nucléique comprenant l'acide nucléique de deux contributeurs ou plus, le système comprenant :

(a) un séquenceur configuré pour (i) recevoir des molécules d'acide nucléique extraites de l'échantillon d'acide nucléique, (ii) amplifier les molécules d'acide nucléique extraites, et (iii) séquencer les molécules d'acide nucléique amplifiées dans des conditions qui produisent des lectures de séquences d'acide nucléique ; et
(b) un ordinateur comprenant un ou plusieurs processeurs configurés pour :

cartographier les lectures de séquences d'acide nucléique avec un ou plusieurs loci de polymorphisme sur une séquence de référence ;
déterminer, en utilisant les lectures de séquences d'acide nucléique cartographiées, des nombres d'allèles des lectures de séquences d'acide nucléique pour un ou plusieurs allèles au niveau du ou des loci de polymorphisme ; et
quantifier, en utilisant un modèle de mélange probabiliste, une ou plusieurs fractions d'acide nucléique des deux contributeurs ou plus dans l'échantillon d'acide nucléique,
dans lequel
l'utilisation du modèle de mélange probabiliste comprend l'application d'un modèle de mélange probabiliste aux nombres d'allèles des lectures de séquences d'acide nucléique, et
le modèle de mélange probabiliste utilise des distributions de probabilité pour modéliser les nombres d'allèles des lectures de séquences d'acide nucléique au niveau du ou des loci de polymorphisme, les distributions de probabilité tenant compte des erreurs dans les lectures de séquences d'acide nucléique, et les deux contributeurs ou plus comprennent un ou plusieurs donneurs d'une greffe et un receveur de la greffe, et dans lequel l'échantillon d'acide nucléique comprend un échantillon obtenu à partir du receveur.

27. Système selon la revendication 26, comprenant en outre un outil pour l'extraction de molécules d'acide nucléique à partir de l'échantillon d'acide nucléique, ou dans lequel les distributions de probabilité comprennent une première distribution binomiale comme suit :

$$n_{1i} \sim BN(n_i, \, p_{1i})$$

où

$n_{1i}$ est un nombre d'allèles de lectures de séquences d'acide nucléique pour l'allèle 1 au locus $i$ ;
$n_i$ est un nombre de lectures total au locus $i$, qui équivaut à un nombre total de copies du génome $n''$ ; et
$p_{1i}$ est un paramètre de probabilité indiquant la probabilité de l'allèle 1 au locus $i$.

28. Produit de programme informatique comprenant un support lisible par machine non transitoire stockant un code de programme qui, lorsqu'il est exécuté par deux processeurs ou plus d'un système informatique, amène le système informatique à mettre en œuvre un procédé de quantification d'un échantillon d'acide nucléique comprenant l'acide nucléique de deux contributeurs ou plus, ledit code de programme comprenant :

du code pour cartographier les lectures de séquences d'acide nucléique avec un ou plusieurs loci de polymorphisme sur une séquence de référence ;

du code pour déterminer, en utilisant les lectures de séquences d'acide nucléique cartographiées, des nombres d'allèles des lectures de séquences d'acide nucléique pour un ou plusieurs allèles au niveau du ou des loci de polymorphisme ; et

du code pour quantifier, en utilisant un modèle de mélange probabiliste, une ou plusieurs fractions d'acide nucléique des deux contributeurs ou plus dans l'échantillon d'acide nucléique,

dans lequel

l'utilisation du modèle de mélange probabiliste comprend l'application d'un modèle de mélange probabiliste aux nombres d'allèles des lectures de séquences d'acide nucléique, et

le modèle de mélange probabiliste utilise des distributions de probabilité pour modéliser les nombres d'allèles des lectures de séquences d'acide nucléique au niveau du ou des loci de polymorphisme, les distributions de probabilité tenant compte d'erreurs dans les lectures de séquences d'acide nucléique, et

les deux contributeurs ou plus comprennent un ou plusieurs donneurs d'une greffe et un receveur de la greffe, et dans lequel l'échantillon d'acide nucléique comprend un échantillon obtenu à partir du receveur.

**Figure 1**

200

**Quantify a Nucleic Acid Sample**

202 — Extract Nucleic Acid molecules from the nucleic acid sample including nucleic acid of one or more contributors

204 — Amplify the extracted nucleic acid molecules

206 — Sequence the amplified nucleic acid molecules using a nucleic acid sequencer to produce nucleic acid sequence reads

208 — Map the nucleic acid sequence reads to one or more polymorphism loci on a reference sequence

210 — Determine, using the mapped nucleic acid sequence reads, allele counts of nucleic acid sequence reads for one or more alleles at the one or more polymorphism loci

212 — Apply a probabilistic mixture model to the allele counts of nucleic acid sequence reads

214 — Quantify, using the probabilistic mixture model, one or more fractions of nucleic acid of the one or more contributors in the nucleic acid sample

**Figure 2A**

EP 3 642 747 B1

**Figure 2B**

Figure 2C

300

Quantifying nucleic acid sample
including nucleic acid of one or
more contributors

302 — Receive nucleic acid sequence reads of one or more alleles at one or more polymorphism loci obtained from the nucleic acid sample

304 — Determine, using the nucleic acid sequence reads, allele counts for each of the one or more alleles at the one or more polymorphism loci

306 — Apply a probabilistic mixture model to the allele counts, wherein the probabilistic mixture model uses probability distributions to model the allele counts at the one or more polymorphism loci, the probability distributions accounting for errors in the nucleic acid sequence reads

308 — Obtain likelihood values of observing the allele counts given model parameters and potential nucleic acid fraction values

310 — Quantify, using the likelihood values, fractions of DNA of the one or more contributors in the DNA mixture sample

312 — Determine, using the likelihood values, at least one genotype for at least one of the contributors

**Figure 3**

**Figure 4**

Figure 5

Figure 6

**Figure 7**

EP 3 642 747 B1

**Figure 8**

**Figure 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62522605
- WO 2013130848 A1
- US 61552374
- US 13066816
- US 86373718
- WO 13555037 A
- US 7601499 B
- US 20120053063 A
- US 20090026082
- WO 2009046445 A

### Non-patent literature cited in the description

- **JIANG P et al.** FetalQuant: deducing fractional fetal DNA concentration from massively parallel sequencing of DNA in maternal plasma. *Bioinformatics 2012*, 2012, vol. 28 (22), 2883-2890
- **SAMBROOK et al.** Molecular Cloning: A Laboratory Manual. Cold Spring Harbor, 2001
- **AUSUBEL et al.** *Current Protocols in Molecular Biology*, 1987
- **FAN et al.** *Proc Natl Acad Sci*, 2008, vol. 105, 16266-16271
- **KOIDE et al.** *Prenatal Diagnosis*, 2005, vol. 25, 604-607
- **CHEN et al.** *Nature Med*, 1996, vol. 2, 1033-1035
- **LO et al.** *Lancet*, 1997, vol. 350, 485-487
- **BOTEZATU et al.** *Clin Chem.*, 2000, vol. 46, 1078-1084
- **SU et al.** *J Mol. Diagn.*, 2004, vol. 6, 101-107
- **ALNEMRI** ; **LIWACK**. *J Biol. Chem*, 1990, vol. 265, 17323-17333
- **RICHARDS** ; **BOYER**. *J Mol Biol*, 1965, vol. 11, 327-240
- **BENTLEY et al.** *Nature*, 2009, vol. 6, 53-59
- **FAN et al.** *Clin Chem*, 2010, vol. 56, 1279-1286
- **KOZAREWA et al.** *Nature Methods*, 2009, vol. 6, 291-295
- **LANGMEAD et al.** *Genome Biology*, 2009, vol. 10, R251-R2510
- **HARRIS T.D. et al.** *Science*, 2008, vol. 320, 106-109
- **MARGULIES, M et al.** *Nature*, 2005, vol. 437, 376-380
- **SONI GV** ; **MELLER A**. *Clin Chem*, 2007, vol. 53, 1996-2001